# EUROPEAN PATENT APPLICATION

(11) **EP 2 993 181 A1**
(43) Date of publication of application: **09.03.2016**
(21) Application number: 15187399.9
(22) Date of filing: 27.04.2012
(51) Int. Cl.: C07K 14/005

(54) **NEW ANTIMICROBIAL AGENTS**

(30) Priority: 27.04.2011 EP 11163897
(62) Divisional of application: 12717317.7
(71) Applicant: Lysando AG, 9497 Triesenberg (LI)
(72) Inventor: Miller, Stefan, 93055 Regensburg (DE)
(74) Representative: Rückerl, Florian

(57) **Abstract**

The present invention relates to a fusion protein comprising an endolysin with an amino acid sequence according to SEQ ID NO: 1 or fragments and/or derivatives thereof and a second or further amino acid sequences, such as an additional cationic or polycationic peptide, an amphipatic peptide, a sushi peptide, a defensin, a hydrophobic peptide or an antimicrobial peptide fused to said endolysin, fragment and/or derivative at the N- and/or C-terminus. Moreover, the present invention relates to nucleic acid molecules encoding said fusion protein, vectors comprising said nucleic acid molecules and host cells comprising either said nucleic acid molecules or said vectors. In addition, the present invention relates to said fusion protein for use as a medicament, in particular for the treatment or prevention of staphylococcal infections, as diagnostic means, as cosmetic substance or as sanitizing agent. The present invention also relates to the use of said fusion protein for the treatment or prevention of staphylococcal contamination of foodstuff, of food processing equipment, of food processing plants, of feed for livestock animals, of surfaces coming into contact with foodstuff, of medical devices, of surfaces in hospitals and surgeries. Furthermore, the present invention relates to a pharmaceutical composition comprising said fusion protein.

## Description

The present invention relates to a fusion protein comprising an endolysin with an amino acid sequence according to SEQ ID NO: 1 and fragments and/or derivatives thereof and an additional cationic or polycationic peptide, an amphipatic peptide, a sushi peptide, a defensin, a hydrophobic peptide or an antimicrobial peptide fused to said endolysin, fragment and/or derivative at the N- and/or C-terminus. Moreover, the present invention relates to nucleic acid molecules encoding said fusion protein, vectors comprising said nucleic acid molecules and host cells comprising either said nucleic acid molecules or said vectors. In addition, the present invention relates to said fusion protein for use as a medicament, in particular for the treatment or prevention of staphylococcal infections, as diagnostic means, as cosmetic substance or as sanitizing agent. The present invention also relates to the use of said fusion protein for the treatment or prevention of staphylococcal contamination of foodstuff, of food processing equipment, of food processing plants, of feed for livestock animals, of surfaces coming into contact with foodstuff, of medical devices, of surfaces in hospitals and surgeries. Furthermore, the present invention relates to a pharmaceutical composition comprising said fusion protein.

The bacterium *Staphylococcus aureus* is a facultative anaerobic, Gram-positive coccus. It occurs almost everywhere in the nature, as well as on the skin and the upper respiratory tract of about 25 to 30 % humans. Usually *Staphylococcus aureus* does not cause any symptoms. Due to more favourable conditions or a weak immune system, *Staphylococcus aureus* may spread and thus, causes in humans infections of the skin, like pyoderma, particularly folliculitis, furuncle, carbuncle, abscesses of the sweat glands and pemphigus, and like scaled skin syndrome, endocarditis, toxic shock syndrome and sepsis. In cases such bacteria are resistant against several important antibiotics (multi-resistant), it is hard to eliminate these bacteria and they become very dangerous after infection of third parties.

Multi-resistant *Staphylococcus aureus* are *Staphylococcus aureus* strains which are resistant against all commercially available β-lactam antibiotics, e.g. penicillin. But they are often resistant against more than one class of antibiotics, such as chinolone, tetracyclins, aminoglycosides, erythromycin and sulfonamides. The increasing rate of multi-resistant S. *aureus* strains represents a serious problem in the therapy of infections due to *S. aureus*. Thus, there is a need for antimicrobial agents against *Staphylococcus aureus*.

Endolysins are peptidoglycan hydrolases encoded by bacteriophages (or bacterial viruses). They are synthesized during late gene expression in the lytic cycle of phage multiplication and mediate the release of progeny virions from infected cells through degradation of the bacterial peptidoglycan. They are either ß-(1,4)-glycosylases (lysozymes), transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this simple concept of combating human pathogens with endolysins received interest. A compelling need to develop totally new classes of antibacterial agents emerged and endolysins used as 'enzybiotics' - a hybrid term of 'enzymes' and 'antibiotics' - perfectly met this need. In 2001, Fischetti and coworkers demonstrated for the first time the therapeutic potential of bacteriophage Cl endolysin towards group A streptococci (Nelson et a1., 2001). Since then many publications have established endolysins as an attractive and complementary alternative to control bacterial infections, particularly by Gram-positive bacteria. Subsequently different endolysins against other Gram-positive pathogens such as *Streptococcus pneumoniae* (Loeffler et al., 2001), *Bacillus anthracis* (Schuch et al., 2002), *S. agalactiae* (Cheng et al., 2005) and *Staphylococcus aureus* (Rashel et al, 2007) have proven their efficacy as enzybiotics. However, it is also known that endolysins can, under some conditions (e.g. high ionic strength), create stable protoplast, where the internal bacterial cell pressure is not sufficient to lead to a cell burst. Under these conditions the bacterial cell wall can regenerate and the bacteria will survive.

Thus, there is a need for new antimicrobial agents against Staphylococcal bacteria.

This object is solved by the subject matter defined in the claims.

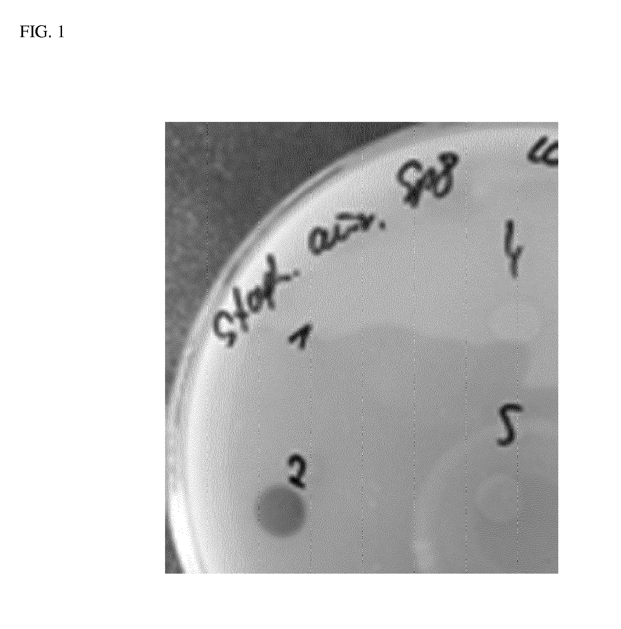

Figure 1 shows a photograph of a Staph. aureus culture grown on an agar plate with a well-defined clear cut spot (black arrow; #2) that appeared within 16h of incubation with Ply2638A-PK.

The term "protein" as used herein refers synonymously to the term "polypeptide". The term "protein" as used herein refers to a linear polymer of amino acid residues linked by peptide bonds in a specific sequence. The amino-acid residues of a protein may be modified by e.g. covalent attachments of various groups such as carbohydrates and phosphate. Other substances may be more loosely associated with the polypeptide chains, such as heme or lipid, giving rise to the conjugated proteins which are also comprised by the term "protein" as used herein. There are various ways in which the polypeptide chains fold have been elucidated, in particular with regard to the presence of alpha helices and beta-pleated sheets. The term "protein" as used herein refers to all four classes of proteins being all-alpha, all-beta, alpha/beta and alpha plus beta. Moreover, the term "protein" refers to a complex, wherein the complex refers to a homomer.

The term "fusion protein" as used herein refers to an expression product resulting from the fusion of two or more nucleic acid sequences. Such a protein may be produced, e.g., in recombinant DNA expression systems. Moreover, the term "fusion protein" as used herein refers to a fusion of a first amino acid sequence, namely the endolysin Ply2638A and fragments and/or derivatives thereof, with a second or further amino acid sequences. The second or further amino acid sequence is preferably a peptide, in particular a cationic and/or a polycationic peptide, an amphipatic peptide, a sushi peptide, a defensin, a hydrophobic peptide and/or an antimicrobial peptide. Preferably, said second and/or further amino acid sequence is foreign to and not substantially homologous with any domain of the first amino acid sequence.

The term "peptide stretch" as used herein refers to any kind of peptide linked to a protein such as an endolysin. In particular the term "peptide stretch" as used herein refers to a cationic peptide or a polycationic peptide, an amphipatic peptide, a sushi peptide, a defensin, a hydrophobic peptide and/or an antimicrobial peptide. However, a peptide stretch in the meaning of the present invention does not refer to His-tags, preferably His₅-tags, His₆-tags, His₇-tags, His₈-tags, His₉-tags, His₁₀-tags, His₁₁-tags, His₁₂-tags, His₁₆-tags and His₂₀-tags, Strep-tags, Avi-tags, Myc-tags, Gst-tags, JS-tags, cystein-tags, FLAG-tags or other tags known in the art, thioredoxin or maltose binding proteins (MBP). The term "tag" in contrast to the term "peptide stretch" as used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His₆-tag may, depending on the respective pH, also be positively charged, but is used as affinity purification tool as it binds to immobilized divalent cations and is not used as a peptide stretch according to the present invention.

The term "peptide" as used herein refers to short polypeptides consisting of from about 2 to about 100 amino acid residues, more preferably from about 4 to about 50 amino acid residues, more preferably to about 5 to 30 amino acid residues, wherein the amino group of one amino acid residue is linked to the carboxyl group of another amino acid residue by a peptide bond. A peptide may have a specific function. A peptide can be a naturally occurring peptide or a synthetically designed and produced peptide. The peptide can be, for example, derived or removed from a native protein by enzymatic or chemical cleavage, or can be prepared using conventional peptide synthesis techniques (e.g., solid phase synthesis) or molecular biology techniques (see Sambrook, J. et al., Molecular Cloning: A Laboratory Manual, Cold Spring Harbor Press, Cold Spring Harbor, N.Y. (1989)). Preferred synthetically produced peptides are e.g. cationic, polycationic peptides, amphipatic peptides and hydrophobic peptides. Preferred naturally occuring peptides are e.g. amphipatic peptides, sushi peptides, defensines and antimicrobial peptides. The peptides have no or only low cell wall degrading activity but may have cell wall disprupting activity. A peptide in the meaning of the present invention does not refer to His-tags, Strep-tags, thioredoxin or maltose binding proteins (MBP) or the like, which are used to purify or locate proteins.

As used herein, the term "cationic peptide" refers to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides.

The term "polycationic peptide" as used herein refers to a synthetically designed and produced peptide composed of mostly positively charged amino acid residues, in particular lysine, arginine and/or histidine residues, more preferably lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers to any naturally occurring peptide that has microbicidal and/or microbistatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, antiparasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. The antimicrobial peptide may be a member of the RNAse A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in *Xenopus laevis, Rana* frogs, more preferably in *Rana catesbeiana,* toad, preferably Asian toad *Bufo bufo gargarizans,* fly, preferably in *Drosophila* more preferably in *Drosophila melanogaster,* in *Aedes aegypti,* in honey bee, bumblebee, preferably in *Bombus pascuorum*, flesh fly, preferably in *Sarcophaga peregrine,* scorpion, horseshoe crab, catfish, preferably in *Parasilurus asotus,* cow, pig, sheep, porcine, bovine, monkey and human.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "amphiphatic peptide" as used herein refers to synthetic peptides having both hydrophilic and hydrophobic functional groups. Preferably, the term "amphiphatic peptide" as used herein refers to a peptide having a defined arrangement of hydrophilic and hydrophobic groups e.g. amphiphatic peptides may be e.g. alpha helical, having predominantly non polar side chains along one side of the helix and polar residues along the remainder of its surface.

The term "hydrophobic group" as used herein refers to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a nonaqueous environment. Examples of amino acid residues with hydrophobic side chains are valine, isoleucine, leucine, methionine, phenylalanine, tryptophan, cysteine, alanine, tyrosine, histidine, threonine, serine, proline and glycine residues.

The term "endolysin" as used herein refers to an enzyme which is suitable to hydrolyse bacterial cell walls. "Endolysins" comprise at least one "enzymatically active domain" (EAD) having at least one of the following activities: endopeptidase, N-acetyl-muramoyl-L-alanine-amidase (amidase), N-acetyl-muramidase, N-acetyl-glucosaminidase (lysozyme) or transglycosylases. In addition, the endolysins may contain also regions which are enzymatically inactive, and bind to the cell wall of the host bacteria, the so-called CBDs (cell wall binding domains). The endolysin may contain one, two or more CBDs. However, the term "endolysin" as used herein refers also to enzymes having at least one EAD but no CBDs. Generally, the cell wall binding domain is able to bind different components on the surface of bacteria. Preferably, the cell wall binding domain is a peptidoglycan binding domain and binds to the bacteria's peptidoglycan.

The term "cell wall" as used herein refers to all components that form the outer cell enclosure of the Gram-positive bacteria and thus guarantee their integrity. In particular, the term "cell wall" as used herein refers to peptidoglycan, the bacterial cell membrane, but also to additional layers deposited on the peptidoglycan as e.g. capsules, outer protein layers or slimes. The fusion proteins aqccording to the present invention have a cell wall degrading activity, i.e. they cleave the components, e.g.the peptidoglycan enzymatically. In contrast the peptides do have no or only very low enzymatic activity but may have cell wall disrupting, i.e. physically disrupting activity.

The term "EAD" as used herein refers to the enzymatically active domain of an endolysin. The EAD is responsible for hydrolysing bacterial peptidoglycans. It exhibits at least one enzymatic activity of an endolysin. The EAD can also be composed of more than one enzymatically active module. The term "EAD" is used herein synonymously with the term "catalytic domain".

The term "domain linker" as used herein refers to an amino acid sequence functioning to connect single protein domains with one another. As a rule domain linkers form no or only few regular secondary structure like α-helices or ß-sheets and can occupy different conformations with the respective structural context. Methods to detect domain linker and properties of linker sequences are well known in the art as e.g. described in Bae et al., 2005, Bioinformatics, 21, 2264-2270 or George & Heringa, 2003, Protein Engineering, 15, 871-879. The term "deletion" as used herein refers to the removal of 1, 2, 3, 4, 5 or more amino acid residues from the respective starting sequence.

The term "insertion" or "addition" as used herein refers to the insertion or addition of 1, 2, 3, 4, 5 or more amino acid residues to the respective starting sequence.

The term "substitution" as used herein refers to the exchange of an amino acid residue located at a certain position for a different one.

The present invention relates to new antibacterial agents against staphylococcal bacteria. Surprisingly the inventors found that in comparison to the wild type Ply2638A endolysin that has only very low bacterial cell wall degrading, i.e. antibacterial activity on *Staphylococcus aureus* strains the fusion proteins according to the present invention have a very strong antibacterial activity on *Staphylococcus aureus* strains. Thus, one aspect of the present invention relates to a fusion protein comprising the endolysin Ply2638A comprising an amino acid sequence according to SEQ ID NO: 1, a fragment and/or derivative thereof and an additional cationic,polycationic peptide, an amphipatic peptide, a sushi peptide, a defensin, a hydrophobic peptide and/or an antimicrobial peptide fused to the N- and/or C-terminus of said endolysin, fragment and/or derivative thereof. The endolysin Ply2638 comprising an amino acid sequence according to SEQ ID NO: 1 is preferably encoded by a nucleotide sequence according to SEQ ID NO: 2.

The endolysin Ply2638 (GENBANK Accession number YP_239818) having an amino acid sequence according to SEQ ID NO: 1 has a length of 486 amino acids. It comprises a C-terminal cell wall binding domain (CBD) and two N-terminal enzymatic active domains (EADs). The putative C-terminal CBD is a peptidoglycan binding domain (PGB, aa 401-468) having an amino acid sequence according to SEQ ID NO: 3. The N-terminal EADs consists of a putative M23 peptidase (aa 47-146) according to SEQ ID NO: 4 and a putative amidase domain (aa 199-325) according to SEQ ID NO: 5. The PGB and the catalytic domains of the endolysin Ply2638 are connected by linker sequences.

Preferred fragments of the endolysin Ply2638A according to the present invention are polypeptides comprising an amino acid sequence according to SEQ ID NO: 3, according to SEQ ID NO: 4 and/or according to SEQ ID NO: 5. Another preferred fragment of the endolysin according to the present invention comprises an amino acid sequence according to SEQ ID NO: 6. The fragment having an amino acid sequence according to SEQ ID NO: 6 differs from the endolysin having an amino acid sequence according to SEQ ID NO: 1 in that the starting methionine residue has been deleted.

The derivatives of the endolysin according to the present invention are polypeptides comprising an amino acid sequence according to SEQ ID NO: 1, 3, 4, 5 and/or 6 but having additional modification and/or alterations. Said modifications and/or alterations can be mutations in particular deletions, insertions, additions, substitutions or any combinations thereof and/or chemical changes of the amino acid residues, e.g. biotinylation, acetylation, pegylation, chemical changes of the amino-, SH- or carboxyl- groups. Said derivatives according to the present invention exhibit the lytic activity of the Ply2638 endolysin or the activity of the fragments according to the present invention. Said activity can be about 10, 20, 30, 40, 50, 60, 70, 80, 90, 100, 110, 120, 130, 140, 150, 160, 170, 180, 190 or about 200 % of the activity of the Ply2638A and/or the activity of the fragments according to the present invention. The activity can be measured by assays well known in the art by a person skilled in the art as e.g. the plate lysis assay or the liquid lysis assay which are e.g. described in Briers et al, J. Biochem. Biophys Methods 70: 531-533, (2007*) or similar publications.*

In a preferred embodiment of the present invention the fusion protein comprising the endolysin according to SEQ ID NO: 1, a fragment and/or derivative thereof and an additional cationic or polycationic peptide, an amphipatic peptide, a sushi peptide, a defensin, a hydrophobic peptide and/or an antimicrobial peptide at the N- and/or C-terminus of said endolysin, fragment and/or derivative thereof according to the present invention comprises additionally a tag such as a His₆-tag, Strep-tag, Avi-tag, Myc-tag, Gst-tag, JS-tag, cystein-tag, FLAG-tag or other tags known in the art at the N-terminus and/or at the C-terminus. In a preferred embodiment of the present invention said tag is linked to the fusion protein according to the present invention at the C-terminus. Said tag may be linked to said fusion protein over additional amino acid residues. Said additional amino acid residues may be at least 1, 2, 3, 4, 5, 6, 7, 8, 9 or 10 additional amino acid residues. In a preferred embodiment of the present invention the tag is linked to the fusion protein according to the present invention by the additional amino acid residues Leu-Glu or Lys-Gly.

The peptide according to the present invention is preferably covalently bound to the endolysin, fragment and/or derivative thereof according to the present invention. Preferably, said peptide consists of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 amino acid residues. Especially preferred is a peptide comprising about 5 to about 100 amino acid residues, about 5 to about 50 or about 5 to about 30 amino acid residues. More preferred is a peptide comprising about 6 to about 42 amino acid residues, about 6 to about 39 amino acid residues, about 6 to about 38 amino acid residues, about 6 to about 31 amino acid residues, about 6 to about 25 amino acid residues, about 6 to about 24 amino acid residues, about 6 to about 22 amino acid residues, about 6 to about 21 amino acid residues, about 6 to about 20 amino acid residues, about 6 to about 19 amino acid residues, about 6 to about 16 amino acid residues, about 6 to about 14 amino acid residues, about 6 to about 12 amino acid residues, about 6 to about 10 amino acid residues or about 6 to about 9 amino acid residues.

Preferably, the peptide is no tag such as a His₆-tag, Strep-tag, Avi-tag, Myc-tag, Gst-tag, JS-tag, cystein-tag, FLAG-tag or other tags known in the art and no thioredoxin or maltose binding proteins (MBP). However, the peptide may comprise in addition such tag or tags or the like, which are used to purify or locate proteins.

Preferably, the peptide has the function to facilitate lysis of Gram-positive bacteria by the fusion protein according to the present invention but has no or only low cell wall degrading activity when administered without being fused to the endolysin, fragment and/or derivative thereof according to the present invention. Subsequently, the antibacterial activity of the fusion protein consisting of the endolysin, fragment and/or derivative thereof according to the present invention and the peptide to be tested can be compared to the endolysin, fragment and/or derivative thereof according the present invention having no peptide as also described in the Examples of the present invention. Preferably, said tests may be carried out on staphylococcal cells as used in the Examples of the present invention. In case the fusion protein has an increased antibacterial activity in comparison to the endolysin, fragment and/or derivative thereof according to the present invention without said peptide for at least one of the tested staphylococcal bacteria species then said peptide has a cell wall disrupting activity. Preferably, the antibacterial activity (in logarithmic units (=log₁₀N₀/Nᵢ)) of the endolysin, fragment and/or derivative thereof according to the present invention is increased, preferably by at least about 5 %, more preferably by at least about 10%, 20%, 30%, 40% 50% 60%, 70%, 80%, 90% or 100% or by any value in the range of up to 100% or even more by a peptide having cell wall disrupting activity.

In a preferred embodiment the peptide according to the present invention is selected from the group of cationic peptides and polycationic peptides.

In one aspect of the present invention the peptide is an cationic and/or polycationic peptide, which comprises one or more of the positively charged amino acid residues of lysine, arginine and/or histidine, in particular of lysine and/or arginine. Preferably, more than about 20, 30, 40, 50, 60, 70, 75, 80, 85, 90, 95 or 99 % of the amino acid residues in said peptide are positively charged amino acid residues, in particular lysine and/or arginine residues. Especially preferred are peptides consisting of about 100 % positively charged amino acid residues, in particular arginine and/or lysine residues, wherein preferably about 60 % to about 70 % of said positively charged amino acid residues are lysine residues and about 30 % to about 40 % of said positively charged amino acid residues are arginine residues. More preferred is a peptide consisting of about 100 % positively charged amino acid residues, in particular arginine and/or lysine residues, wherein preferably about 64 % to about 68 % of said positively charged amino acid residues are lysine and about 32 % to about 36 % of said positively charged amino acid residues are arginine. Peptides consisting of either only arginine or only lysine are also preferred.

Especially preferred are cationic and/or polycationic peptides comprising at least one motive according to SEQ ID NO: 11 (KRKKRK). In particular cationic peptides comprising at least 2, 3, 4, 5, 6, 7, 8, 9, 10, 11, 12, 13, 14, 15, 16 or 17 motives according to SEQ ID NO: 11 (KRKKRK) are preferred. More preferred are cationic peptides comprising at least one KRK motive (lys-arg-lys), preferable at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33 KRK motives.

In another preferred embodiment of the present invention the cationic peptide comprises beside the positively charged amino acid residues, in particular lysine and/or arginine residues, neutrally charged amino acid residues, in particular glycine and/or serine residues. Preferred are cationic peptides consisting of about 70 % to about 100 %, or about 80 % to about 95 %, or about 85 % to about 90 % positively charged amino acid residues, in particular lysine and/or arginine residues and of about 0 % to about 30 %, or about 5 % to about 20 %, or about 10 % to about 20 % neutrally charged amino acid residues, in particular glycine and/or serine residues. Preferred are peptides consisting of about 4 % to about 8 % serine residues, of about 33 % to about 36 % arginine residues and of about 56 % to about 63 % lysine residues. Especially preferred are peptides comprising at least one motive according to SEQ ID NO: 32 (KRXKR), wherein X is any other amino acid residue than lysine, arginine and histidine. Especially preferred are peptides comprising at least one motive according to SEQ ID NO: 33 (KRSKR). More preferred are cationic peptides comprising at least about 2, 3, 4, 5, 6, 7, 8, 9, 10, 11, 12, 13, 14, 15, 16, 17, 18, 19 or about 20 motives according to SEQ ID NO: 32 (KRXKR) or SEQ ID NO: 33 (KRSKR).

Also preferred are peptides consisting of about 9 to about 16 % glycine residues, of about 4 to about 11 % serine residues, of about 26 to about 32 % arginine residues and of about 47 to about 55 % lysine residues. Especially preferred are peptides comprising at least one motive according to SEQ ID NO: 34 (KRGSG). More preferred are cationic peptides comprising at least about 2, 3, 4, 5, 6, 7, 8, 9, 10, 11, 12, 13, 14, 15, 16, 17, 18, 19 or about 20 motives according to SEQ ID NO: 34 (KRGSG).

In another preferred embodiment of the present invention the cationic peptide comprises beside the positively charged amino acid residues, in particular lysine and/or arginine residues, hydrophobic amino acid residues, in particular valine, isoleucine, leucine, methionine, phenylalanine, tryptophan, cysteine, alanine, tyrosine, histidine, threonin, serine, proline and/or glycine residues. Preferred are cationic peptides consisting of about 70 % to about 100 %, or about 80 % to about 95 %, or about 85 % to about 90 % positively charged amino acid residues, in particular lysine and/or arginine residues and of about 0 % to about 30 %, or about 5 % to about 20 %, or about 10 % to about 20 % hydrophobic amino acid residues in particular valine, isoleucine, leucine, methionine, phenylalanine, tryptophan, cysteine, alanine, tyrosine, histidine, threonin, serine, proline and/or glycine residues.

Especially preferred peptides are presented in the following table:

**Table 1:**

| **Peptide** | **length** | **SEQ ID NO:** |
|---|---|---|
| KRKKRK | 6 | SEQ ID NO: 11 |
| KRKKRKKRK | 9 | SEQ ID NO: 12 |
| RRRRRRRRR | 9 | SEQ ID NO: 13 |
| KKKKKKKK | 8 | SEQ ID NO: 14 |
| KRKKRKKRKK | 10 | SEQ ID NO: 15 |
| KRKKRKKRKKRK | 12 | SEQ ID NO: 16 |
| KRKKRKKRKKRKKR | 14 | SEQ ID NO: 17 |
| KKKKKKKKKKKKKKKK | 16 | SEQ ID NO: 18 |
| KRKKRKKRKKRKKRKKRKK | 19 | SEQ ID NO: 19 |
| RRRRRRRRRRRRRRRRRRR | 19 | SEQ ID NO: 20 |
| KKKKKKKKKKKKKKKKKKK | 19 | SEQ ID NO: 21 |
| KRKKRKKRKRSKRKKRKKRK | 20 | SEQ ID NO: 22 |
| KRKKRKKRKRSKRKKRKKRKK | 21 | SEQ ID NO: 23 |
| KRKKRKKRKKRKKRKKRKKRK | 21 | SEQ ID NO: 24 |
| KRKKRKKRKRGSGKRKKRKKRK | 22 | SEQ ID NO: 25 |
| KRKKRKKRKRGSGSGKRKKRKKRK | 24 | SEQ ID NO: 26 |
| KRKKRKKRKKRKKRKKRKKRKKRKK | 25 | SEQ ID NO: 27 |
| KRKKRKKRKRSKRKKRKKRKRSKRKKRKKRK | 31 | SEQ ID NO: 28 |
| KRKKRKKRKRGSGSGKRKKRKKRKGSGSGKRKKRKKRK | 38 | SEQ ID NO: 29 |
| KRKKRKKRKKRKKRKKRKKRKKRKKRKKRKKRKKRKKRK | 39 | SEQ ID NO: 30 |
| KRKKRKKRKRSKRKKRKKRKRSKRKKRKKRKRSKRKKRKKRK | 42 | SEQ ID NO: 31 |

In a further preferred embodiment of the present invention the fused peptide stretch is an amphipathic peptide, which comprises one or more of the positively charged amino acid residues of lysine, arginine and/or histidine, combined to one or more of the hydrophobic amino acid residues of valine, isoleucine, leucine, methionine, phenylalanine, tryptophan, cysteine, alanine, tyrosine, histidine, threonin, serine, proline and/or glycine. Side chains of the amino acid residues are preferably oriented in order that cationic and hydrophobic surfaces are clustered at opposite sides of the peptide. Preferably, more than about 30, 40, 50, 60 or 70% of the amino acids in said peptide are positively charged amino acids. Preferably, more than about 30, 40, 50, 60 or 70% of the amino acid residues in said peptide are hydrophobic amino acid residues. Advantageously, the amphipathic peptide is fused at the N-terminal and/or the C-terminal end of the polypeptide, fragment and/or derivative according to the present invention having cell wall degrading activity, thus enhancing the amphipathicity of the latter proteins.

In a preferred embodiment at least about 30, 40, 50, 60 or 70% of the said amino acid residues of the amphipathic peptide are either arginine or lysine residues and/or at least about 30, 40, 50, 60 or 70% of the said amino acid residues of the amphipathic peptide are of the hydrophobic amino acid residues valine, isoleucine, leucine, methionine, phenylalanine, tryptophan, cysteine, alanine, tyrosine, histidine, threonin, serine, proline and/or glycine. Preferred amphipatic peptides are Pleurocidin according to SEQ ID NO:45, Cecropin P1 according to SEQ ID NO:52, Buforin II according to SEQ ID NO:47, Buforin I according to SEQ ID NO:61 and Magainin according to SEQ ID NO:44. Further preferred amphipatic peptides are Cathelidicine e.g. LL-37 according to SEQ ID NO:43.

In a further aspect of the present invention the fused peptide stretch is an antimicrobial peptide, which comprises a positive net charge and around 50% hydrophobic amino acid residues. The antimicrobial peptides are amphipathic, with a length of about 12 to about 50 amino acid residues.

Preferred antimicrobial peptides are listed in the following Table 2.

| **Peptid** | **Sequenz** | |
|---|---|---|
| LL-37 | LLGDFFRKSKEKIGKEFKRIVQRIKDFLRNLVPRTES | SEQ ID NO: 42 |
| SMAP-29 | RGLRRLGRKIAHGVKKYGPTVLRIIRIAG | SEQ ID NO: 49 |
| Indolicidin | ILPWKWPWWPWRR | SEQ ID NO: 43 |
| Protegrin | RGGRLCYCRRRFCVCVGR | SEQ ID NO: 51 |
| Cecropin P1 | SWLSKTAKKLENSAKKRISEGIAIAIQGGPR | SEQ ID NO: 52 |
| Magainin | GIGKFLHSAKKFGKAFVGEIMNS | SEQ ID NO: 44 |
| Pleurocidin | GWGSFFKKAAHVGKHVGKAALTHYL | SEQ ID NO: 45 |
| Cecropin A (A.aeqypti) | GGLKKLGKKLEGAGKRVFNAAEKALPVVAGAKALRK | SEQ ID NO: 46 |
| Cecropin A (D. melanogaster) | GWLKKIGKKIERVGQHTRDATIQGLGIPQQAANVAATARG | SEQ ID NO: 53 |
| Buforin II | TRSSRAGLQFPVGRVHRLLRK | SEQ ID NO: 47 |
| Sarcotoxin IA | GWLKKIGKKIERVGQHTRDATIQGLGIAQQAANVAATAR | SEQ ID NO: 48 |
| Ascaphine | GIKDWIKGAAKKLIKTVASHIANQ | SEQ ID NO: 54 |
| Apidaecine | ANRPVYIPPPRPPHPRL | SEQ ID NO: 55 |
| Nigrocine | GLLSKVLGVGKKVLCGVSGLVC | SEQ ID NO: 56 |
| Pseudin 1 | GLNTLKKVFQGLHEAIKLINNHVQ | SEQ ID NO: 41 |
| Parasin 1 | KGRGKQGGKVRAKAKTRSS | SEQ ID NO: 57 |
| Lycotoxin | IWLTALKFLGKHAAKKLAKQQLSKL | SEQ ID NO: 58 |
| Ranalexin | FLGGLIVPAMICAVTKKC | SEQ ID NO: 59 |
| Melittin | GIGAVLKVLTTGLPALISWIKRKRQQ | SEQ ID NO: 60 |
| OR-7 | KTYYGTNGVHCTKNSLWGKVRLKNMKYDQNTTYM GRLQDILLGWATGAFGKTFH | SEQ ID NO: 50 |
| Buforin I | AGRGKQGGKVRAKAKTRSSRAGLQFPVGRVHRLLRKGNY | SEQ ID NO: 61 |

In a further aspect of the present invention the fused peptide stretch is a sushi peptide which is described by Ding JL, Li P, Ho B Cell Mol Life Sci. 2008 Apr;65(7-8):1202-19. The Sushi peptides: structural characterization and mode of action against Gram-negative bacteria. Preferred sushi peptides are sushi peptides S1 and S3 and multiples thereof; FASEB J. 2000 Sep;14(12):1801-13.

In a further aspect of the present invention the fused peptide stretch is a defensin, preferably Cathelicidine, Cecropin P1, Cecropin A or Magainin II.

In a further aspect of the present invention the fused peptide stretch is a hydrophobic peptide, preferably having the amino acid sequence Phe-Phe-Val-Ala-Pro .

Further preferred peptide stretches are listed in the following Table 3:

| | | |
|---|---|---|
| Alpha 4 | PNRAKRVITTFRT | SEQ ID NO: 62 |
| Artilysin1 | GFFIPAVILPSIAFLIVP | SEQ ID NO: 63 |
| Artilysin2 | GKPGWLIKKALVFKKLIRRPLKRLA | SEQ ID NO: 64 |
| WLBU2 variant | KRWVKRVKRVKRWVKRVVRVVKRWVKR | SEQ ID NO: 65 |

In another preferred embodiment of the present invention the peptide of the fusion protein according to the present invention comprise modifications and/or alterations of the amino acid sequences. Such alterations and/or modifications may comprise mutations such as deletions, insertions and additions, substitutions or combinations thereof and/or chemical changes of the amino acid residues, e.g. biotinylation, acetylation, peglyation, chemical changes of the amino-, SH- or carboxyl-groups.

A fusion protein according to the present invention as already outlined above is composed of
(a) an endolysin, fragment and/or derivative thereof according to the present invention, and
(b) a peptide fused to said endolysin, fragment and/or derivative thereof at the Nor C-Terminus, and optionally
(c) a tag, such as a His-tag, Strep-tag, Avi-tag, Myc-tag, Gst-tag, JS-tag, cystein-tag, FLAG-tag or other tags known in the art at the N- and/or C-Terminus.

The two and three components of the fusion protein, respectively, as outlined above may be linked to each other over additional amino acid residues e.g. due to cloning reasons. Moreover, the peptide may be linked to the N- and/or C-terminus of the endolysin, fragment and/or derivative thereof by additional amino acid residues. Said additional amino acid residues may be at least 1, 2, 3, 4, 5, 6, 7, 8, 9 or 10 additional amino acid residues. In a preferred embodiment of the present invention the peptide is linked to the endolysin, fragment and/or derivative according to the present invention by the additional amino acid residues Gly-Ser, Gly-Gly-Ser or Gly-Ser-Gly. The additional amino acid residues linking the N- and/or C-terminus of the endolysin, fragment and/or derivative thereof and the peptide are preferably Gly-Ser-Gly. In case the fusion protein additionally comprises a tag, the fusion protein according to the present invention is preferably linked to said tag by the additional amino acid residues Leu-Glu or Lys-Gly. Preferably the peptide comprises the amino acid methionine (Met), methionine, glycine and serine (Met-Gly-Ser) or alanine, methionine or glycine (Ala-Met-Gly).

Especially preferred is a fusion protein comprising an endolysin, fragment and/or derivative according to the present invention and a peptide having an amino acid sequence according to SEQ ID NO: 12 at the N- and/or C-terminus of the endolysin, fragment and/or derivative according to the present invention. More preferred are fusion proteins having an amino acid sequence according to SEQ ID NO: 7 and SEQ ID NO: 9. Also preferred are fusion proteins having an amino acid sequence according to SEQ ID NO: 7 and SEQ ID NO: 9 with an additional His₆-tag linked to the C- and/or N-terminus of the fusion protein. The fusion proteins having an amino acid sequence according to SEQ ID NO: 7 and SEQ ID NO: 9, respectively, with an additional C-terminal and/or N-terminal His₆-tag linked to the C- and/or N-terminus of the fusion protein by the additional amino acid residues lysine and glycine (Lys-Gly).

In case the peptide is fused to the endolysin, fragment and/or derivative thereof according to the present invention at the C-Terminus, the fusion protein comprises the additional tag preferably at the N-terminus. In an especially preferred embodiment of the present invention the peptide is fused to the endolysin, fragment and/or derivative thereof according to the present invention at the N-Terminus the fusion protein comprises an additional tag preferably at the C-terminus.

Fusion proteins are constructed by linking at least two nucleic acid sequences using standard cloning techniques as described e.g. by Sambrook et al. 2001, Molecular Cloning: A Laboratory Manual. Such a protein may be produced, e.g., in recombinant DNA expression systems. Such fusion proteins according to the present invention can be obtained by fusing the nucleic acids for endolysin, fragment and/or derivative thereof and the respective peptide.

As some fusion proteins may either be toxic upon expression in bacteria, or not homogenous due to protein degradation, the strategy might be to express these fusion proteins fused or linked to other additional proteins. Example for these other additional protein is Thioredoxin, which was shown to mediate expression of toxic antimicrobial peptides in *E.coli* (TrxA mediating fusion expression of antimicrobial peptide CM4 from multiple joined genes in Escherichia coli. Zhou L, Zhao Z, Li B, Cai Y, Zhang S. Protein Expr Purif. 2009 Apr;64(2):225-230).

For antimicrobial function of the fusion proteins according to the present invention it may be necessary to remove the additional fusion protein by proteolytic cleavage. Commercially available kits like the pET32 expression system (Novagen), may need to modify e.g. the N-terminus of the fusion depending on the protease used, like from MGS to AMGS, were the remaining alanine residue results from an introduced Enterokinase cleavage site.

In a preferred embodiment of the present invention the fusion protein consists of a peptide according to SEQ ID NO: 11 to 34 and an endolysin according to SEQ ID NO: 1, 3, 4, 5 and/or 6. In a preferred embodiment the fusion protein comprises a peptide selected from the group of peptides according to SEQ ID NO: 11 to 34 and an endolysin selected from the group of endolysins according to SEQ ID NO: 1, 3, 4, 5 and/or 6.

The following table exemplifies the above outlined assembly of specifically preferred fusion proteins according to the present invention listed in the first column starting with the starting methionine residue at the N-terminus in the second column and ending with the optional tag at the C-terminus in the last column:

**Table 4:**

| **Fusion protein according to the present invention (SEQ ID NO:)** | **First amino acid residue (N-term)** | **additional amino acid residues** | **peptide stretch (SEQ ID NO:)** | **additional amino acid residues** | **polypeptide, fragment, derivative according to the present invention (SEQ ID NO:)** | **additional amino acid residues** | **tag (C-term)** |
|---|---|---|---|---|---|---|---|
| SEQ: 7 | | Ala-Met-Gly | SEQ: 12 | - | SEQ: 6 | - | - |
| SEQ: 9 | | Ala-Met-Gly | SEQ: 12 C-terminal | Gly-Ser-Gly | SEQ: 6 | Lys-Gly | - |

The present invention further relates to an isolated nucleic acid molecule encoding the fusion protein according to the present invention. Especially preferred isolated nucleic acid molecules according to the present invention comprise a nucleic acid sequence according to SEQ ID NO: 8 and 10. The present invention further relates to a vector comprising the nucleic acid molecule according to the present invention. Said vector may provide for the constitutive or inducible expression of said fusion protein according to the present invention.

The invention also relates to a method for obtaining said fusion proteins from a micro-organism, such as a genetically modified suitable host cell which expresses said fusion proteins. Said host cell may be a micro-organism such as bacteria or yeast or an animal cell as e.g. a mammalian cell, in particular a human cell. In one embodiment of the present invention the host cell is an *Escherichia coli* cell. The host may be selected due to mere biotechnological reasons, e.g. yield, solubility, costs, etc. but may be also selected from a medical point of view, e.g. a non-pathological bacteria or yeast or human cells. Another aspect of the present invention is related to a method for genetically transforming a suitable host cell in order to obtain the expression of the fusion proteins according to the present invention, wherein the host cell is genetically modified by the introduction of a genetic material encoding said fusion proteins into the host cell and obtain their translation and expression by genetic engineering methods well known by the man skilled in the art.

The fusion proteins may be obtained from a micro-organism, such as a genetically modified suitable host cell which expresses said fusion proteins. Said host cell may be a micro-organism such as bacteria or yeast or fungi or an animal cell as e.g. a mammalian cell, in particular a human cell. In one embodiment of the present invention the yeast cell is a *Pichia pastoris* cell. The host may be selected due to mere biotechnological reasons, e.g. yield, solubility, costs, etc. but may be also selected from a medical point of view, e.g. a non-pathological bacteria or yeast, human cells.

In a further aspect the present invention relates to a composition, preferably a pharmaceutical composition, comprising a fusion protein according to the present invention and/or a host transformed with a nucleic acid molecule or a vector comprising a nucleotide sequence encoding a fusion protein according to the present invention.

The present invention also relates to fusion protein according to the present invention and/or a host transformed with a nucleic acid comprising a nucleotide sequence encoding a fusion protein according to the present invention for use as a medicament. In a further aspect the present invention relates to the use of a fusion protein according to the present invention and/or a host transformed with a vector comprising a nucleic acid molecule comprising a nucleotide sequence encoding a fusion protein according to the present invention in the manufacture of a medicament for the treatment and/or prevention of a disorder, disease or condition associated with Gram-positive bacteria, preferably staphylococcal bacteria, more preferably *Staphylococcus aureus*. In particular the treatment and/or prevention of the disorder, disease or condition may be caused by Gram-positive bacteria of bacterial groups, families, genera or species comprising strains pathogenic for humans or animals like *Staphylococcus aureus*.

In another specific embodiment of the present invention the disorder, disease or condition is caused by *Staphylococcus aureus*, in particular infections of the skin, like pyoderma, particularly folliculitis, furuncle, carbuncle, abscesses of the sweat glands and pemphigus, and like scaled skin syndrome. The scaled skin syndrome can appear in three clinical pictures: dermatitis exfoliativa, impetigo bullosa and scarlatiniform erythroderma. Moreover the disorder, disease or condition caused by *Staphylococcus aureus* is *Staphylococcus* pneumonia, hospitalism, in particular surgical wound infections, mastitis puerperalis and enterokolitis, and food poisonings.

The present invention further relates to a medicament comprising a fusion protein according to the present invention and/or a host transformed with a nucleic acid comprising a nucleotide sequence encoding a fusion protein according to the present invention.

In a further aspect the present invention relates to a method of treating a disorder, disease or condition in a subject in need of treatment and/or prevention, which method comprises administering to said subject an effective amount of a fusion protein according to the present invention and/or an effective amount of a host transformed with a nucleic acid comprising a nucleotide sequence encoding a fusion protein according to the present invention or a composition according to the present invention. The subject may be a human or an animal.

In particular said method of treatment may be for the treatment and/or prevention of infections of the skin, of soft tissues, the respiratory system, the lung, the digestive tract, the eye, the ear, the teeth, the nasopharynx, the mouth, the bones, the vagina, of wounds of bacteraemia and/or endocarditis caused by staphylococcal bacteria, in particular by the staphylococcal bacteria as listed above.

The dosage and route of administration used in a method of treatment or prophylaxis according to the present invention depends on the specific disease/site of infection to be treated. The route of administration may be for example oral, topical, nasopharyngeal, parenteral, intravenous, rectal or any other route of administration.

For application of a fusion protein according to the present invention and/or an effective amount of a host transformed with a nucleic acid comprising a nucleotide sequence encoding a fusion protein according to the present invention or a composition according to the present invention to a site of infection or site endangered to be infected a formulation may be used that protects the active compounds from environmental influences such as proteases, oxidation, immune response etc., until it reaches the site of infection. Therefore, the formulation may be capsule, dragee, pill, powder, suppository, emulsion, suspension, gel, lotion, cream, salve, injectable solution, syrup, spray, inhalant or any other medical reasonable galenic formulation. Preferably, the galenic formulation may comprise suitable carriers, stabilizers, flavourings, buffers or other suitable reagents. For example, for topical application the formulation may be a lotion, cream, gel, salve or plaster, for nasopharyngeal application the formulation may be saline solution to be applied via a spray to the nose. For oral administration in case of the treatment and/or prevention of a specific infection site e.g. in the intestine, it can be necessary to protect a fusion protein according to the present invention from the harsh digestive environment of the gastrointestinal tract until the site of infection is reached. Thus, bacteria as carrier, which survive the initial steps of digestion in the stomach and which secret later on a fusion protein according to the present invention into the intestinal environment can be used.

Preferably, a fusion protein according to the present invention is used for medical treatment, if the infection to be treated or prevented is caused by multiresistant bacterial strains, in particular by strains resistant against one or more of the following antibiotics: streptomycin, tetracycline, cephalothin, gentamicin, cefotaxime, cephalosporin, ceftazidime or imipenem. Furthermore, a fusion protein according to the present invention can be used in methods of treatment by administering it in combination with or in addition to conventional antibacterial agents, such as antibiotics, lantibiotics, bacteriocins or endolysins.

The present invention also relates to a pharmaceutical pack comprising one or more compartments, wherein at least one compartment comprises one or more fusion protein according to the present invention and/or one or more hosts transformed with a nucleic acid comprising a nucleotide sequence encoding a fusion protein according to the present invention or a composition according to the present invention.

In another aspect the present invention relates to a process of preparation of a pharmaceutical composition, said process comprising admixing one or more fusion protein according to the present invention and/or one or more hosts transformed with a nucleic acid comprising a nucleotide sequence encoding a fusion protein according to the present invention with a pharmaceutically acceptable diluent, excipient or carrier.

In an even further aspect the composition according to the present invention is a cosmetic composition. Several bacterial species can cause irritations on environmentally exposed surfaces of the patient's body such as the skin. In order to prevent such irritations or in order to eliminate minor manifestations of said bacterial pathogens, special cosmetic preparations may be employed, which comprise sufficient amounts of the fusion protein according to the present invention in order to degrade already existing or freshly settling pathogenic Gram-positive, preferably staphylococcal bacteria, more preferably *Staphylococcus aureus*.

In a further aspect the present invention relates to fusion protein according to the present invention for use as diagnostic means in medicinal, food or feed or environmental diagnostics, in particular as a diagnostic means for the diagnostic of bacteria infection caused in particular by Gram-positive bacteria, preferably staphylococcal bacteria, more preferably *Staphylococcus aureus*. In this respect the fusion protein according to the present invention may be used as a tool to specifically degrade pathogenic bacteria, in particular Gram-positive pathogenic bacteria, preferably staphylococcal bacteria, more preferably *Staphylococcus aureus*. The degradation of the bacterial cells by the fusion protein according to the present invention can be supported by the addition of detergents like Triton X-100 or other additives which weaken the bacterial cell envelope like polymyxin B. Specific cell degradation is needed as an initial step for subsequent specific detection of bacteria using nucleic acid based methods like PCR, nucleic acid hybridization or NASBA (Nucleic Acid Sequence Based Amplification), immunological methods like IMS, immunofluorescence or ELISA techniques, or other methods relying on the cellular content of the bacterial cells like enzymatic assays using proteins specific for distinct bacterial groups or species (e.g. β-galactosidase for enterobacteria, coagulase for coagulase positive strains).

In a further aspect the present invention relates to the use of the fusion protein according to the present invention for the treatment or prevention of Gram-positive bacterial, preferably staphylococcal contamination of foodstuff, of food processing equipment, of food processing plants, of feed for livestock animals, of surfaces coming into contact with foodstuff such as shelves and food deposit areas and in all other situations, where pathogenic, facultative pathogenic or other undesirable bacteria can potentially infest food material, of medical devices and of all kind of surfaces in hospitals and surgeries.

In particular, a fusion protein of the present invention may be used prophylactically as sanitizing agent. Said sanitizing agent may be used before or after surgery, or for example during hemodialysis. Moreover, premature infants and immunocompromised persons, or those subjects with need for prosthetic devices may be treated with a fusion protein according to the present invention. Said treatment may be either prophylactically or during acute infection. In the same context, nosocomial infections, especially by antibiotic resistant strains like *Methicillin*-*resistant Staphyloccoccus aureus (MRSA), in particular MRSA from horse, pig and poultry*, *Methicillin-sensitive Staphyloccoccus aureus and Oxacillin-resistant Staphylococcus aureus (ORSA)* species may be treated prophylactically or during acute phase with a fusion protein of the present invention. Therefore, a fusion protein according to the present invention may be used as a disinfectant also in combination with other ingredients useful in a disinfecting solution like detergents, tensids, solvents, antibiotics, lantibiotics, or bacteriocins.

In a preferred embodiment a fusion protein according to the present invention can be used in methods of treatments by administering it in combination with at least one of the following antibiotics: β-lactams, aminoglycosides, fluoroquinolones, macrolides, novobiocin, rifampicin, oxazolidinones, fusidic acid, mupirocin, pleuromutilins, daptomycin, vancomycin, tetracyclines, sulfonamides, chloramphenicol, trimetoprim, fosfomycin, cycloserine and polymyxin.

In another preferred embodiment a fusion protein according to the present invention can be used in methods of eliminating, reducing or preventing bacterial biofilms of *Staphylococcus aureus* by administering it in combination with at least one of the following antibiotics: β-lactams, aminoglycosides, fluoroquinolones, macrolides, novobiocin, rifampicin, oxazolidinones, fusidic acid, mupirocin, pleuromutilins, daptomycin, vancomycin, tetracyclines, sulfonamides, chloramphenicol, trimetoprim, fosfomycin and cycloserine.

For the use of the fusion protein according to the present invention for eliminating, reducing or preventing bacterial biofilms as a disinfectant e.g. in hospital, dental surgery, veterinary, kitchen or bathroom, the fusion protein can be prepared in a composition in form of e.g. a fluid, a powder, a gel, or an ingredient of a wet wipe or a disinfection sheet product. Said composition may additionally comprise suitable carrier, additives, diluting agents and/or excipients for its respective use and form, respectively, - but also agents that support the antimicrobial activity like EDTA or agents enhance the antimicrobial activity of the fusion proteins. The fusion protein may also be used with common disinfectant agents like, Alcohols, Aldehydes, Oxidizing agents, Phenolics, Quaternary ammonium compounds or UV-light. For disinfecting for example surfaces, objects and/or devices the fusion protein can be applied on said surfaces, objects and/or devices. The application may occur for instance by wetting the disinfecting composition with any means such as a cloth or rag, by spraying, pouring. The fusion proteins may be used in varying concentration depending on the respective application and the "reaction time" intended to obtain full antimicrobial activity.

In a further aspect the present invention relates to the use of the fusion protein according to the present invention as a food additive or feed additive for livestock animals.

Further scope of applicability of the present invention will become apparent from the detailed description given hereinafter, however, it should be understood that the detailed description and specific examples, while indicating preferred embodiments of the invention, are given by way of illustration only, since various changes and modifications within the spirit and scope of the invention will become apparent to those skilled in the art from this detailed description. It is to be understood that both the foregoing general description and the following detailed description are exemplary and explanatory only and are not restrictive of the invention, as claimed.

The following examples explain the present invention but are not considered to be limiting. Unless indicated differently, molecular biological standard methods were used, as e.g., described by Sambrock et al., 1989, Molecular Cloning: A Laboratory Manual, 2nd edition, Cold Spring Harbor Laboratory Press, Cold Spring Harbor, New York.

### Example 1: Cloning of the fusion proteins of Staphylococcus aureus phage 2638A

Ply2638A having an amino acid sequence according to SEQ ID NO: 1 is a modular endolysin of 486 amino acid residues originating from *Staphylococcus aureus* phage 2638A with putative N-terminal peptidase/amidase domains and a C-terminal peptidoglycan binding domain.

Codon optimized and synthesized gene of Ply2638A was used as a template in standard PCR reaction with KAPAHiFi™ polymerase (PEQLAB, Erlangen, Germany) using the following PCR parameters and the primers are presented in the table below:

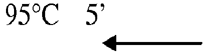

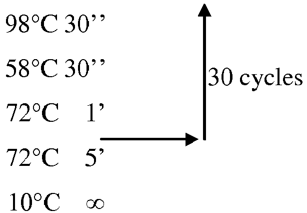

To extend the 5'- or 3'-end of Ply2638A with a fragment encoding the polycationic 9-mer peptide Lys-Arg-Lys-Lys-Arg-Lys-Lys-Arg-Lys (SEQ ID NO: 15) primers presented in table 5 were used for PCR. Ply2638A-PK additionally carries a Gly-Ser-Gly spacer between the Ply2638A and the polycationic peptide.

**Table 5: Primer sequences used for the cloning of the nucleic acids encoding the fusion proteins PK-Ply2638A and Ply2638A-PK**

| Primer | SEQ ID NO: | Nucleic acid sequence | Experiment |
|---|---|---|---|
| Ply2638A forward | SEQ ID NO: 35 | cat gcc atg ggc ctg acc gca att gat tac ctg acc | Amplification of the Ply2638A gene |
| Ply2638A reverse | SEQ ID NO: 36 | ccg ctc gag tca tca ttt gat ttc acc cca cag ttt gcc c | Amplification of the Ply2638A gene |
| PK-Ply2638A forward | SEQ ID NO: 37 | cat gcc atg ggc aag cgt aag aaa cgc aaa aaa cgc aaa ctg acc gca att gat tac ctg acc | Extension of the Ply2638A gene |
| PK-Ply2638A reverse | SEQ ID NO: 38 | ccg ctc gag tca tca ttt gat ttc acc cca cag ttt gcc c | Extension of the Ply2638A gene |
| Ply2638A-PK forward | SEQ ID NO: 39 | cat gcc atg ggc ctg acc gca att gat tac ctg acc | Extension of the Ply2638A gene |
| Ply2638A-PK reverse | SEQ ID NO: 40 | ccg ctc gag tca tca ttt gcg ttt ttt gcg ttt ctt acg ctt tcc cga ccc ttt gat ttc acc cca cag ttt gc | Extension of the Ply2638A gene |

Both extended fragments were digested with NcoI and Xhol and ligated in a linearized pET32b expression vector (Merck KGaA, Darmstadt, Germany) by following standard cloning protocols. By this way a fusion protein with thioredoxin, S-tag, His-tag and thrombin/enterokinase restriction sites (all comprised in the pET32b vector) was generated.

### Example 2: Purification of the fusion proteins of Staphylococcus aureus phage 2638A

Recombinant expression of proteins is performed in exponentially growing E. coli T7 Express lysY/Iq cells (New England Biolabs, Frankfurt.a.M., Germany) after induction with 0.5 mM IPTG (isopropylthiogalactoside) at 37°C for a period of 4 hours. Proteins were pre-purified by Ni²⁺ affinity chromatography (Äkta EXPLORER, GE Healthcare) using the His-tag, encoded by the pET32b expression vector. The Ni2+ affinity chromatography is performed in 4 subsequent steps, all at room temperature:
1. Equilibration of the HiTrap IMAC FF 5 ml column (GE Healthcare) with 5 column volumes of Washing Buffer (20 mM imidazole, 1M NaCl and 20 mM HEPES; pH 7.4) at a flow rate of 5 ml/min.
2. Loading of the total lysate (with wanted endolysin/artilysin) on the HiTrap IMAC FF column at a flow rate of 5 ml/min.
3. Washing of the column with 15 column volumes of Washing Buffer at a flow rate of 5 ml/min.
4. Elution of bound protein is done with a 4 column volume gradient to 100% of Elution Buffer (500 mM imidazole, 0.5 M NaCl and 20 mM HEPES; pH 7.4) at a flow rate of 5ml/min

After pre-purification the fused protein is digested overnight with recombinant enterokinase at room temperature. Ni²⁺ affinity chromatography is performed again, with repeating step 1 - 3 of the protocol with the target protein in the flowthrough collected in step 3 (His-tagged thioredoxin bound to the column).

### Example 3: Antimicrobial activity of the fusion protein of Ply2638A with polykationic stretches on the N-terminus and C-terminus

*Staphylococcus aureus* SP8, *Staphylococcus aureus* SP10, *Staphylococcus aureus* SP17, *Staphylococcus aureus* KS13, *Staphylococcus aureus* S53 (MRSA), *Staphylococcus aureus* AV4 (MRSA), *Staphylococcus aureus* 796N (MRSA) cells (Katholieke Universiteit Leuven, Belgium) as well as *Staphylococcus aureus* DSMZ 346 and *Staphylococcus intermedius* DSMZ 20373 (DSMZ, Braunschweig, Germany) were used as test strains.

Overnight cultures were diluted 10-fold in fresh LB medium and grown to OD600=0.6. The culture was spun down and diluted 10-fold in dilution buffer (10 mM HEPES, pH 7.4). Bacteria were incubated at room temperature with each 10 µg fusion protein at a final concentration of 100 µg/ml in buffer (20 mM HEPES 0.5 M NaCl; pH 7.4). After 1 hour cell dilution series were made in PBS and plated on LB. Additionally, a negative control was plated using buffer (20 mM HEPES 0.5 M NaCl; pH 7.4). The residual colonies were counted after an overnight incubation at 37°C. Based on the counted cell numbers the antibacterial activity as logarithmic units (=log10N₀/Nᵢ with No = number of untreated cells and Nᵢ = number of treated cells) was calculated. All samples were replicated at least in four fold.

**Table 6: Antimicrobial activity of the Ply2638 derivative modified with cationic peptide stretch against Staphylococcal bacteria**

| | PK-Ply2638A | Ply2638A-PK | Ply2638A | Ply2638A-PK | Ply2638A |
|---|---|---|---|---|---|
| *S*. *aureus* SP8 | + | +++ | - | 5 | - |
| *S*. *aureus* SP10 | + | +++ | - | >4 | - |
| *S*. *aureus* SP17 | + | +++ | - | 3 | 0,5 |
| *S. aureus* S53 (MRSDA) | n.d. | ++ | - | 3 | - |
| *S. aureus* AV4 (MRSDA) | n.d. | +++ | - | 4 | - |
| *S. aureus* 796N (MRSDA) | n.d. | +++ | - | 4 | - |
| *S. aureus* KS13 | n.d. | ++ | - | 3 | n.d. |
| *S. aureus* DSMZ 346 | - | ++ | - | 2 | - |
| *S. intermedius* DSMZ 20373 | n.d. | - | - | - | - |
| Staphylococcus aureus ATCC 6538 | + | ++ | n.d | 2 | n.d. |

| | | | | | |
|---|---|---|---|---|---|
| Abbreviations: -: no activity measured; +: 0.5 - 1 log; ++: 2-3 log; +++: 4 or more logs; n.d.: not determined; Reduction of cell numbers is displayed in logarithmic units | | | | | |

### EXAMPLE 4: Cloning, expression and purification of Ply2638A modified with various peptide stretches on the N-terminus or the C-terminus.

The endolysin Ply2638A is encoded by the nucleic acid sequence according to SEQ ID NO:2. The nucleic acid sequence according to SEQ ID NO:2 was synthetically produced with a BamH I (5'-GGA TCC-3') restriction site at the 5'-end of the nucleic acid sequence and an Xho I (5'-CTC GAG-3') restriction site at the 3'-end of the nucleic acid sequence.

The following peptide stretches were used for the generation of fusion proteins with Ply2638A: Pseudin 1 (SEQ ID NO:41), LL-37 (SEQ ID NO: 42), Indolicidin (SEQ ID NO: 43), Magainin 44 (SEQ ID NO:43), Pleurocidin (SEQ ID NO:44), Cecropin A (A. aegypti) (SEQ ID NO:45), Buforin II (SEQ ID NO:46), Sarcotoxin IA (SEQ ID NO:47), SMAP-29 (SEQ ID NO:49), OR-7 (SEQ ID NO:50).

The nucleic acid molecules encoding the respective peptide stretches were synthetically produced with a Nco I restriction site plus two additional nucleotides (5'-CCA TGG GC-3') at the 5'-end of the nucleic acid molecule and a BamH I (5'-GGA TCC-3') restriction site at the 3'-end of the nucleic acid molecule.

Fusion proteins were constructed by linking at least two nucleic acid sequences using standard cloning techniques as described e.g. by Sambrook et al. 2001, Molecular Cloning: A Laboratory Manual. Therefore the nucleic acid molecules encoding the peptide stretches were cleaved in a digest with the respective restriction enzymes Nco I and BamH I. Subsequently the cleaved nucleic acids encoding the peptide stretches were ligated into the pET32 b expression vector (Novagen, Darmstadt, Germany), which was also cleaved in a digest with the respective restriction enzymes Nco I and BamH I as described above. The modification of the pET32b expression vector refers to the deletion of the sequence encoding a S-tag and the central His-tag.

Subsequently, the nucleic acid molecule encoding the enzyme Ply2638A was cleaved in a digest with the restriction enzyme BamH I and Xho I and ligated into the the modified pET32 b expression vector.

The sequence of the endolysin-peptide-fusions was controlled via DNA-sequencing and correct clones were transformed into E.coli BL21(DE3) (Novagen, Darmstadt, Germany) for protein expression.

Recombinant expression of the fusion proteins was performed in *E. coli* BL21 (DE3) cells (Novagen, Darmstadt, Germany). The cells were growing until an optical density of OD600nm of 0.5-0.8 was reached. Then the expression of the fusion protein was induced with 1 mM IPTG (isopropylthiogalactoside) and the expression was performed at 37°C for a period of 4 hours.

E.coli BL21 cells were harvested by centrifugation for 20 min at 6000g and disrupted via sonication on ice. Soluble and insoluble fraction of the E.coli crude extract were separated by centrifugation (Sorvall, SS34, 30 min, 15 000 rpm). All proteins were purified by Ni2+ affinity chromatography (Akta FPLC, GE Healthcare) using the C-terminal 6xHis-tag, encoded by the modified pET32b vector (S-tag and central His-tag deleted), which fuses thioredoxin on the N-terminus of the proteins of interest. The vector also contains an enterokinase cleavage site (DDDDK) right before the protein of interest. This site allows the proteolytic cleavage between thioredoxin and the protein of interest, which can purified via the remaining C-terminal His-tag. Expressed fusion proteins were not toxic to the host resulting in high yields of produced protein. For antimicrobial function of the fusion protein it was necessary to remove the thioredoxin by proteolytic cleavage. Therefore the fusion protein was cleaved with 2-4 units/mg recombinant enterokinase (Novagen, Darmstadt, Germany) according to the manufactures instructions to remove the thioredoxin following the protocol provided by the manufacturer. After enterokinase cleavage the fusion protein was purified via His-tag purification as described below.

The Ni²⁺ affinity chromatography is performed in 4 subsequent steps, all at room temperature:
1. *Equilibration* of the *Histrap FF 5 ml* column (GE Healthcare) with up to 10 column volumes of Washing Buffer (20 mM imidazole, 1 M NaCl and 20 mM Hepes on pH 7.4) at a flow rate of 3-5 ml/min.
2. *Loading* of the total lysate (with wanted fusion protein) on the *Histrap FF 5 ml* column at a flow rate of 3-5 ml/min.
3. Washing of the column with up to 10 column volumes of Washing Buffer to remove unbound sample followed by a second washing step with 10% Elution buffer (500 mM imidazole, 0.5 M NaCl and 20 mM Hepes on pH 7.4) at a flow rate of 3-5 ml/min.
4. Elution of bounded fusion proteins from the column with a linear gradient of 4 column volumes of Elution Buffer (500 mM imidazole, 0.5 M NaCl and 20 mM Hepes on pH 7.4) to 100% at a flow rate of 3-5 ml/min.

Purified stock solutions of fusion proteins in Elution Buffer (20 mM Hepes pH 7.4; 0.5 M NaCl; 500 mM imidazole) were at least 90% pure as determined visually on SDS-PAGE gels.

### EXAMPLE 5: Antimicrobial activity of Ply2638A modified with various peptide stretches on the N-terminus or the C-terminus.

### Plating assay:

Exponentially growing cells of Staphylococci were taken (1 ml) harvested and resuspended in 10 mM HEPES (pH 7.4) and diluted 1:10 in 10 mM HEPES (pH7.4). Fusion proteins were diluted in 20 mM HEPES, 0.5 M NaCl (pH7.4) and incubated with the bacteria for 120 minutes at room temperature. Final concentration of the protein was about 100 µg/ml. After incubation serial dilutions (1:10) were made and bacteria were plated on appropriated agar plates. The residual colonies were counted after an overnight incubation at 37°C. Based on the counted cell numbers the antibacterial activity as logarithmic units (=log10N0/Ni with N0 = number of untreated cells and Ni = number of treated cells) was calculated (Table 4). All samples were replicated at least in four fold.

### Lysis test:

Staphylococcal cells of were grown in BHI medium until and optical density at 600nm of 0.7-1 was reached indicating exponential growth. Cells were harvested by centrifugation and resuspended in 20 mM HEPES (pH 7.4), 250 mM NaCl. Cells were resuspended at an optical density at 600nm of 1.0 and incubated with fusion proteins. Activity was measured spectrophotometrically at 600nm.

### Dot assay:

The dot assay was used test the antimicrobial activity of these fusion proteins against Staphylococcus aureus. Therefore, 200 µl overnight culture of Staphylococcus aureus, were mixed with 3 ml Top-Agar (LB with 0,75% Agar) plated and incubated until a solid agar layer is formed. Then 5 µl fusion protein solutions (ca. 500-800 µg/ml) were dotted. After overnight incubation under appropriate conditions formation of halos has to be analyzed.

### Results:

Six strains of Staphylococcus aureus were tested for their susceptibility to unmodified Ply2638A and Ply2638A-PK. Furthermore Ply2638A-PK was tested on 3 MRSA strains and Staphylococcus intermedius. Ply2638A shows no or only minor reduction in cell numbers compared to negative control without protein. Contrary to the endolysin the Artilysin shows good activity in plating assay using standard protocol (see above) with exception of Staphylococcus intermedius which was lysed neither by the endolysin nor by the Artilysin; see Table 6 above.

Table 7 below shows the results of the fusion proteins having the peptide stretch on the N-terminus of Ply2638A.

Table 8 below shows the results of the fusion proteins having the peptide stretch on the C-terminus of Ply2638A.

**Table 7**

| | LL-37-Ply2638A | SMAP-29-Ply2638A | Cecropin A-Ply2638A | Sarcotoxin IA-Ply2638A | OR-7-Ply2638A | Indolicidin-Ply2638A | Pseudin1-Ply2638A | Magainin-Ply2638A | Pleurocidin-Ply2638A | BuforinII-Ply2638A |
|---|---|---|---|---|---|---|---|---|---|---|
| *S. aureus* SP8 | + | ++ | - | - | + | + | - | + | + | - |
| *S. aureus* SP10 | + | + | + | - | - | + | - | + | - | - |
| *S. aureus* SP17 | - | + | + | - | + | + | - | + | + | + |
| *S. aureus* S53 | + | - | + | + | - | + | + | + | - | + |
| *S. aureus* AV4 | - | + | - | - | - | + | + | + | - | - |
| *S. aureus* 796N | + | + | - | - | - | + | - | + | + | - |
| *S. aureus* KS13 | + | + | - | - | + | + | - | - | + | + |
| *S. aureus* DSMZ 346 | - | + | - | - | + | + | - | - | - | - |
| *S. intermedius* DSMZ 20373 | - | - | - | - | - | - | - | - | - | - |

**Table 8**

| | Ply2638A-LL-37 | Ply2638A-SMAP-29 | Ply2638A-Cecropin A | Ply2638A-Sarcotoxin IA | Ply2638A-OR-7 | Ply2638A-Indolicidin | Ply2638A-Pseudin1 | Ply2638A-Magainin | Ply2638A-Pleurocidin | Ply2638A-BuforinII |
|---|---|---|---|---|---|---|---|---|---|---|
| *S. aureus* SP8 | ++ | ++ | ++ | +++ | +++ | +++ | +++ | +++ | +++ | ++ |
| *S. aureus* SP10 | ++ | +++ | ++ | +++ | ++ | +++ | ++ | + | ++ | ++ |
| *S. aureus* SP17 | ++ | +++ | ++ | +++ | + | +++ | +++ | +++ | ++ | + |
| *S. aureus* S53 | +++ | ++ | + | +++ | ++ | ++ | + | ++ | +++ | ++ |
| *S. aureus* AV4 | + | +++ | ++ | +++ | + | +++ | +++ | +++ | ++ | ++ |
| *S. aureus* 796N | ++ | + | ++ | +++ | ++ | ++ | ++ | ++ | ++ | + |
| *S. aureus* KS13 | ++ | +++ | ++ | ++ | + | ++ | +++ | ++ | +++ | + |
| *S. aureus* DSMZ 346 | ++ | ++ | +++ | ++ | + | +++ | ++ | ++ | +++ | + |
| *S. intermedius* DSMZ 20373 | - | - | - | - | - | - | - | - | - | - |

| | | | | | | | | | | |
|---|---|---|---|---|---|---|---|---|---|---|
| Abbreviations: -: no activity measured; +: 0.5 - 1 log; ++: 2-3 log; +++: 4 or more logs; n.d.: not determined | | | | | | | | | | |

Furthermore, the comparison of photometric lysis tests (using 30 µg/ml protein) showed that fusion proteins contrary to wild type Ply2638A have lytic activity on the test strain Staphylococcus aureus Sp17. With fusion proteins a well-defined clear cut spot appeared within 16h.

Some embodiments of the invention are exemplified in the following items 1 to 16.
1. A fusion protein comprising an endolysin having the amino acid sequence according to SEQ ID NO: 1, a fragment and/or derivative thereof and an additional cationic or polycationic peptide, an amphipatic peptide, a sushi peptide, a defensin, a hydrophobic peptide or an antimicrobial peptide fused to the N- and/or C-terminus of said endolysin, fragment and/or derivative thereof.
2. The fusion protein according to item 1, wherein the fragment comprises an amino acid sequence according to SEQ ID NO: 3, 4 and/or 5.
3. The fusion protein according to item 1 or 2, wherein the derivative has a deletion, addition, insertion and/or substitution in the amino acid sequence according to SEQ ID NO: 1, 3, 4, 5 and/or 6.
4. The fusion protein according to any one of the preceding items comprising additionally a tag, preferably a His₆-tag.
5. The fusion protein according to any one of the preceding items, wherein said cationic or polycationic peptide, amphipatic peptide, sushi peptide, defensin, hydrophobic peptide or antimicrobial peptide comprise about 5 to about 100 amino acid residues, in particular about 5 to 50 amino acid residues, in particular about 5 to 30 amino acid residues.
6. The fusion protein according to any one of the preceding items wherein said cationic and/or polycationic peptide comprises at least one amino acid residue selected out of the group consisting of arginine, histidine and lysine residues, in particular wherein at least 70% of the amino acid residues comprised in said peptide are arginine, histidine and/or lysine residues, in particular arginine and/or lysine residues.
7. The fusion protein according to any one of the preceding items wherein said cationic and/or polycationic peptide comprises an amino acid sequence according to SEQ ID NO: 11 to 34, said antimicrobial peptide comprises an amino acid sequence according to SEQ ID NO: 41 to 61.
8. The fusion protein according to item 1 having the amino acid sequence according to SEQ ID NO:66, 68, 70, 72, 74, 76, 78, 80, 82, 84, 86, 88, 90, 92, 94, 96, 98, 100, 102 or 104.
9. An isolated nucleic acid molecule encoding a fusion protein according to any one of items 1 to 8.
10. A vector comprising the nucleic acid molecule according to item 9.
11. A host cell comprising the nucleic acid molecule according to item 9 or the vector according to item 10.
12. The fusion protein according to any one of items 1 to 8 for use as human medical, veterinary medical or diagnostic substance, as an antimicrobial in food, feed additives for livestock animals, cosmetics, as disinfecting agent or in the environmental field.
13. The fusion protein according to any one of items 1 to 8 for use as a medicament for treatment or prevention of staphylococcal bacterial infections.
14. The use of the fusion protein according to any one of items 1 to 8 for the treatment or prevention of staphylococcal bacterial contamination of foodstuff, of feed for livestock animals, of food processing equipment, of food processing plants, of surfaces coming into contact with foodstuff, of medical devices, of surfaces in hospitals and surgeries.
15. Pharmaceutical composition comprising the fusion protein according to any one of items 1 to 8.
16. The fusion protein according to item 1 to 8 for use according to items 12 to 14, wherein said fusion protein is used in combination with or in addition to antibiotics.

## Claims

1. A fusion protein comprising a first amino acid sequence and a second or further amino acid sequences, wherein the first amino acid sequence is the endolysin Ply2638A according to SEQ ID NO:1, or a fragment and/or derivative thereof.

2. The fusion protein according to claim 1, wherein the fragment comprises an amino acid sequence according to SEQ ID NO: 3, 4 and/or 5.

3. The fusion protein according to claim 1 or 2, wherein the derivative has a deletion, addition, insertion and/or substitution in the amino acid sequence according to SEQ ID NO: 1, 3, 4, 5 and/or 6.

4. The fusion protein according to claim 3, wherein the derivative has 1, 2, 3, 4, or 5 amino acid residues deleted, 1, 2, 3, 4, or 5 amino acid residues inserted, 1, 2, 3, 4, or 5 amino acid residues added and/or an amino acid residue located at a certain position exchanged for a different one.

5. The fusion protein according to any one of the preceding claims, wherein the second or further amino acid sequence is a cationic and/or a polycationic peptide, an amphipatic peptide, a sushi peptide, a defensin, a hydrophobic peptide and/or an antimicrobial peptide.

6. The fusion protein according to any one of the preceding claims, wherein the second and/or further amino acid sequence is foreign to and not substantially homologous with any domain of the first amino acid sequence.

7. The fusion protein according to any one of the preceding claims wherein said cationic and/or polycationic peptide comprises at least one amino acid residue selected out of the group consisting of arginine, histidine and lysine residues, in particular wherein at least 70% of the amino acid residues comprised in said peptide are arginine, histidine and/or lysine residues, in particular arginine and/or lysine residues.

8. The fusion protein according to any one of the preceding claims wherein said cationic and/or polycationic peptide comprises an amino acid sequence according to SEQ ID NO: 11 to 34, said antimicrobial peptide comprises an amino acid sequence according to SEQ ID NO: 41 to 61.

9. An isolated nucleic acid molecule encoding a fusion protein according to any one of claims 1 to 8.

10. A vector comprising the nucleic acid molecule according to claim 9.

11. A host cell comprising the nucleic acid molecule according to claim 9 or the vector according to claim 10.

12. The fusion protein according to any one of claims 1 to 8 for use as human medical, veterinary medical or diagnostic substance, as an antimicrobial in food, feed additives for livestock animals, cosmetics, as disinfecting agent or in the environmental field.

13. The fusion protein according to any one of claims 1 to 8 for use in a method for treatment or prevention of staphylococcal bacterial infections.

14. The use of the fusion protein according to any one of claims 1 to 8 for the treatment or prevention of staphylococcal bacterial contamination of foodstuff, of feed for livestock animals, of food processing equipment, of food processing plants, of surfaces coming into contact with foodstuff, of medical devices, of surfaces in hospitals and surgeries.

15. Pharmaceutical composition comprising the fusion protein according to any one of claims 1 to 8.


SEQUENCE LISTING

<110>  LYSANDO AG

<120>  New Antimicrobial Agents

<130>  LYS-009 PCT/EP DIV1

<140>  unknown
<141>  2012-04-27

<150>  EP 11163897.9
<151>  2011-04-27

<160>  105

<170>  PatentIn version 3.3

<210>  1
<211>  486
<212>  PRT
<213>  unknown

<220>
<223>  Ply2638 A endolysin

<400>  1

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys
                485

<210>  2
<211>  1458
<212>  DNA
<213>  unknown

<220>
<223>  Ply2638A endolysin

<400>  2
atgctgaccg caattgatta cctgaccaaa aaaggctgga aaatctcttc cgatccgcgt      60

acctatgatg gctatccgaa aaactacggc taccgcaact accacgagaa cggtatcaac     120

tatgatgaat tttgcggcgg ctatcaccgt gcctttgatg tttactccaa cgaaacgaac     180

gatgtgccag cagttactag cggtactgtt atcgaggcga acgattacgg taacttcggc     240

ggtactttcg ttatccgcga cgcgaacgac aacgattgga tctatggcca cctgcagcgc     300

ggttccatgc gttttgttgt tggcgacaaa gtgaaccagg gcgacattat tggtctgcag     360

ggtaacagca actattacga caacccgatg agcgttcacc tgcatctgca gctgcgtccg     420

aaagacgcca aaaaagacga aaaaagccag gtttgctctg gtctggcgat ggagaaatac     480

gatatcacca acctgaacgc gaaacaggac aaaagcaaaa acggctctgt caaagaactg     540

aaacacatct actctaacca cattaaaggt aacaaaatca ccgctccgaa accgtctatt     600

cagggtgtcg tgatccacaa cgattacggt tccatgactc caagccagta cctcccgtgg     660

ctgtacgctc gtgaaaacaa cggtactcac gttaacggct gggcatctgt atacgccaac     720

cgcaacgaag tactgtggta ccacccgact gattatgttg aatggcattg cggtaaccag     780

tgggcaaacg cgaacctgat tggtttcgaa gtctgtgaat cttacccggg tcgtatttct     840

gacaaactgt tcctggagaa cgaagaggct actctcaaag tggctgcaga cgtgatgaaa 900

agctacggtc tgccagtaaa ccgtaacacc gttcgcctgc ataacgaatt tttcggcacc 960

tcctgtccgc accgttcttg ggatctgcac gttggtaaag gcgaaccgta caccactacg 1020

aacatcaaca aaatgaaaga ctacttcatc aaacgtatca aacactacta tgatggcggc 1080

aaactggaag tatctaaagc tgcgacgatc aaacagtccg acgtcaaaca ggaggtaaaa 1140

aaacaggagg cgaaacagat tgttaaagcc accgactgga aacagaacaa agacggcatc 1200

tggtacaaag ctgaacacgc ttctttcacc gtgactgcac cagagggcat tatcacgcgt 1260

tataaaggtc cgtggaccgg tcatccacag gctggtgtgc tgcagaaagg tcagacgatc 1320

aaatatgacg aggttcagaa attcgacggt catgtatggg tatcttggga aacgtttgaa 1380

ggtgaaacgg tctatatgcc ggtgcgcact tgggatgcga aaaccggtaa agtgggcaaa 1440

ctgtggggtg aaatcaaa 1458

<210> 3
<211> 68
<212> PRT
<213> unknown

<220>
<223> peptidoglycan binding domain of Ply2638A endolysin

<400> 3

Tyr Lys Ala Glu His Ala Ser Phe Thr Val Thr Ala Pro Glu Gly Ile
1               5                   10                  15

Ile Thr Arg Tyr Lys Gly Pro Trp Thr Gly His Pro Gln Ala Gly Val
            20                  25                  30

Leu Gln Lys Gly Gln Thr Ile Lys Tyr Asp Glu Val Gln Lys Phe Asp
        35                  40                  45

Gly His Val Trp Val Ser Trp Glu Thr Phe Glu Gly Glu Thr Val Tyr
    50                  55                  60

Met Pro Val Arg
65

<210> 4
<211> 100
<212> PRT
<213> unknown

<220>
<223> M23 peptidase domain of Ply2638A endolysin

<400> 4

Tyr His Arg Ala Phe Asp Val Tyr Ser Asn Glu Thr Asn Asp Val Pro
1               5                   10                  15

Ala Val Thr Ser Gly Thr Val Ile Glu Ala Asn Asp Tyr Gly Asn Phe
            20                  25                  30

Gly Gly Thr Phe Val Ile Arg Asp Ala Asn Asp Asn Asp Trp Ile Tyr
        35                  40                  45

Gly His Leu Gln Arg Gly Ser Met Arg Phe Val Val Gly Asp Lys Val
    50                  55                  60

Asn Gln Gly Asp Ile Ile Gly Leu Gln Gly Asn Ser Asn Tyr Tyr Asp
65                  70                  75                  80

Asn Pro Met Ser Val His Leu His Leu Gln Leu Arg Pro Lys Asp Ala
                85                  90                  95

Lys Lys Asp Glu
            100

<210>  5
<211>  127
<212>  PRT
<213>  unknown

<220>
<223>  putative amidase domain of Ply2638A endolysin

<400>  5

Ile Gln Gly Val Val Ile His Asn Asp Tyr Gly Ser Met Thr Pro Ser
1               5                   10                  15

Gln Tyr Leu Pro Trp Leu Tyr Ala Arg Glu Asn Asn Gly Thr His Val
            20                  25                  30

Asn Gly Trp Ala Ser Val Tyr Ala Asn Arg Asn Glu Val Leu Trp Tyr
        35                  40                  45

His Pro Thr Asp Tyr Val Glu Trp His Cys Gly Asn Gln Trp Ala Asn
    50                  55                  60

Ala Asn Leu Ile Gly Phe Glu Val Cys Glu Ser Tyr Pro Gly Arg Ile
65                  70                  75                  80

Ser Asp Lys Leu Phe Leu Glu Asn Glu Glu Ala Thr Leu Lys Val Ala
                85                  90                  95

Ala Asp Val Met Lys Ser Tyr Gly Leu Pro Val Asn Arg Asn Thr Val

100                 105                 110

Arg Leu His Asn Glu Phe Phe Gly Thr Ser Cys Pro His Arg Ser
        115                 120                 125

<210>  6
<211>  485
<212>  PRT
<213>  unknown

<220>
<223>  Ply2638A endolysin without Met

<400>  6

Leu Thr Ala Ile Asp Tyr Leu Thr Lys Lys Gly Trp Lys Ile Ser Ser
1               5                   10                  15

Asp Pro Arg Thr Tyr Asp Gly Tyr Pro Lys Asn Tyr Gly Tyr Arg Asn
            20                  25                  30

Tyr His Glu Asn Gly Ile Asn Tyr Asp Glu Phe Cys Gly Gly Tyr His
        35                  40                  45

Arg Ala Phe Asp Val Tyr Ser Asn Glu Thr Asn Asp Val Pro Ala Val
    50                  55                  60

Thr Ser Gly Thr Val Ile Glu Ala Asn Asp Tyr Gly Asn Phe Gly Gly
65                  70                  75                  80

Thr Phe Val Ile Arg Asp Ala Asn Asp Asn Asp Trp Ile Tyr Gly His
                85                  90                  95

Leu Gln Arg Gly Ser Met Arg Phe Val Val Gly Asp Lys Val Asn Gln
            100                 105                 110

Gly Asp Ile Ile Gly Leu Gln Gly Asn Ser Asn Tyr Tyr Asp Asn Pro
        115                 120                 125

Met Ser Val His Leu His Leu Gln Leu Arg Pro Lys Asp Ala Lys Lys
    130                 135                 140

Asp Glu Lys Ser Gln Val Cys Ser Gly Leu Ala Met Glu Lys Tyr Asp
145                 150                 155                 160

Ile Thr Asn Leu Asn Ala Lys Gln Asp Lys Ser Lys Asn Gly Ser Val
                165                 170                 175

Lys Glu Leu Lys His Ile Tyr Ser Asn His Ile Lys Gly Asn Lys Ile
            180                 185                 190

Thr Ala Pro Lys Pro Ser Ile Gln Gly Val Val Ile His Asn Asp Tyr
        195                 200                 205

Gly Ser Met Thr Pro Ser Gln Tyr Leu Pro Trp Leu Tyr Ala Arg Glu
    210                 215                 220

Asn Asn Gly Thr His Val Asn Gly Trp Ala Ser Val Tyr Ala Asn Arg
225                 230                 235                 240

Asn Glu Val Leu Trp Tyr His Pro Thr Asp Tyr Val Glu Trp His Cys
                245                 250                 255

Gly Asn Gln Trp Ala Asn Ala Asn Leu Ile Gly Phe Glu Val Cys Glu
            260                 265                 270

Ser Tyr Pro Gly Arg Ile Ser Asp Lys Leu Phe Leu Glu Asn Glu Glu
        275                 280                 285

Ala Thr Leu Lys Val Ala Ala Asp Val Met Lys Ser Tyr Gly Leu Pro
    290                 295                 300

Val Asn Arg Asn Thr Val Arg Leu His Asn Glu Phe Phe Gly Thr Ser
305                 310                 315                 320

Cys Pro His Arg Ser Trp Asp Leu His Val Gly Lys Gly Glu Pro Tyr
                325                 330                 335

Thr Thr Thr Asn Ile Asn Lys Met Lys Asp Tyr Phe Ile Lys Arg Ile
            340                 345                 350

Lys His Tyr Tyr Asp Gly Gly Lys Leu Glu Val Ser Lys Ala Ala Thr
        355                 360                 365

Ile Lys Gln Ser Asp Val Lys Gln Glu Val Lys Lys Gln Glu Ala Lys
    370                 375                 380

Gln Ile Val Lys Ala Thr Asp Trp Lys Gln Asn Lys Asp Gly Ile Trp
385                 390                 395                 400

Tyr Lys Ala Glu His Ala Ser Phe Thr Val Thr Ala Pro Glu Gly Ile
                405                 410                 415

Ile Thr Arg Tyr Lys Gly Pro Trp Thr Gly His Pro Gln Ala Gly Val
            420                 425                 430

Leu Gln Lys Gly Gln Thr Ile Lys Tyr Asp Glu Val Gln Lys Phe Asp

435                 440                 445

Gly His Val Trp Val Ser Trp Glu Thr Phe Glu Gly Glu Thr Val Tyr
    450                 455                 460

Met Pro Val Arg Thr Trp Asp Ala Lys Thr Gly Lys Val Gly Lys Leu
465                 470                 475                 480

Trp Gly Glu Ile Lys
                485

<210>  7
<211>  497
<212>  PRT
<213>  unknown

<220>
<223>  PK-Ply2638A

<400>  7

Ala Met Gly Lys Arg Lys Lys Arg Lys Lys Arg Lys Leu Thr Ala Ile
1               5                   10                  15

Asp Tyr Leu Thr Lys Lys Gly Trp Lys Ile Ser Ser Asp Pro Arg Thr
            20                  25                  30

Tyr Asp Gly Tyr Pro Lys Asn Tyr Gly Tyr Arg Asn Tyr His Glu Asn
        35                  40                  45

Gly Ile Asn Tyr Asp Glu Phe Cys Gly Gly Tyr His Arg Ala Phe Asp
    50                  55                  60

Val Tyr Ser Asn Glu Thr Asn Asp Val Pro Ala Val Thr Ser Gly Thr
65                  70                  75                  80

Val Ile Glu Ala Asn Asp Tyr Gly Asn Phe Gly Gly Thr Phe Val Ile
                85                  90                  95

Arg Asp Ala Asn Asp Asn Asp Trp Ile Tyr Gly His Leu Gln Arg Gly
            100                 105                 110

Ser Met Arg Phe Val Val Gly Asp Lys Val Asn Gln Gly Asp Ile Ile
        115                 120                 125

Gly Leu Gln Gly Asn Ser Asn Tyr Tyr Asp Asn Pro Met Ser Val His
    130                 135                 140

Leu His Leu Gln Leu Arg Pro Lys Asp Ala Lys Lys Asp Glu Lys Ser
145                 150                 155                 160

Gln Val Cys Ser Gly Leu Ala Met Glu Lys Tyr Asp Ile Thr Asn Leu
                165                 170                 175

Asn Ala Lys Gln Asp Lys Ser Lys Asn Gly Ser Val Lys Glu Leu Lys
            180                 185                 190

His Ile Tyr Ser Asn His Ile Lys Gly Asn Lys Ile Thr Ala Pro Lys
        195                 200                 205

Pro Ser Ile Gln Gly Val Val Ile His Asn Asp Tyr Gly Ser Met Thr
    210                 215                 220

Pro Ser Gln Tyr Leu Pro Trp Leu Tyr Ala Arg Glu Asn Asn Gly Thr
225                 230                 235                 240

His Val Asn Gly Trp Ala Ser Val Tyr Ala Asn Arg Asn Glu Val Leu
                245                 250                 255

Trp Tyr His Pro Thr Asp Tyr Val Glu Trp His Cys Gly Asn Gln Trp
            260                 265                 270

Ala Asn Ala Asn Leu Ile Gly Phe Glu Val Cys Glu Ser Tyr Pro Gly
        275                 280                 285

Arg Ile Ser Asp Lys Leu Phe Leu Glu Asn Glu Glu Ala Thr Leu Lys
    290                 295                 300

Val Ala Ala Asp Val Met Lys Ser Tyr Gly Leu Pro Val Asn Arg Asn
305                 310                 315                 320

Thr Val Arg Leu His Asn Glu Phe Phe Gly Thr Ser Cys Pro His Arg
                325                 330                 335

Ser Trp Asp Leu His Val Gly Lys Gly Glu Pro Tyr Thr Thr Thr Asn
            340                 345                 350

Ile Asn Lys Met Lys Asp Tyr Phe Ile Lys Arg Ile Lys His Tyr Tyr
        355                 360                 365

Asp Gly Gly Lys Leu Glu Val Ser Lys Ala Ala Thr Ile Lys Gln Ser
    370                 375                 380

Asp Val Lys Gln Glu Val Lys Lys Gln Glu Ala Lys Gln Ile Val Lys
385                 390                 395                 400

Ala Thr Asp Trp Lys Gln Asn Lys Asp Gly Ile Trp Tyr Lys Ala Glu

405                 410                 415

His Ala Ser Phe Thr Val Thr Ala Pro Glu Gly Ile Ile Thr Arg Tyr
            420                 425                 430

Lys Gly Pro Trp Thr Gly His Pro Gln Ala Gly Val Leu Gln Lys Gly
        435                 440                 445

Gln Thr Ile Lys Tyr Asp Glu Val Gln Lys Phe Asp Gly His Val Trp
    450                 455                 460

Val Ser Trp Glu Thr Phe Glu Gly Glu Thr Val Tyr Met Pro Val Arg
465                 470                 475                 480

Thr Trp Asp Ala Lys Thr Gly Lys Val Gly Lys Leu Trp Gly Glu Ile
                485                 490                 495

Lys

<210>  8
<211>  1491
<212>  DNA
<213>  unknown

<220>
<223>  PK-Ply2638A

<400>  8
gccatgggca agcgtaagaa acgcaaaaaa cgcaaactga ccgcaattga ttacctgacc      60

aaaaaaggct ggaaaatctc ttccgatccg cgtacctatg atggctatcc gaaaaactac     120

ggctaccgca actaccacga gaacggtatc aactatgatg aattttgcgg cggctatcac     180

cgtgcctttg atgtttactc caacgaaacg aacgatgtgc cagcagttac tagcggtact     240

gttatcgagg cgaacgatta cggtaacttc ggcggtactt tcgttatccg cgacgcgaac     300

gacaacgatt ggatctatgg ccacctgcag cgcggttcca tgcgttttgt tgttggcgac     360

aaagtgaacc agggcgacat tattggtctg cagggtaaca gcaactatta cgacaacccg     420

atgagcgttc acctgcatct gcagctgcgt ccgaaagacg ccaaaaaaga cgaaaaaagc     480

caggtttgct ctggtctggc gatggagaaa tacgatatca ccaacctgaa cgcgaaacag     540

gacaaaagca aaaacggctc tgtcaaagaa ctgaaacaca tctactctaa ccacattaaa     600

ggtaacaaaa tcaccgctcc gaaaccgtct attcagggtg tcgtgatcca caacgattac     660

ggttccatga ctccaagcca gtacctcccg tggctgtacg ctcgtgaaaa caacggtact     720

cacgttaacg gctgggcatc tgtatacgcc aaccgcaacg aagtactgtg gtaccacccg     780

actgattatg ttgaatggca ttgcggtaac cagtgggcaa acgcgaacct gattggtttc     840

gaagtctgtg aatcttaccc gggtcgtatt tctgacaaac tgttcctgga gaacgaagag 900

gctactctca aagtggctgc agacgtgatg aaaagctacg gtctgccagt aaaccgtaac 960

accgttcgcc tgcataacga atttttcggc acctcctgtc cgcaccgttc ttgggatctg 1020

cacgttggta aaggcgaacc gtacaccact acgaacatca acaaaatgaa agactacttc 1080

atcaaacgta tcaaacacta ctatgatggc ggcaaactgg aagtatctaa agctgcgacg 1140

atcaaacagt ccgacgtcaa acaggaggta aaaaaacagg aggcgaaaca gattgttaaa 1200

gccaccgact ggaaacagaa caaagacggc atctggtaca aagctgaaca cgcttctttc 1260

accgtgactg caccagaggg cattatcacg cgttataaag gtccgtggac cggtcatcca 1320

caggctggtg tgctgcagaa aggtcagacg atcaaatatg acgaggttca gaaattcgac 1380

ggtcatgtat gggtatcttg ggaaacgttt gaaggtgaaa cggtctatat gccggtgcgc 1440

acttgggatg cgaaaaccgg taaagtgggc aaactgtggg gtgaaatcaa a 1491

<210> 9
<211> 500
<212> PRT
<213> unknown

<220>
<223> Ply2638A-PK

<400> 9

Ala Met Gly Leu Thr Ala Ile Asp Tyr Leu Thr Lys Lys Gly Trp Lys
1               5                   10                  15

Ile Ser Ser Asp Pro Arg Thr Tyr Asp Gly Tyr Pro Lys Asn Tyr Gly
            20                  25                  30

Tyr Arg Asn Tyr His Glu Asn Gly Ile Asn Tyr Asp Glu Phe Cys Gly
        35                  40                  45

Gly Tyr His Arg Ala Phe Asp Val Tyr Ser Asn Glu Thr Asn Asp Val
    50                  55                  60

Pro Ala Val Thr Ser Gly Thr Val Ile Glu Ala Asn Asp Tyr Gly Asn
65                  70                  75                  80

Phe Gly Gly Thr Phe Val Ile Arg Asp Ala Asn Asp Asn Asp Trp Ile
                85                  90                  95

Tyr Gly His Leu Gln Arg Gly Ser Met Arg Phe Val Val Gly Asp Lys
            100                 105                 110

Val Asn Gln Gly Asp Ile Ile Gly Leu Gln Gly Asn Ser Asn Tyr Tyr

        115                 120                 125

Asp Asn Pro Met Ser Val His Leu His Leu Gln Leu Arg Pro Lys Asp
    130                 135                 140

Ala Lys Lys Asp Glu Lys Ser Gln Val Cys Ser Gly Leu Ala Met Glu
145                 150                 155                 160

Lys Tyr Asp Ile Thr Asn Leu Asn Ala Lys Gln Asp Lys Ser Lys Asn
                165                 170                 175

Gly Ser Val Lys Glu Leu Lys His Ile Tyr Ser Asn His Ile Lys Gly
            180                 185                 190

Asn Lys Ile Thr Ala Pro Lys Pro Ser Ile Gln Gly Val Val Ile His
        195                 200                 205

Asn Asp Tyr Gly Ser Met Thr Pro Ser Gln Tyr Leu Pro Trp Leu Tyr
    210                 215                 220

Ala Arg Glu Asn Asn Gly Thr His Val Asn Gly Trp Ala Ser Val Tyr
225                 230                 235                 240

Ala Asn Arg Asn Glu Val Leu Trp Tyr His Pro Thr Asp Tyr Val Glu
                245                 250                 255

Trp His Cys Gly Asn Gln Trp Ala Asn Ala Asn Leu Ile Gly Phe Glu
            260                 265                 270

Val Cys Glu Ser Tyr Pro Gly Arg Ile Ser Asp Lys Leu Phe Leu Glu
        275                 280                 285

Asn Glu Glu Ala Thr Leu Lys Val Ala Ala Asp Val Met Lys Ser Tyr
    290                 295                 300

Gly Leu Pro Val Asn Arg Asn Thr Val Arg Leu His Asn Glu Phe Phe
305                 310                 315                 320

Gly Thr Ser Cys Pro His Arg Ser Trp Asp Leu His Val Gly Lys Gly
                325                 330                 335

Glu Pro Tyr Thr Thr Thr Asn Ile Asn Lys Met Lys Asp Tyr Phe Ile
            340                 345                 350

Lys Arg Ile Lys His Tyr Tyr Asp Gly Gly Lys Leu Glu Val Ser Lys
        355                 360                 365

Ala Ala Thr Ile Lys Gln Ser Asp Val Lys Gln Glu Val Lys Lys Gln
    370                 375                 380

Glu Ala Lys Gln Ile Val Lys Ala Thr Asp Trp Lys Gln Asn Lys Asp
385                 390                 395                 400

Gly Ile Trp Tyr Lys Ala Glu His Ala Ser Phe Thr Val Thr Ala Pro
                405                 410                 415

Glu Gly Ile Ile Thr Arg Tyr Lys Gly Pro Trp Thr Gly His Pro Gln
            420                 425                 430

Ala Gly Val Leu Gln Lys Gly Gln Thr Ile Lys Tyr Asp Glu Val Gln
        435                 440                 445

Lys Phe Asp Gly His Val Trp Val Ser Trp Glu Thr Phe Glu Gly Glu
    450                 455                 460

Thr Val Tyr Met Pro Val Arg Thr Trp Asp Ala Lys Thr Gly Lys Val
465                 470                 475                 480

Gly Lys Leu Trp Gly Glu Ile Lys Gly Ser Gly Lys Arg Lys Lys Arg
                485                 490                 495

Lys Lys Arg Lys
            500

<210>  10
<211>  1500
<212>  DNA
<213>  unknown

<220>
<223>  Ply2638A-PK

<400>  10
gccatgggcc tgaccgcaat tgattacctg accaaaaaag gctggaaaat ctcttccgat      60

ccgcgtacct atgatggcta tccgaaaaac tacggctacc gcaactacca cgagaacggt     120

atcaactatg atgaattttg cggcggctat caccgtgcct ttgatgttta ctccaacgaa     180

acgaacgatg tgccagcagt tactagcggt actgttatcg aggcgaacga ttacggtaac     240

ttcggcggta ctttcgttat ccgcgacgcg aacgacaacg attggatcta tggccacctg     300

cagcgcggtt ccatgcgttt tgttgttggc gacaaagtga accagggcga cattattggt     360

ctgcagggta acagcaacta ttacgacaac ccgatgagcg ttcacctgca tctgcagctg     420

cgtccgaaag acgccaaaaa agacgaaaaa agccaggttt gctctggtct ggcgatggag     480

aaatacgata tcaccaacct gaacgcgaaa caggacaaaa gcaaaaacgg ctctgtcaaa     540

gaactgaaac acatctactc taaccacatt aaaggtaaca aaatcaccgc tccgaaaccg 600

tctattcagg gtgtcgtgat ccacaacgat tacggttcca tgactccaag ccagtacctc 660

ccgtggctgt acgctcgtga aaacaacggt actcacgtta acggctgggc atctgtatac 720

gccaaccgca acgaagtact gtggtaccac ccgactgatt atgttgaatg gcattgcggt 780

aaccagtggg caaacgcgaa cctgattggt ttcgaagtct gtgaatctta cccgggtcgt 840

atttctgaca aactgttcct ggagaacgaa gaggctactc tcaaagtggc tgcagacgtg 900

atgaaaagct acggtctgcc agtaaaccgt aacaccgttc gcctgcataa cgaatttttc 960

ggcacctcct gtccgcaccg ttcttgggat ctgcacgttg gtaaaggcga accgtacacc 1020

actacgaaca tcaacaaaat gaaagactac ttcatcaaac gtatcaaaca ctactatgat 1080

ggcggcaaac tggaagtatc taaagctgcg acgatcaaac agtccgacgt caaacaggag 1140

gtaaaaaaac aggaggcgaa acagattgtt aaagccaccg actggaaaca gaacaaagac 1200

ggcatctggt acaaagctga acacgcttct ttcaccgtga ctgcaccaga gggcattatc 1260

acgcgttata aaggtccgtg gaccggtcat ccacaggctg gtgtgctgca gaaaggtcag 1320

acgatcaaat atgacgaggt tcagaaattc gacggtcatg tatgggtatc ttgggaaacg 1380

tttgaaggtg aaacggtcta tatgccggtg cgcacttggg atgcgaaaac cggtaaagtg 1440

ggcaaactgt ggggtgaaat caaagggtcg ggaaagcgta agaaacgcaa aaaacgcaaa 1500

<210> 11
<211> 6
<212> PRT
<213> unknown

<220>
<223> synthetic peptide

<400> 11

Lys Arg Lys Lys Arg Lys
1               5

<210> 12
<211> 9
<212> PRT
<213> unknown

<220>
<223> synthetic peptide

<400> 12

Lys Arg Lys Lys Arg Lys Lys Arg Lys
1               5

<210> 13
<211> 9

<212>  PRT
<213>  unknown

<220>
<223>  synthetic peptide

<400>  13

Arg Arg Arg Arg Arg Arg Arg Arg Arg
1               5


<210>  14
<211>  8
<212>  PRT
<213>  unknown

<220>
<223>  synthetic peptide

<400>  14

Lys Lys Lys Lys Lys Lys Lys Lys
1               5


<210>  15
<211>  10
<212>  PRT
<213>  unknown

<220>
<223>  synthetic peptide

<400>  15

Lys Arg Lys Lys Arg Lys Lys Arg Lys Lys
1               5                   10


<210>  16
<211>  12
<212>  PRT
<213>  unknown

<220>
<223>  synthetic peptide

<400>  16

Lys Arg Lys Lys Arg Lys Lys Arg Lys Lys Arg Lys
1               5                   10


<210>  17
<211>  14
<212>  PRT
<213>  unknown

<220>
<223>  synthetic peptide

<400>  17

Lys Arg Lys Lys Arg Lys Lys Arg Lys Lys Arg Lys Lys Arg
1               5                   10

<210>  18
<211>  16
<212>  PRT
<213>  unknown

<220>
<223>  synthetic peptide

<400>  18

Lys Lys Lys Lys Lys Lys Lys Lys Lys Lys Lys Lys Lys Lys Lys Lys
1               5                   10                  15

<210>  19
<211>  19
<212>  PRT
<213>  unknown

<220>
<223>  synthetic peptide

<400>  19

Lys Arg Lys Lys Arg Lys Lys Arg Lys Lys Arg Lys Lys Arg Lys Lys
1               5                   10                  15

Arg Lys Lys

<210>  20
<211>  19
<212>  PRT
<213>  unknown

<220>
<223>  synthetic peptide

<400>  20

Arg Arg Arg Arg Arg Arg Arg Arg Arg Arg Arg Arg Arg Arg Arg Arg
1               5                   10                  15

Arg Arg Arg

<210>  21
<211>  19
<212>  PRT
<213>  unknown

<220>
<223>  synthetic peptide

<400>  21

Lys Lys Lys Lys Lys Lys Lys Lys Lys Lys Lys Lys Lys Lys Lys Lys
1               5                   10                  15

Lys Lys Lys

<210>  22
<211>  20
<212>  PRT
<213>  unknown

<220>
<223>  synthetic peptide

<400>  22

Lys Arg Lys Lys Arg Lys Lys Arg Lys Arg Ser Lys Arg Lys Lys Arg
1               5                   10                  15

Lys Lys Arg Lys
            20

<210>  23
<211>  21
<212>  PRT
<213>  unknown

<220>
<223>  synthetic peptide

<400>  23

Lys Arg Lys Lys Arg Lys Lys Arg Lys Arg Ser Lys Arg Lys Lys Arg
1               5                   10                  15

Lys Lys Arg Lys Lys
            20

<210>  24
<211>  21
<212>  PRT
<213>  unknown

<220>
<223>  synthetic peptide

<400>  24

Lys Arg Lys Lys Arg Lys Lys Arg Lys Lys Arg Lys Lys Arg Lys Lys
1               5                   10                  15

Arg Lys Lys Arg Lys
            20

<210>  25
<211>  22
<212>  PRT
<213>  unknown

<220>
<223>  synthetic peptide

<400>  25

Lys Arg Lys Lys Arg Lys Lys Arg Lys Arg Gly Ser Gly Lys Arg Lys
1               5                   10                  15

Lys Arg Lys Lys Arg Lys
            20

<210>  26
<211>  24
<212>  PRT
<213>  unknown

<220>
<223>  synthetic peptide

<400>  26

Lys Arg Lys Lys Arg Lys Lys Arg Lys Arg Gly Ser Gly Ser Gly Lys
1               5                   10                  15

Arg Lys Lys Arg Lys Lys Arg Lys
            20

<210>  27
<211>  25
<212>  PRT
<213>  unknown

<220>
<223>  synthetic peptide

<400>  27

Lys Arg Lys Lys Arg Lys Lys Arg Lys Lys Arg Lys Lys Arg Lys Lys
1               5                   10                  15

Arg Lys Lys Arg Lys Lys Arg Lys Lys
            20                  25

<210>  28
<211>  31
<212>  PRT
<213>  unknown

<220>
<223>  synthetic peptide

<400>  28

Lys Arg Lys Lys Arg Lys Lys Arg Lys Arg Ser Lys Arg Lys Lys Arg
1               5                   10                  15

Lys Lys Arg Lys Arg Ser Lys Arg Lys Lys Arg Lys Lys Arg Lys
            20                  25                  30

<210>  29
<211>  38
<212>  PRT
<213>  unknown

<220>
<223>  synthetic peptide

<400>  29

Lys Arg Lys Lys Arg Lys Lys Arg Lys Arg Gly Ser Gly Ser Gly Lys
1               5                   10                  15

Arg Lys Lys Arg Lys Lys Arg Lys Gly Ser Gly Ser Gly Lys Arg Lys
            20                  25                  30

Lys Arg Lys Lys Arg Lys
        35

<210>  30
<211>  39
<212>  PRT
<213>  unknown

<220>
<223>  synthetic peptide

<400>  30

Lys Arg Lys Lys Arg Lys Lys Arg Lys Lys Arg Lys Lys Arg Lys Lys
1               5                   10                  15

Arg Lys Lys Arg Lys Lys Arg Lys Lys Arg Lys Lys Arg Lys Lys Arg
            20                  25                  30

Lys Lys Arg Lys Lys Arg Lys
        35

<210>  31
<211>  42
<212>  PRT
<213>  unknown

<220>
<223>  synthetic peptide

<400>  31

Lys Arg Lys Lys Arg Lys Lys Arg Lys Arg Ser Lys Arg Lys Lys Arg
1               5                   10                  15

Lys Lys Arg Lys Arg Ser Lys Arg Lys Lys Arg Lys Lys Arg Lys Arg
            20                  25                  30

Ser Lys Arg Lys Lys Arg Lys Lys Arg Lys
        35                  40

<210>  32
<211>  5
<212>  PRT
<213>  unknown

<220>
<223>  synthetic peptide

<220>
<221>  misc_feature
<222>  (3)..(3)
<223>  wherein Xaa is any other amino acid residue than lysine, arginine
       and histidine

<400>  32

Lys Arg Xaa Lys Arg
1               5

<210>  33
<211>  5
<212>  PRT
<213>  unknown

<220>
<223>  synthetic peptide

<400>  33

Lys Arg Ser Lys Arg
1               5

<210>  34
<211>  5
<212>  PRT
<213>  unknown

<220>
<223>  synthetic peptide

<400>  34

Lys Arg Gly Ser Gly
1               5

<210>  35

<211>  36
<212>  DNA
<213>  unknown

<220>
<223>  Ply2638A forward primer

<400>  35
catgccatgg gcctgaccgc aattgattac ctgacc                                36


<210>  36
<211>  40
<212>  DNA
<213>  unknown

<220>
<223>  Ply2638A reverse primer

<400>  36
ccgctcgagt catcatttga tttcacccca cagtttgccc                            40


<210>  37
<211>  63
<212>  DNA
<213>  unknown

<220>
<223>  PK-Ply2638A forward primer

<400>  37
catgccatgg gcaagcgtaa gaaacgcaaa aaacgcaaac tgaccgcaat tgattacctg      60

acc                                                                    63


<210>  38
<211>  40
<212>  DNA
<213>  unknown

<220>
<223>  PK-Ply2638A reverse primer

<400>  38
ccgctcgagt catcatttga tttcacccca cagtttgccc                            40


<210>  39
<211>  36
<212>  DNA
<213>  unknown

<220>
<223>  Ply2638A-PK forward primer

<400>  39
catgccatgg gcctgaccgc aattgattac ctgacc                                36


<210>  40
<211>  74

<212>  DNA
<213>  unknown

<220>
<223>  Ply2638A-PK reverse primer

<400>  40
ccgctcgagt catcatttgc gttttttgcg tttcttacgc tttcccgacc ctttgatttc     60

accccacagt ttgc                                                        74


<210>  41
<211>  24
<212>  PRT
<213>  unknown

<220>
<223>  Pseudin 1

<400>  41

Gly Leu Asn Thr Leu Lys Lys Val Phe Gln Gly Leu His Glu Ala Ile
1               5                   10                  15


Lys Leu Ile Asn Asn His Val Gln
            20


<210>  42
<211>  37
<212>  PRT
<213>  unknown

<220>
<223>  LL-37

<400>  42

Leu Leu Gly Asp Phe Phe Arg Lys Ser Lys Glu Lys Ile Gly Lys Glu
1               5                   10                  15


Phe Lys Arg Ile Val Gln Arg Ile Lys Asp Phe Leu Arg Asn Leu Val
            20                  25                  30


Pro Arg Thr Glu Ser
        35


<210>  43
<211>  13
<212>  PRT
<213>  unknown

<220>
<223>  Indolicidin

<400>  43

Ile Leu Pro Trp Lys Trp Pro Trp Trp Pro Trp Arg Arg

1               5                   10


<210>  44
<211>  23
<212>  PRT
<213>  unknown

<220>
<223>  Magainin

<400>  44

Gly Ile Gly Lys Phe Leu His Ser Ala Lys Lys Phe Gly Lys Ala Phe
1               5                   10                  15


Val Gly Glu Ile Met Asn Ser
            20


<210>  45
<211>  25
<212>  PRT
<213>  unknown

<220>
<223>  Pleurocidin

<400>  45

Gly Trp Gly Ser Phe Phe Lys Lys Ala Ala His Val Gly Lys His Val
1               5                   10                  15


Gly Lys Ala Ala Leu Thr His Tyr Leu
            20                  25


<210>  46
<211>  36
<212>  PRT
<213>  unknown

<220>
<223>  Cecropin A (A.aegypti)

<400>  46

Gly Gly Leu Lys Lys Leu Gly Lys Lys Leu Glu Gly Ala Gly Lys Arg
1               5                   10                  15


Val Phe Asn Ala Ala Glu Lys Ala Leu Pro Val Val Ala Gly Ala Lys
            20                  25                  30


Ala Leu Arg Lys
        35


<210>  47
<211>  21

<212>  PRT
<213>  unknown

<220>
<223>  Buforin II

<400>  47

Thr Arg Ser Ser Arg Ala Gly Leu Gln Phe Pro Val Gly Arg Val His
1               5                   10                  15

Arg Leu Leu Arg Lys
            20

<210>  48
<211>  39
<212>  PRT
<213>  unknown

<220>
<223>  Sarcotoxin IA

<400>  48

Gly Trp Leu Lys Lys Ile Gly Lys Lys Ile Glu Arg Val Gly Gln His
1               5                   10                  15

Thr Arg Asp Ala Thr Ile Gln Gly Leu Gly Ile Ala Gln Gln Ala Ala
            20                  25                  30

Asn Val Ala Ala Thr Ala Arg
        35

<210>  49
<211>  29
<212>  PRT
<213>  unknown

<220>
<223>  SMAP-29

<400>  49

Arg Gly Leu Arg Arg Leu Gly Arg Lys Ile Ala His Gly Val Lys Lys
1               5                   10                  15

Tyr Gly Pro Thr Val Leu Arg Ile Ile Arg Ile Ala Gly
            20                  25

<210>  50
<211>  54
<212>  PRT
<213>  unknown

<220>
<223>  OR-7

<400>  50

Lys Thr Tyr Tyr Gly Thr Asn Gly Val His Cys Thr Lys Asn Ser Leu
1               5                   10                  15

Trp Gly Lys Val Arg Leu Lys Asn Met Lys Tyr Asp Gln Asn Thr Thr
            20                  25                  30

Tyr Met Gly Arg Leu Gln Asp Ile Leu Leu Gly Trp Ala Thr Gly Ala
        35                  40                  45

Phe Gly Lys Thr Phe His
    50

<210>  51
<211>  18
<212>  PRT
<213>  unknown

<220>
<223>  Protegrin

<400>  51

Arg Gly Gly Arg Leu Cys Tyr Cys Arg Arg Arg Phe Cys Val Cys Val
1               5                   10                  15

Gly Arg

<210>  52
<211>  31
<212>  PRT
<213>  unknown

<220>
<223>  Cecropin P1

<400>  52

Ser Trp Leu Ser Lys Thr Ala Lys Lys Leu Glu Asn Ser Ala Lys Lys
1               5                   10                  15

Arg Ile Ser Glu Gly Ile Ala Ile Ala Ile Gln Gly Gly Pro Arg
            20                  25                  30

<210>  53
<211>  40
<212>  PRT
<213>  unknown

<220>
<223>  Cecropin A (D. melanogaster)

<400>  53

Gly Trp Leu Lys Lys Ile Gly Lys Lys Ile Glu Arg Val Gly Gln His
1               5                   10                  15

Thr Arg Asp Ala Thr Ile Gln Gly Leu Gly Ile Pro Gln Gln Ala Ala
            20                  25                  30

Asn Val Ala Ala Thr Ala Arg Gly
        35                  40

<210>  54
<211>  24
<212>  PRT
<213>  unknown

<220>
<223>  Ascaphine

<400>  54

Gly Ile Lys Asp Trp Ile Lys Gly Ala Ala Lys Lys Leu Ile Lys Thr
1               5                   10                  15

Val Ala Ser His Ile Ala Asn Gln
            20

<210>  55
<211>  17
<212>  PRT
<213>  unknown

<220>
<223>  Apidaecine

<400>  55

Ala Asn Arg Pro Val Tyr Ile Pro Pro Pro Arg Pro Pro His Pro Arg
1               5                   10                  15

Leu

<210>  56
<211>  22
<212>  PRT
<213>  unknown

<220>
<223>  Nigrocine

<400>  56

Gly Leu Leu Ser Lys Val Leu Gly Val Gly Lys Lys Val Leu Cys Gly
1               5                   10                  15

Val Ser Gly Leu Val Cys
            20

<210>  57
<211>  19
<212>  PRT
<213>  unknown

<220>
<223>  Parasin 1

<400>  57

Lys Gly Arg Gly Lys Gln Gly Gly Lys Val Arg Ala Lys Ala Lys Thr
1               5                   10                  15

Arg Ser Ser

<210>  58
<211>  25
<212>  PRT
<213>  unknown

<220>
<223>  Lycotoxin

<400>  58

Ile Trp Leu Thr Ala Leu Lys Phe Leu Gly Lys His Ala Ala Lys Lys
1               5                   10                  15

Leu Ala Lys Gln Gln Leu Ser Lys Leu
            20                  25

<210>  59
<211>  18
<212>  PRT
<213>  unknown

<220>
<223>  Ranalexin

<400>  59

Phe Leu Gly Gly Leu Ile Val Pro Ala Met Ile Cys Ala Val Thr Lys
1               5                   10                  15

Lys Cys

<210>  60
<211>  26
<212>  PRT
<213>  unknown

<220>
<223>  Melittin

<400>  60

Gly Ile Gly Ala Val Leu Lys Val Leu Thr Thr Gly Leu Pro Ala Leu
1               5                   10                  15

Ile Ser Trp Ile Lys Arg Lys Arg Gln Gln
            20                  25

<210>  61
<211>  38
<212>  PRT
<213>  unknown

<220>
<223>  Buforin I

<400>  61

Gly Arg Gly Lys Gln Gly Gly Lys Val Arg Ala Lys Ala Lys Thr Arg
1               5                   10                  15

Ser Ser Arg Ala Gly Leu Gln Phe Pro Val Gly Arg Val His Arg Leu
            20                  25                  30

Leu Arg Lys Gly Asn Tyr
        35

<210>  62
<211>  13
<212>  PRT
<213>  unknown

<220>
<223>  Alpha 4

<400>  62

Pro Asn Arg Ala Lys Arg Val Ile Thr Thr Phe Arg Thr
1               5                   10

<210>  63
<211>  18
<212>  PRT
<213>  unknown

<220>
<223>  Artilysin1

<400>  63

Gly Phe Phe Ile Pro Ala Val Ile Leu Pro Ser Ile Ala Phe Leu Ile
1               5                   10                  15

Val Pro

<210> 64
<211> 25
<212> PRT
<213> unknown

<220>
<223> Artilysin2

<400> 64

Gly Lys Pro Gly Trp Leu Ile Lys Lys Ala Leu Val Phe Lys Lys Leu
1               5                   10                  15

Ile Arg Arg Pro Leu Lys Arg Leu Ala
            20                  25

<210> 65
<211> 27
<212> PRT
<213> unknown

<220>
<223> WLBU2 variant

<400> 65

Lys Arg Trp Val Lys Arg Val Lys Arg Val Lys Arg Trp Val Lys Arg
1               5                   10                  15

Val Val Arg Val Val Lys Arg Trp Val Lys Arg
            20                  25

<210> 66
<211> 526
<212> PRT
<213> artificial

<220>
<223> LL-37-Ply2638A

<400> 66

Met Leu Leu Gly Asp Phe Phe Arg Lys Ser Lys Glu Lys Ile Gly Lys
1               5                   10                  15

Glu Phe Lys Arg Ile Val Gln Arg Ile Lys Asp Phe Leu Arg Asn Leu
            20                  25                  30

Val Pro Arg Thr Glu Ser Gly Ser Met Leu Thr Ala Ile Asp Tyr Leu
        35                  40                  45

Thr Lys Lys Gly Trp Lys Ile Ser Ser Asp Pro Arg Thr Tyr Asp Gly
    50                  55                  60

Tyr Pro Lys Asn Tyr Gly Tyr Arg Asn Tyr His Glu Asn Gly Ile Asn
65                  70                  75                  80

Tyr Asp Glu Phe Cys Gly Gly Tyr His Arg Ala Phe Asp Val Tyr Ser
                85                  90                  95

Asn Glu Thr Asn Asp Val Pro Ala Val Thr Ser Gly Thr Val Ile Glu
            100                 105                 110

Ala Asn Asp Tyr Gly Asn Phe Gly Gly Thr Phe Val Ile Arg Asp Ala
        115                 120                 125

Asn Asp Asn Asp Trp Ile Tyr Gly His Leu Gln Arg Gly Ser Met Arg
    130                 135                 140

Phe Val Val Gly Asp Lys Val Asn Gln Gly Asp Ile Ile Gly Leu Gln
145                 150                 155                 160

Gly Asn Ser Asn Tyr Tyr Asp Asn Pro Met Ser Val His Leu His Leu
                165                 170                 175

Gln Leu Arg Pro Lys Asp Ala Lys Lys Asp Glu Lys Ser Gln Val Cys
            180                 185                 190

Ser Gly Leu Ala Met Glu Lys Tyr Asp Ile Thr Asn Leu Asn Ala Lys
        195                 200                 205

Gln Asp Lys Ser Lys Asn Gly Ser Val Lys Glu Leu Lys His Ile Tyr
    210                 215                 220

Ser Asn His Ile Lys Gly Asn Lys Ile Thr Ala Pro Lys Pro Ser Ile
225                 230                 235                 240

Gln Gly Val Val Ile His Asn Asp Tyr Gly Ser Met Thr Pro Ser Gln
                245                 250                 255

Tyr Leu Pro Trp Leu Tyr Ala Arg Glu Asn Asn Gly Thr His Val Asn
            260                 265                 270

Gly Trp Ala Ser Val Tyr Ala Asn Arg Asn Glu Val Leu Trp Tyr His
        275                 280                 285

Pro Thr Asp Tyr Val Glu Trp His Cys Gly Asn Gln Trp Ala Asn Ala
    290                 295                 300

Asn Leu Ile Gly Phe Glu Val Cys Glu Ser Tyr Pro Gly Arg Ile Ser
305                 310                 315                 320

Asp Lys Leu Phe Leu Glu Asn Glu Glu Ala Thr Leu Lys Val Ala Ala
                325                 330                 335

Asp Val Met Lys Ser Tyr Gly Leu Pro Val Asn Arg Asn Thr Val Arg
            340                 345                 350

Leu His Asn Glu Phe Phe Gly Thr Ser Cys Pro His Arg Ser Trp Asp
        355                 360                 365

Leu His Val Gly Lys Gly Glu Pro Tyr Thr Thr Thr Asn Ile Asn Lys
    370                 375                 380

Met Lys Asp Tyr Phe Ile Lys Arg Ile Lys His Tyr Tyr Asp Gly Gly
385                 390                 395                 400

Lys Leu Glu Val Ser Lys Ala Ala Thr Ile Lys Gln Ser Asp Val Lys
                405                 410                 415

Gln Glu Val Lys Lys Gln Glu Ala Lys Gln Ile Val Lys Ala Thr Asp
            420                 425                 430

Trp Lys Gln Asn Lys Asp Gly Ile Trp Tyr Lys Ala Glu His Ala Ser
        435                 440                 445

Phe Thr Val Thr Ala Pro Glu Gly Ile Ile Thr Arg Tyr Lys Gly Pro
    450                 455                 460

Trp Thr Gly His Pro Gln Ala Gly Val Leu Gln Lys Gly Gln Thr Ile
465                 470                 475                 480

Lys Tyr Asp Glu Val Gln Lys Phe Asp Gly His Val Trp Val Ser Trp
                485                 490                 495

Glu Thr Phe Glu Gly Glu Thr Val Tyr Met Pro Val Arg Thr Trp Asp
            500                 505                 510

Ala Lys Thr Gly Lys Val Gly Lys Leu Trp Gly Glu Ile Lys
        515                 520                 525

<210>  67
<211>  1581
<212>  DNA
<213>  artificial

<220>

<223>  LL-37-Ply2638A

<400>  67
atgctcctgg gtgacttctt tcgcaaatcc aaagagaaaa tcggcaaaga gttcaaacgt      60

atcgtgcagc gcattaaaga ctttctgcgt aacctggttc cgcgtaccga atctggatcc     120

atgctaactg ctattgacta tcttacgaaa aaaggttgga aaatatcatc tgaccctcgc     180

acttacgatg gttaccctaa aaactacggc tacagaaatt accatgaaaa cggcattaat     240

tatgatgagt tttgtggtgg ttatcataga gcttttgatg tttacagtaa cgaaactaac     300

gacgtgcctg ctgttactag cggaacagtt attgaagcaa acgattacgg taattttggt     360

ggtacattcg ttattagaga cgctaacgat aacgattgga tatatgggca tctacaacgt     420

ggctcaatgc gatttgttgt aggcgacaaa gtcaatcaag gtgacattat tggtttacaa     480

ggtaatagca actattacga caatcctatg agtgtacatt tacatttaca attacgccct     540

aaagacgcaa agaaagatga aaaatcacaa gtatgtagtg gtttggctat ggaaaaatat     600

gacattacaa atttaaatgc taaacaagat aaatcaaaga atgggagcgt gaaagagttg     660

aaacatatct attcaaacca tattaaaggt aacaagatta cagcaccaaa acctagtatt     720

caaggtgtgg tcatccacaa tgattatggt agtatgacac ctagtcaata cttaccatgg     780

ttatatgcac gtgagaataa cggtacacac gttaacggtt gggctagtgt ttatgcaaat     840

agaaacgaag tgctttggta tcatccgaca gactacgtag agtggcattg tggtaatcaa     900

tgggcaaatg ctaacttaat cggatttgaa gtgtgtgagt cgtatcctgg tagaatctcg     960

gacaaattat tcttagaaaa tgaagaagcg acattgaaag tagctgcgga tgtgatgaag    1020

tcgtacggat taccagttaa tcgcaacact gtacgtctgc ataacgaatt cttcggaact    1080

tcttgtccac atcgttcgtg ggacttgcat gttggcaaag gtgagcctta cacaactact    1140

aatattaata aaatgaaaga ctacttcatc aaacgcatca aacattatta tgacggtgga    1200

aagctagaag taagcaaagc agcaactatc aaacaatctg acgttaagca agaagttaaa    1260

aagcaagaag caaaacaaat tgtgaaagca acagattgga aacagaataa agatggcatt    1320

tggtataaag ctgaacatgc ttcgttcaca gtgacagcac cagagggaat tatcacaaga    1380

tacaaaggtc cttggactgg tcacccacaa gctggtgtat tacaaaaagg tcaaacgatt    1440

aaatatgatg aggttcaaaa atttgacggt catgtttggg tatcgtggga aacgtttgag    1500

ggcgaaactg tatacatgcc ggtacgcaca tgggacgcta aaactggtaa agttggtaag    1560

ttgtggggcg aaattaaatg a                                              1581

<210>  68
<211>  518
<212>  PRT
<213>  artificial

<220>
<223>  SMAP-29-Ply2638A

<400>  68

Met Arg Gly Leu Arg Arg Leu Gly Arg Lys Ile Ala His Gly Val Lys
1               5                   10                  15

Lys Tyr Gly Pro Thr Val Leu Arg Ile Ile Arg Ile Ala Gly Gly Ser
            20                  25                  30

Met Leu Thr Ala Ile Asp Tyr Leu Thr Lys Lys Gly Trp Lys Ile Ser
        35                  40                  45

Ser Asp Pro Arg Thr Tyr Asp Gly Tyr Pro Lys Asn Tyr Gly Tyr Arg
    50                  55                  60

Asn Tyr His Glu Asn Gly Ile Asn Tyr Asp Glu Phe Cys Gly Gly Tyr
65                  70                  75                  80

His Arg Ala Phe Asp Val Tyr Ser Asn Glu Thr Asn Asp Val Pro Ala
                85                  90                  95

Val Thr Ser Gly Thr Val Ile Glu Ala Asn Asp Tyr Gly Asn Phe Gly
            100                 105                 110

Gly Thr Phe Val Ile Arg Asp Ala Asn Asp Asn Asp Trp Ile Tyr Gly
        115                 120                 125

His Leu Gln Arg Gly Ser Met Arg Phe Val Val Gly Asp Lys Val Asn
    130                 135                 140

Gln Gly Asp Ile Ile Gly Leu Gln Gly Asn Ser Asn Tyr Tyr Asp Asn
145                 150                 155                 160

Pro Met Ser Val His Leu His Leu Gln Leu Arg Pro Lys Asp Ala Lys
                165                 170                 175

Lys Asp Glu Lys Ser Gln Val Cys Ser Gly Leu Ala Met Glu Lys Tyr
            180                 185                 190

Asp Ile Thr Asn Leu Asn Ala Lys Gln Asp Lys Ser Lys Asn Gly Ser
        195                 200                 205

Val Lys Glu Leu Lys His Ile Tyr Ser Asn His Ile Lys Gly Asn Lys
    210                 215                 220

Ile Thr Ala Pro Lys Pro Ser Ile Gln Gly Val Val Ile His Asn Asp
225                 230                 235                 240

Tyr Gly Ser Met Thr Pro Ser Gln Tyr Leu Pro Trp Leu Tyr Ala Arg
                245                 250                 255

Glu Asn Asn Gly Thr His Val Asn Gly Trp Ala Ser Val Tyr Ala Asn
            260                 265                 270

Arg Asn Glu Val Leu Trp Tyr His Pro Thr Asp Tyr Val Glu Trp His
        275                 280                 285

Cys Gly Asn Gln Trp Ala Asn Ala Asn Leu Ile Gly Phe Glu Val Cys
    290                 295                 300

Glu Ser Tyr Pro Gly Arg Ile Ser Asp Lys Leu Phe Leu Glu Asn Glu
305                 310                 315                 320

Glu Ala Thr Leu Lys Val Ala Ala Asp Val Met Lys Ser Tyr Gly Leu
                325                 330                 335

Pro Val Asn Arg Asn Thr Val Arg Leu His Asn Glu Phe Phe Gly Thr
            340                 345                 350

Ser Cys Pro His Arg Ser Trp Asp Leu His Val Gly Lys Gly Glu Pro
        355                 360                 365

Tyr Thr Thr Thr Asn Ile Asn Lys Met Lys Asp Tyr Phe Ile Lys Arg
    370                 375                 380

Ile Lys His Tyr Tyr Asp Gly Gly Lys Leu Glu Val Ser Lys Ala Ala
385                 390                 395                 400

Thr Ile Lys Gln Ser Asp Val Lys Gln Glu Val Lys Lys Gln Glu Ala
                405                 410                 415

Lys Gln Ile Val Lys Ala Thr Asp Trp Lys Gln Asn Lys Asp Gly Ile
            420                 425                 430

Trp Tyr Lys Ala Glu His Ala Ser Phe Thr Val Thr Ala Pro Glu Gly
        435                 440                 445

Ile Ile Thr Arg Tyr Lys Gly Pro Trp Thr Gly His Pro Gln Ala Gly
    450                 455                 460

Val Leu Gln Lys Gly Gln Thr Ile Lys Tyr Asp Glu Val Gln Lys Phe
465                 470                 475                 480

Asp Gly His Val Trp Val Ser Trp Glu Thr Phe Glu Gly Glu Thr Val

485                 490                 495

Tyr Met Pro Val Arg Thr Trp Asp Ala Lys Thr Gly Lys Val Gly Lys
            500                 505                 510

Leu Trp Gly Glu Ile Lys
        515

<210>  69
<211>  1557
<212>  DNA
<213>  artificial

<220>
<223>  SMAP-29-Ply2638A

<400>  69
atgcgtggtc tgcgtcgcct gggtcgcaaa attgcgcacg gcgtcaaaaa atacggcccg      60

accgtgctgc gcattatccg catcgctggt ggatccatgc taactgctat tgactatctt     120

acgaaaaaag gttggaaaat atcatctgac cctcgcactt acgatggtta ccctaaaaac     180

tacggctaca gaaattacca tgaaaacggc attaattatg atgagttttg tggtggttat     240

catagagctt ttgatgttta cagtaacgaa actaacgacg tgcctgctgt tactagcgga     300

acagttattg aagcaaacga ttacggtaat tttggtggta cattcgttat tagagacgct     360

aacgataacg attggatata tgggcatcta caacgtggct caatgcgatt tgttgtaggc     420

gacaaagtca atcaaggtga cattattggt ttacaaggta atagcaacta ttacgacaat     480

cctatgagtg tacatttaca tttacaatta cgccctaaag acgcaaagaa agatgaaaaa     540

tcacaagtat gtagtggttt ggctatggaa aaatatgaca ttacaaattt aaatgctaaa     600

caagataaat caaagaatgg gagcgtgaaa gagttgaaac atatctattc aaaccatatt     660

aaaggtaaca agattacagc accaaaacct agtattcaag gtgtggtcat ccacaatgat     720

tatggtagta tgacacctag tcaatactta ccatggttat atgcacgtga gaataacggt     780

acacacgtta acggttgggc tagtgtttat gcaaatagaa acgaagtgct ttggtatcat     840

ccgacagact acgtagagtg gcattgtggt aatcaatggg caaatgctaa cttaatcgga     900

tttgaagtgt gtgagtcgta tcctggtaga atctcggaca aattattctt agaaaatgaa     960

gaagcgacat tgaaagtagc tgcggatgtg atgaagtcgt acggattacc agttaatcgc    1020

aacactgtac gtctgcataa cgaattcttc ggaacttctt gtccacatcg ttcgtgggac    1080

ttgcatgttg gcaaaggtga gccttacaca actactaata ttaataaaat gaaagactac    1140

ttcatcaaac gcatcaaaca ttattatgac ggtggaaagc tagaagtaag caaagcagca    1200

actatcaaac aatctgacgt taagcaagaa gttaaaaagc aagaagcaaa acaaattgtg    1260

aaagcaacag attggaaaca gaataaagat ggcatttggt ataaagctga acatgcttcg    1320

ttcacagtga cagcaccaga gggaattatc acaagataca aaggtccttg gactggtcac 1380

ccacaagctg gtgtattaca aaaaggtcaa acgattaaat atgatgaggt tcaaaaattt 1440

gacggtcatg tttgggtatc gtgggaaacg tttgagggcg aaactgtata catgccggta 1500

cgcacatggg acgctaaaac tggtaaagtt ggtaagttgt ggggcgaaat taaatga 1557

<210> 70
<211> 525
<212> PRT
<213> artificial

<220>
<223> Cecropin A (A.aegypti)-Ply2638A

<400> 70

Met Gly Gly Leu Lys Lys Leu Gly Lys Lys Leu Glu Gly Ala Gly Lys
1               5                   10                  15

Arg Val Phe Asn Ala Ala Glu Lys Ala Leu Pro Val Val Ala Gly Ala
            20                  25                  30

Lys Ala Leu Arg Lys Gly Ser Met Leu Thr Ala Ile Asp Tyr Leu Thr
        35                  40                  45

Lys Lys Gly Trp Lys Ile Ser Ser Asp Pro Arg Thr Tyr Asp Gly Tyr
    50                  55                  60

Pro Lys Asn Tyr Gly Tyr Arg Asn Tyr His Glu Asn Gly Ile Asn Tyr
65                  70                  75                  80

Asp Glu Phe Cys Gly Gly Tyr His Arg Ala Phe Asp Val Tyr Ser Asn
                85                  90                  95

Glu Thr Asn Asp Val Pro Ala Val Thr Ser Gly Thr Val Ile Glu Ala
            100                 105                 110

Asn Asp Tyr Gly Asn Phe Gly Gly Thr Phe Val Ile Arg Asp Ala Asn
        115                 120                 125

Asp Asn Asp Trp Ile Tyr Gly His Leu Gln Arg Gly Ser Met Arg Phe
    130                 135                 140

Val Val Gly Asp Lys Val Asn Gln Gly Asp Ile Ile Gly Leu Gln Gly
145                 150                 155                 160

Asn Ser Asn Tyr Tyr Asp Asn Pro Met Ser Val His Leu His Leu Gln
                165                 170                 175

Leu Arg Pro Lys Asp Ala Lys Lys Asp Glu Lys Ser Gln Val Cys Ser
            180                 185                 190

Gly Leu Ala Met Glu Lys Tyr Asp Ile Thr Asn Leu Asn Ala Lys Gln
        195                 200                 205

Asp Lys Ser Lys Asn Gly Ser Val Lys Glu Leu Lys His Ile Tyr Ser
    210                 215                 220

Asn His Ile Lys Gly Asn Lys Ile Thr Ala Pro Lys Pro Ser Ile Gln
225                 230                 235                 240

Gly Val Val Ile His Asn Asp Tyr Gly Ser Met Thr Pro Ser Gln Tyr
                245                 250                 255

Leu Pro Trp Leu Tyr Ala Arg Glu Asn Asn Gly Thr His Val Asn Gly
            260                 265                 270

Trp Ala Ser Val Tyr Ala Asn Arg Asn Glu Val Leu Trp Tyr His Pro
        275                 280                 285

Thr Asp Tyr Val Glu Trp His Cys Gly Asn Gln Trp Ala Asn Ala Asn
    290                 295                 300

Leu Ile Gly Phe Glu Val Cys Glu Ser Tyr Pro Gly Arg Ile Ser Asp
305                 310                 315                 320

Lys Leu Phe Leu Glu Asn Glu Glu Ala Thr Leu Lys Val Ala Ala Asp
                325                 330                 335

Val Met Lys Ser Tyr Gly Leu Pro Val Asn Arg Asn Thr Val Arg Leu
            340                 345                 350

His Asn Glu Phe Phe Gly Thr Ser Cys Pro His Arg Ser Trp Asp Leu
        355                 360                 365

His Val Gly Lys Gly Glu Pro Tyr Thr Thr Thr Asn Ile Asn Lys Met
    370                 375                 380

Lys Asp Tyr Phe Ile Lys Arg Ile Lys His Tyr Tyr Asp Gly Gly Lys
385                 390                 395                 400

Leu Glu Val Ser Lys Ala Ala Thr Ile Lys Gln Ser Asp Val Lys Gln
                405                 410                 415

Glu Val Lys Lys Gln Glu Ala Lys Gln Ile Val Lys Ala Thr Asp Trp
            420                 425                 430

Lys Gln Asn Lys Asp Gly Ile Trp Tyr Lys Ala Glu His Ala Ser Phe
      435                 440                 445

Thr Val Thr Ala Pro Glu Gly Ile Ile Thr Arg Tyr Lys Gly Pro Trp
    450                 455                 460

Thr Gly His Pro Gln Ala Gly Val Leu Gln Lys Gly Gln Thr Ile Lys
465                 470                 475                 480

Tyr Asp Glu Val Gln Lys Phe Asp Gly His Val Trp Val Ser Trp Glu
                485                 490                 495

Thr Phe Glu Gly Glu Thr Val Tyr Met Pro Val Arg Thr Trp Asp Ala
            500                 505                 510

Lys Thr Gly Lys Val Gly Lys Leu Trp Gly Glu Ile Lys
      515                 520                 525

<210>  71
<211>  1578
<212>  DNA
<213>  artificial

<220>
<223>  Cecropin A (A.aegypti)-Ply2638A

<400>  71
atgggcggcc tgaaaaaact gggcaaaaaa ctggaaggtg ccggcaaacg tgtgttcaac      60

gctgcagaaa aagcactgcc ggttgtagct ggtgctaaag ctctccgtaa aggatccatg     120

ctaactgcta ttgactatct tacgaaaaaa ggttggaaaa tatcatctga ccctcgcact     180

tacgatggtt accctaaaaa ctacggctac agaaattacc atgaaaacgg cattaattat     240

gatgagtttt gtggtggtta tcatagagct tttgatgttt acagtaacga aactaacgac     300

gtgcctgctg ttactagcgg aacagttatt gaagcaaacg attacggtaa ttttggtggt     360

acattcgtta ttagagacgc taacgataac gattggatat atgggcatct acaacgtggc     420

tcaatgcgat ttgttgtagg cgacaaagtc aatcaaggtg acattattgg tttacaaggt     480

aatagcaact attacgacaa tcctatgagt gtacatttac atttacaatt acgccctaaa     540

gacgcaaaga aagatgaaaa atcacaagta tgtagtggtt tggctatgga aaaatatgac     600

attacaaatt taaatgctaa acaagataaa tcaaagaatg ggagcgtgaa agagttgaaa     660

catatctatt caaaccatat taaaggtaac aagattacag caccaaaacc tagtattcaa     720

ggtgtggtca tccacaatga ttatggtagt atgacaccta gtcaatactt accatggtta     780

tatgcacgtg agaataacgg tacacacgtt aacggttggg ctagtgttta tgcaaataga     840

aacgaagtgc tttggtatca tccgacagac tacgtagagt ggcattgtgg taatcaatgg 900

gcaaatgcta acttaatcgg atttgaagtg tgtgagtcgt atcctggtag aatctcggac 960

aaattattct tagaaaatga agaagcgaca ttgaaagtag ctgcggatgt gatgaagtcg 1020

tacggattac cagttaatcg caacactgta cgtctgcata acgaattctt cggaacttct 1080

tgtccacatc gttcgtggga cttgcatgtt ggcaaaggtg agccttacac aactactaat 1140

attaataaaa tgaaagacta cttcatcaaa cgcatcaaac attattatga cggtggaaag 1200

ctagaagtaa gcaaagcagc aactatcaaa caatctgacg ttaagcaaga agttaaaaag 1260

caagaagcaa aacaaattgt gaaagcaaca gattggaaac agaataaaga tggcatttgg 1320

tataaagctg aacatgcttc gttcacagtg acagcaccag agggaattat cacaagatac 1380

aaaggtcctt ggactggtca cccacaagct ggtgtattac aaaaaggtca aacgattaaa 1440

tatgatgagg ttcaaaaatt tgacggtcat gtttgggtat cgtgggaaac gtttgagggc 1500

gaaactgtat acatgccggt acgcacatgg gacgctaaaa ctggtaaagt tggtaagttg 1560

tggggcgaaa ttaaatga 1578

<210> 72
<211> 528
<212> PRT
<213> artificial

<220>
<223> Sarcotoxin IA-Ply2638A

<400> 72

Met Gly Trp Leu Lys Lys Ile Gly Lys Lys Ile Glu Arg Val Gly Gln
1               5                   10                  15

His Thr Arg Asp Ala Thr Ile Gln Gly Leu Gly Ile Ala Gln Gln Ala
            20                  25                  30

Ala Asn Val Ala Ala Thr Ala Arg Gly Ser Met Leu Thr Ala Ile Asp
        35                  40                  45

Tyr Leu Thr Lys Lys Gly Trp Lys Ile Ser Ser Asp Pro Arg Thr Tyr
    50                  55                  60

Asp Gly Tyr Pro Lys Asn Tyr Gly Tyr Arg Asn Tyr His Glu Asn Gly
65                  70                  75                  80

Ile Asn Tyr Asp Glu Phe Cys Gly Gly Tyr His Arg Ala Phe Asp Val
                85                  90                  95

Tyr Ser Asn Glu Thr Asn Asp Val Pro Ala Val Thr Ser Gly Thr Val
            100                 105                 110

Ile Glu Ala Asn Asp Tyr Gly Asn Phe Gly Gly Thr Phe Val Ile Arg
        115                 120                 125

Asp Ala Asn Asp Asn Asp Trp Ile Tyr Gly His Leu Gln Arg Gly Ser
    130                 135                 140

Met Arg Phe Val Val Gly Asp Lys Val Asn Gln Gly Asp Ile Ile Gly
145                 150                 155                 160

Leu Gln Gly Asn Ser Asn Tyr Tyr Asp Asn Pro Met Ser Val His Leu
                165                 170                 175

His Leu Gln Leu Arg Pro Lys Asp Ala Lys Lys Asp Glu Lys Ser Gln
            180                 185                 190

Val Cys Ser Gly Leu Ala Met Glu Lys Tyr Asp Ile Thr Asn Leu Asn
        195                 200                 205

Ala Lys Gln Asp Lys Ser Lys Asn Gly Ser Val Lys Glu Leu Lys His
    210                 215                 220

Ile Tyr Ser Asn His Ile Lys Gly Asn Lys Ile Thr Ala Pro Lys Pro
225                 230                 235                 240

Ser Ile Gln Gly Val Val Ile His Asn Asp Tyr Gly Ser Met Thr Pro
                245                 250                 255

Ser Gln Tyr Leu Pro Trp Leu Tyr Ala Arg Glu Asn Asn Gly Thr His
            260                 265                 270

Val Asn Gly Trp Ala Ser Val Tyr Ala Asn Arg Asn Glu Val Leu Trp
        275                 280                 285

Tyr His Pro Thr Asp Tyr Val Glu Trp His Cys Gly Asn Gln Trp Ala
    290                 295                 300

Asn Ala Asn Leu Ile Gly Phe Glu Val Cys Glu Ser Tyr Pro Gly Arg
305                 310                 315                 320

Ile Ser Asp Lys Leu Phe Leu Glu Asn Glu Glu Ala Thr Leu Lys Val
                325                 330                 335

Ala Ala Asp Val Met Lys Ser Tyr Gly Leu Pro Val Asn Arg Asn Thr
            340                 345                 350

Val Arg Leu His Asn Glu Phe Phe Gly Thr Ser Cys Pro His Arg Ser

355                 360                 365

Trp Asp Leu His Val Gly Lys Gly Glu Pro Tyr Thr Thr Thr Asn Ile
    370                 375                 380

Asn Lys Met Lys Asp Tyr Phe Ile Lys Arg Ile Lys His Tyr Tyr Asp
385                 390                 395                 400

Gly Gly Lys Leu Glu Val Ser Lys Ala Ala Thr Ile Lys Gln Ser Asp
                405                 410                 415

Val Lys Gln Glu Val Lys Lys Gln Glu Ala Lys Gln Ile Val Lys Ala
            420                 425                 430

Thr Asp Trp Lys Gln Asn Lys Asp Gly Ile Trp Tyr Lys Ala Glu His
        435                 440                 445

Ala Ser Phe Thr Val Thr Ala Pro Glu Gly Ile Ile Thr Arg Tyr Lys
    450                 455                 460

Gly Pro Trp Thr Gly His Pro Gln Ala Gly Val Leu Gln Lys Gly Gln
465                 470                 475                 480

Thr Ile Lys Tyr Asp Glu Val Gln Lys Phe Asp Gly His Val Trp Val
                485                 490                 495

Ser Trp Glu Thr Phe Glu Gly Glu Thr Val Tyr Met Pro Val Arg Thr
            500                 505                 510

Trp Asp Ala Lys Thr Gly Lys Val Gly Lys Leu Trp Gly Glu Ile Lys
        515                 520                 525

<210>  73
<211>  1587
<212>  DNA
<213>  artificial

<220>
<223>  Sarcotoxin IA-Ply2638A

<400>  73
atgggatggc tcaaaaagat tggcaagaaa atcgagcgag tcggtcagca tacgcgtgat      60

gcaactatcc agggtttagg tatcgcacag caagcagcta atgtagcagc tactgctcgg     120

ggatccatgc taactgctat tgactatctt acgaaaaaag gttggaaaat atcatctgac     180

cctcgcactt acgatggtta ccctaaaaac tacggctaca gaaattacca tgaaaacggc     240

attaattatg atgagttttg tggtggttat catagagctt ttgatgttta cagtaacgaa     300

actaacgacg tgcctgctgt tactagcgga acagttattg aagcaaacga ttacggtaat     360

tttggtggta cattcgttat tagagacgct aacgataacg attggatata tgggcatcta 420

caacgtggct caatgcgatt tgttgtaggc gacaaagtca atcaaggtga cattattggt 480

ttacaaggta atagcaacta ttacgacaat cctatgagtg tacatttaca tttacaatta 540

cgccctaaag acgcaaagaa agatgaaaaa tcacaagtat gtagtggttt ggctatggaa 600

aaatatgaca ttacaaattt aaatgctaaa caagataaat caaagaatgg gagcgtgaaa 660

gagttgaaac atatctattc aaaccatatt aaaggtaaca agattacagc accaaaacct 720

agtattcaag gtgtggtcat ccacaatgat tatggtagta tgacacctag tcaatactta 780

ccatggttat atgcacgtga gaataacggt acacacgtta acggttgggc tagtgtttat 840

gcaaatagaa acgaagtgct ttggtatcat ccgacagact acgtagagtg gcattgtggt 900

aatcaatggg caaatgctaa cttaatcgga tttgaagtgt gtgagtcgta tcctggtaga 960

atctcggaca aattattctt agaaaatgaa gaagcgacat tgaaagtagc tgcggatgtg 1020

atgaagtcgt acggattacc agttaatcgc aacactgtac gtctgcataa cgaattcttc 1080

ggaacttctt gtccacatcg ttcgtgggac ttgcatgttg gcaaaggtga gccttacaca 1140

actactaata ttaataaaat gaaagactac ttcatcaaac gcatcaaaca ttattatgac 1200

ggtggaaagc tagaagtaag caaagcagca actatcaaac aatctgacgt taagcaagaa 1260

gttaaaaagc aagaagcaaa acaaattgtg aaagcaacag attggaaaca gaataaagat 1320

ggcatttggt ataaagctga acatgcttcg ttcacagtga cagcaccaga gggaattatc 1380

acaagataca aaggtccttg gactggtcac ccacaagctg gtgtattaca aaaaggtcaa 1440

acgattaaat atgatgaggt tcaaaaattt gacggtcatg tttgggtatc gtgggaaacg 1500

tttgagggcg aaactgtata catgccggta cgcacatggg acgctaaaac tggtaaagtt 1560

ggtaagttgt ggggcgaaat taaatga 1587

<210> 74
<211> 543
<212> PRT
<213> artificial

<220>
<223> OR-7-Ply2638A

<400> 74

Met Lys Thr Tyr Tyr Gly Thr Asn Gly Val His Cys Thr Lys Asn Ser
1               5                   10                  15

Leu Trp Gly Lys Val Arg Leu Lys Asn Met Lys Tyr Asp Gln Asn Thr
            20                  25                  30

Thr Tyr Met Gly Arg Leu Gln Asp Ile Leu Leu Gly Trp Ala Thr Gly

35                  40                  45

Ala Phe Gly Lys Thr Phe His Gly Ser Met Leu Thr Ala Ile Asp Tyr
    50                  55                  60

Leu Thr Lys Lys Gly Trp Lys Ile Ser Ser Asp Pro Arg Thr Tyr Asp
65                  70                  75                  80

Gly Tyr Pro Lys Asn Tyr Gly Tyr Arg Asn Tyr His Glu Asn Gly Ile
                85                  90                  95

Asn Tyr Asp Glu Phe Cys Gly Gly Tyr His Arg Ala Phe Asp Val Tyr
            100                 105                 110

Ser Asn Glu Thr Asn Asp Val Pro Ala Val Thr Ser Gly Thr Val Ile
        115                 120                 125

Glu Ala Asn Asp Tyr Gly Asn Phe Gly Gly Thr Phe Val Ile Arg Asp
    130                 135                 140

Ala Asn Asp Asn Asp Trp Ile Tyr Gly His Leu Gln Arg Gly Ser Met
145                 150                 155                 160

Arg Phe Val Val Gly Asp Lys Val Asn Gln Gly Asp Ile Ile Gly Leu
                165                 170                 175

Gln Gly Asn Ser Asn Tyr Tyr Asp Asn Pro Met Ser Val His Leu His
            180                 185                 190

Leu Gln Leu Arg Pro Lys Asp Ala Lys Lys Asp Glu Lys Ser Gln Val
        195                 200                 205

Cys Ser Gly Leu Ala Met Glu Lys Tyr Asp Ile Thr Asn Leu Asn Ala
    210                 215                 220

Lys Gln Asp Lys Ser Lys Asn Gly Ser Val Lys Glu Leu Lys His Ile
225                 230                 235                 240

Tyr Ser Asn His Ile Lys Gly Asn Lys Ile Thr Ala Pro Lys Pro Ser
                245                 250                 255

Ile Gln Gly Val Val Ile His Asn Asp Tyr Gly Ser Met Thr Pro Ser
            260                 265                 270

Gln Tyr Leu Pro Trp Leu Tyr Ala Arg Glu Asn Asn Gly Thr His Val
        275                 280                 285

Asn Gly Trp Ala Ser Val Tyr Ala Asn Arg Asn Glu Val Leu Trp Tyr
    290                 295                 300

His Pro Thr Asp Tyr Val Glu Trp His Cys Gly Asn Gln Trp Ala Asn
305                 310                 315                 320

Ala Asn Leu Ile Gly Phe Glu Val Cys Glu Ser Tyr Pro Gly Arg Ile
                325                 330                 335

Ser Asp Lys Leu Phe Leu Glu Asn Glu Glu Ala Thr Leu Lys Val Ala
            340                 345                 350

Ala Asp Val Met Lys Ser Tyr Gly Leu Pro Val Asn Arg Asn Thr Val
        355                 360                 365

Arg Leu His Asn Glu Phe Phe Gly Thr Ser Cys Pro His Arg Ser Trp
    370                 375                 380

Asp Leu His Val Gly Lys Gly Glu Pro Tyr Thr Thr Thr Asn Ile Asn
385                 390                 395                 400

Lys Met Lys Asp Tyr Phe Ile Lys Arg Ile Lys His Tyr Tyr Asp Gly
                405                 410                 415

Gly Lys Leu Glu Val Ser Lys Ala Ala Thr Ile Lys Gln Ser Asp Val
            420                 425                 430

Lys Gln Glu Val Lys Lys Gln Glu Ala Lys Gln Ile Val Lys Ala Thr
        435                 440                 445

Asp Trp Lys Gln Asn Lys Asp Gly Ile Trp Tyr Lys Ala Glu His Ala
    450                 455                 460

Ser Phe Thr Val Thr Ala Pro Glu Gly Ile Ile Thr Arg Tyr Lys Gly
465                 470                 475                 480

Pro Trp Thr Gly His Pro Gln Ala Gly Val Leu Gln Lys Gly Gln Thr
                485                 490                 495

Ile Lys Tyr Asp Glu Val Gln Lys Phe Asp Gly His Val Trp Val Ser
            500                 505                 510

Trp Glu Thr Phe Glu Gly Glu Thr Val Tyr Met Pro Val Arg Thr Trp
        515                 520                 525

Asp Ala Lys Thr Gly Lys Val Gly Lys Leu Trp Gly Glu Ile Lys
    530                 535                 540

<210> 75
<211> 1632
<212> DNA
<213> artificial

<220>
<223> OR-7-Ply2638A

<400> 75
atgaaaacct actatggcac caacggcgta cactgcacca aaaacagcct gtggggtaaa 60

gtgcgtctga aaaacatgaa atacgaccag aacaccacgt atatgggccg tctccaggat 120

atcctgctgg gttgggcaac tggtgccttc ggcaaaacct ttcacggatc catgctaact 180

gctattgact atcttacgaa aaaaggttgg aaaatatcat ctgaccctcg cacttacgat 240

ggttacccta aaaactacgg ctacagaaat taccatgaaa acggcattaa ttatgatgag 300

ttttgtggtg gttatcatag agcttttgat gtttacagta acgaaactaa cgacgtgcct 360

gctgttacta gcggaacagt tattgaagca aacgattacg gtaattttgg tggtacattc 420

gttattagag acgctaacga taacgattgg atatatgggc atctacaacg tggctcaatg 480

cgatttgttg taggcgacaa agtcaatcaa ggtgacatta ttggtttaca aggtaatagc 540

aactattacg acaatcctat gagtgtacat ttacatttac aattacgccc taaagacgca 600

aagaaagatg aaaaatcaca agtatgtagt ggtttggcta tggaaaaata tgacattaca 660

aatttaaatg ctaaacaaga taaatcaaag aatgggagcg tgaaagagtt gaaacatatc 720

tattcaaacc atattaaagg taacaagatt acagcaccaa aacctagtat tcaaggtgtg 780

gtcatccaca atgattatgg tagtatgaca cctagtcaat acttaccatg gttatatgca 840

cgtgagaata acggtacaca cgttaacggt tgggctagtg tttatgcaaa tagaaacgaa 900

gtgctttggt atcatccgac agactacgta gagtggcatt gtggtaatca atgggcaaat 960

gctaacttaa tcggatttga agtgtgtgag tcgtatcctg gtagaatctc ggacaaatta 1020

ttcttagaaa atgaagaagc gacattgaaa gtagctgcgg atgtgatgaa gtcgtacgga 1080

ttaccagtta atcgcaacac tgtacgtctg cataacgaat tcttcggaac ttcttgtcca 1140

catcgttcgt gggacttgca tgttggcaaa ggtgagcctt acacaactac taatattaat 1200

aaaatgaaag actacttcat caaacgcatc aaacattatt atgacggtgg aaagctagaa 1260

gtaagcaaag cagcaactat caaacaatct gacgttaagc aagaagttaa aaagcaagaa 1320

gcaaaacaaa ttgtgaaagc aacagattgg aaacagaata aagatggcat ttggtataaa 1380

gctgaacatg cttcgttcac agtgacagca ccagagggaa ttatcacaag atacaaaggt 1440

ccttggactg gtcacccaca agctggtgta ttacaaaaag gtcaaacgat taaatatgat 1500

gaggttcaaa aatttgacgg tcatgtttgg gtatcgtggg aaacgtttga gggcgaaact 1560

gtatacatgc cggtacgcac atgggacgct aaaactggta aagttggtaa gttgtggggc 1620

gaaattaaat ga                                                          1632

<210>  76
<211>  502
<212>  PRT
<213>  artificial

<220>
<223>  Indolicidin-Ply2638A

<400>  76

Met Ile Leu Pro Trp Lys Trp Pro Trp Trp Pro Trp Arg Arg Gly Ser
1               5                   10                  15

Met Leu Thr Ala Ile Asp Tyr Leu Thr Lys Lys Gly Trp Lys Ile Ser
            20                  25                  30

Ser Asp Pro Arg Thr Tyr Asp Gly Tyr Pro Lys Asn Tyr Gly Tyr Arg
        35                  40                  45

Asn Tyr His Glu Asn Gly Ile Asn Tyr Asp Glu Phe Cys Gly Gly Tyr
    50                  55                  60

His Arg Ala Phe Asp Val Tyr Ser Asn Glu Thr Asn Asp Val Pro Ala
65                  70                  75                  80

Val Thr Ser Gly Thr Val Ile Glu Ala Asn Asp Tyr Gly Asn Phe Gly
                85                  90                  95

Gly Thr Phe Val Ile Arg Asp Ala Asn Asp Asn Asp Trp Ile Tyr Gly
            100                 105                 110

His Leu Gln Arg Gly Ser Met Arg Phe Val Val Gly Asp Lys Val Asn
        115                 120                 125

Gln Gly Asp Ile Ile Gly Leu Gln Gly Asn Ser Asn Tyr Tyr Asp Asn
    130                 135                 140

Pro Met Ser Val His Leu His Leu Gln Leu Arg Pro Lys Asp Ala Lys
145                 150                 155                 160

Lys Asp Glu Lys Ser Gln Val Cys Ser Gly Leu Ala Met Glu Lys Tyr
                165                 170                 175

Asp Ile Thr Asn Leu Asn Ala Lys Gln Asp Lys Ser Lys Asn Gly Ser
            180                 185                 190

Val Lys Glu Leu Lys His Ile Tyr Ser Asn His Ile Lys Gly Asn Lys

195                 200                 205

Ile Thr Ala Pro Lys Pro Ser Ile Gln Gly Val Val Ile His Asn Asp
    210                 215                 220

Tyr Gly Ser Met Thr Pro Ser Gln Tyr Leu Pro Trp Leu Tyr Ala Arg
225                 230                 235                 240

Glu Asn Asn Gly Thr His Val Asn Gly Trp Ala Ser Val Tyr Ala Asn
                245                 250                 255

Arg Asn Glu Val Leu Trp Tyr His Pro Thr Asp Tyr Val Glu Trp His
            260                 265                 270

Cys Gly Asn Gln Trp Ala Asn Ala Asn Leu Ile Gly Phe Glu Val Cys
        275                 280                 285

Glu Ser Tyr Pro Gly Arg Ile Ser Asp Lys Leu Phe Leu Glu Asn Glu
    290                 295                 300

Glu Ala Thr Leu Lys Val Ala Ala Asp Val Met Lys Ser Tyr Gly Leu
305                 310                 315                 320

Pro Val Asn Arg Asn Thr Val Arg Leu His Asn Glu Phe Phe Gly Thr
                325                 330                 335

Ser Cys Pro His Arg Ser Trp Asp Leu His Val Gly Lys Gly Glu Pro
            340                 345                 350

Tyr Thr Thr Thr Asn Ile Asn Lys Met Lys Asp Tyr Phe Ile Lys Arg
        355                 360                 365

Ile Lys His Tyr Tyr Asp Gly Gly Lys Leu Glu Val Ser Lys Ala Ala
    370                 375                 380

Thr Ile Lys Gln Ser Asp Val Lys Gln Glu Val Lys Lys Gln Glu Ala
385                 390                 395                 400

Lys Gln Ile Val Lys Ala Thr Asp Trp Lys Gln Asn Lys Asp Gly Ile
                405                 410                 415

Trp Tyr Lys Ala Glu His Ala Ser Phe Thr Val Thr Ala Pro Glu Gly
            420                 425                 430

Ile Ile Thr Arg Tyr Lys Gly Pro Trp Thr Gly His Pro Gln Ala Gly
        435                 440                 445

Val Leu Gln Lys Gly Gln Thr Ile Lys Tyr Asp Glu Val Gln Lys Phe
    450                 455                 460

Asp Gly His Val Trp Val Ser Trp Glu Thr Phe Glu Gly Glu Thr Val
465                 470                 475                 480

Tyr Met Pro Val Arg Thr Trp Asp Ala Lys Thr Gly Lys Val Gly Lys
                485                 490                 495

Leu Trp Gly Glu Ile Lys
            500

<210>  77
<211>  1509
<212>  DNA
<213>  artificial

<220>
<223>  Indolicidin-Ply2638A

<400>  77
atgatcctgc cgtggaaatg gccgtggtgg ccatggcgtc gcggatccat gctaactgct      60

attgactatc ttacgaaaaa aggttggaaa atatcatctg accctcgcac ttacgatggt     120

taccctaaaa actacggcta cagaaattac catgaaaacg gcattaatta tgatgagttt     180

tgtggtggtt atcatagagc ttttgatgtt tacagtaacg aaactaacga cgtgcctgct     240

gttactagcg gaacagttat tgaagcaaac gattacggta attttggtgg tacattcgtt     300

attagagacg ctaacgataa cgattggata tatgggcatc tacaacgtgg ctcaatgcga     360

tttgttgtag gcgacaaagt caatcaaggt gacattattg gtttacaagg taatagcaac     420

tattacgaca atcctatgag tgtacattta catttacaat tacgccctaa agacgcaaag     480

aaagatgaaa aatcacaagt atgtagtggt ttggctatgg aaaaatatga cattacaaat     540

ttaaatgcta aacaagataa atcaaagaat gggagcgtga aagagttgaa acatatctat     600

tcaaaccata ttaaaggtaa caagattaca gcaccaaaac ctagtattca aggtgtggtc     660

atccacaatg attatggtag tatgacacct agtcaatact taccatggtt atatgcacgt     720

gagaataacg gtacacacgt taacggttgg gctagtgttt atgcaaatag aaacgaagtg     780

ctttggtatc atccgacaga ctacgtagag tggcattgtg gtaatcaatg ggcaaatgct     840

aacttaatcg gatttgaagt gtgtgagtcg tatcctggta gaatctcgga caaattattc     900

ttagaaaatg aagaagcgac attgaaagta gctgcggatg tgatgaagtc gtacggatta     960

ccagttaatc gcaacactgt acgtctgcat aacgaattct tcggaacttc ttgtccacat    1020

cgttcgtggg acttgcatgt tggcaaaggt gagccttaca caactactaa tattaataaa    1080

atgaaagact acttcatcaa acgcatcaaa cattattatg acggtggaaa gctagaagta    1140

agcaaagcag caactatcaa acaatctgac gttaagcaag aagttaaaaa gcaagaagca 1200

aaacaaattg tgaaagcaac agattggaaa cagaataaag atggcatttg gtataaagct 1260

gaacatgctt cgttcacagt gacagcacca gagggaatta tcacaagata caaaggtcct 1320

tggactggtc acccacaagc tggtgtatta caaaaaggtc aaacgattaa atatgatgag 1380

gttcaaaaat ttgacggtca tgtttgggta tcgtggaaaa cgtttgaggg cgaaactgta 1440

tacatgccgg tacgcacatg ggacgctaaa actggtaaag ttggtaagtt gtggggcgaa 1500

attaaatga 1509

<210> 78
<211> 513
<212> PRT
<213> artificial

<220>
<223> Pseudin1-Ply2638A

<400> 78

Met Gly Leu Asn Thr Leu Lys Lys Val Phe Gln Gly Leu His Glu Ala
1               5                   10                  15

Ile Lys Leu Ile Asn Asn His Val Gln Gly Ser Met Leu Thr Ala Ile
            20                  25                  30

Asp Tyr Leu Thr Lys Lys Gly Trp Lys Ile Ser Ser Asp Pro Arg Thr
        35                  40                  45

Tyr Asp Gly Tyr Pro Lys Asn Tyr Gly Tyr Arg Asn Tyr His Glu Asn
    50                  55                  60

Gly Ile Asn Tyr Asp Glu Phe Cys Gly Gly Tyr His Arg Ala Phe Asp
65                  70                  75                  80

Val Tyr Ser Asn Glu Thr Asn Asp Val Pro Ala Val Thr Ser Gly Thr
                85                  90                  95

Val Ile Glu Ala Asn Asp Tyr Gly Asn Phe Gly Gly Thr Phe Val Ile
            100                 105                 110

Arg Asp Ala Asn Asp Asn Asp Trp Ile Tyr Gly His Leu Gln Arg Gly
        115                 120                 125

Ser Met Arg Phe Val Val Gly Asp Lys Val Asn Gln Gly Asp Ile Ile
    130                 135                 140

Gly Leu Gln Gly Asn Ser Asn Tyr Tyr Asp Asn Pro Met Ser Val His
145                 150                 155                 160

Leu His Leu Gln Leu Arg Pro Lys Asp Ala Lys Lys Asp Glu Lys Ser
                165                 170                 175

Gln Val Cys Ser Gly Leu Ala Met Glu Lys Tyr Asp Ile Thr Asn Leu
            180                 185                 190

Asn Ala Lys Gln Asp Lys Ser Lys Asn Gly Ser Val Lys Glu Leu Lys
        195                 200                 205

His Ile Tyr Ser Asn His Ile Lys Gly Asn Lys Ile Thr Ala Pro Lys
    210                 215                 220

Pro Ser Ile Gln Gly Val Val Ile His Asn Asp Tyr Gly Ser Met Thr
225                 230                 235                 240

Pro Ser Gln Tyr Leu Pro Trp Leu Tyr Ala Arg Glu Asn Asn Gly Thr
                245                 250                 255

His Val Asn Gly Trp Ala Ser Val Tyr Ala Asn Arg Asn Glu Val Leu
            260                 265                 270

Trp Tyr His Pro Thr Asp Tyr Val Glu Trp His Cys Gly Asn Gln Trp
        275                 280                 285

Ala Asn Ala Asn Leu Ile Gly Phe Glu Val Cys Glu Ser Tyr Pro Gly
    290                 295                 300

Arg Ile Ser Asp Lys Leu Phe Leu Glu Asn Glu Glu Ala Thr Leu Lys
305                 310                 315                 320

Val Ala Ala Asp Val Met Lys Ser Tyr Gly Leu Pro Val Asn Arg Asn
                325                 330                 335

Thr Val Arg Leu His Asn Glu Phe Phe Gly Thr Ser Cys Pro His Arg
            340                 345                 350

Ser Trp Asp Leu His Val Gly Lys Gly Glu Pro Tyr Thr Thr Thr Asn
        355                 360                 365

Ile Asn Lys Met Lys Asp Tyr Phe Ile Lys Arg Ile Lys His Tyr Tyr
    370                 375                 380

Asp Gly Gly Lys Leu Glu Val Ser Lys Ala Ala Thr Ile Lys Gln Ser
385                 390                 395                 400

Asp Val Lys Gln Glu Val Lys Lys Gln Glu Ala Lys Gln Ile Val Lys

405                 410                 415

Ala Thr Asp Trp Lys Gln Asn Lys Asp Gly Ile Trp Tyr Lys Ala Glu
            420                 425                 430

His Ala Ser Phe Thr Val Thr Ala Pro Glu Gly Ile Ile Thr Arg Tyr
        435                 440                 445

Lys Gly Pro Trp Thr Gly His Pro Gln Ala Gly Val Leu Gln Lys Gly
    450                 455                 460

Gln Thr Ile Lys Tyr Asp Glu Val Gln Lys Phe Asp Gly His Val Trp
465                 470                 475                 480

Val Ser Trp Glu Thr Phe Glu Gly Glu Thr Val Tyr Met Pro Val Arg
                485                 490                 495

Thr Trp Asp Ala Lys Thr Gly Lys Val Gly Lys Leu Trp Gly Glu Ile
            500                 505                 510

Lys

<210>  79
<211>  1542
<212>  DNA
<213>  artificial

<220>
<223>  Pseudin1-Ply2638A

<400>  79
atgggtctga acaccctgaa aaaagtattc cagggtctgc atgaagcgat taaactgatc      60

aacaaccacg ttcagggatc catgctaact gctattgact atcttacgaa aaaaggttgg     120

aaaatatcat ctgaccctcg cacttacgat ggttacccta aaaactacgg ctacagaaat     180

taccatgaaa acggcattaa ttatgatgag ttttgtggtg gttatcatag agcttttgat     240

gtttacagta acgaaactaa cgacgtgcct gctgttacta gcggaacagt tattgaagca     300

aacgattacg gtaattttgg tggtacattc gttattagag acgctaacga taacgattgg     360

atatatgggc atctacaacg tggctcaatg cgatttgttg taggcgacaa agtcaatcaa     420

ggtgacatta ttggtttaca aggtaatagc aactattacg acaatcctat gagtgtacat     480

ttacatttac aattacgccc taaagacgca aagaaagatg aaaaatcaca agtatgtagt     540

ggtttggcta tggaaaaata tgacattaca aatttaaatg ctaaacaaga taaatcaaag     600

aatgggagcg tgaaagagtt gaaacatatc tattcaaacc atattaaagg taacaagatt     660

acagcaccaa aacctagtat tcaaggtgtg gtcatccaca atgattatgg tagtatgaca     720

cctagtcaat acttaccatg gttatatgca cgtgagaata acggtacaca cgttaacggt 780

tgggctagtg tttatgcaaa tagaaacgaa gtgctttggt atcatccgac agactacgta 840

gagtggcatt gtggtaatca atgggcaaat gctaacttaa tcggatttga agtgtgtgag 900

tcgtatcctg gtagaatctc ggacaaatta ttcttagaaa atgaagaagc gacattgaaa 960

gtagctgcgg atgtgatgaa gtcgtacgga ttaccagtta atcgcaacac tgtacgtctg 1020

cataacgaat tcttcggaac ttcttgtcca catcgttcgt gggacttgca tgttggcaaa 1080

ggtgagcctt acacaactac taatattaat aaaatgaaag actacttcat caaacgcatc 1140

aaacattatt atgacggtgg aaagctagaa gtaagcaaag cagcaactat caaacaatct 1200

gacgttaagc aagaagttaa aaagcaagaa gcaaaacaaa ttgtgaaagc aacagattgg 1260

aaacagaata aagatggcat ttggtataaa gctgaacatg cttcgttcac agtgacagca 1320

ccagagggaa ttatcacaag atacaaaggt ccttggactg gtcacccaca agctggtgta 1380

ttacaaaaag gtcaaacgat taaatatgat gaggttcaaa aatttgacgg tcatgtttgg 1440

gtatcgtggg aaacgtttga gggcgaaact gtatacatgc cggtacgcac atgggacgct 1500

aaaactggta aagttggtaa gttgtggggc gaaattaaat ga 1542

<210> 80
<211> 512
<212> PRT
<213> artificial

<220>
<223> Magainin-Ply2638A

<400> 80

Met Gly Ile Gly Lys Phe Leu His Ser Ala Lys Lys Phe Gly Lys Ala
1               5                   10                  15

Phe Val Gly Glu Ile Met Asn Ser Gly Ser Met Leu Thr Ala Ile Asp
            20                  25                  30

Tyr Leu Thr Lys Lys Gly Trp Lys Ile Ser Ser Asp Pro Arg Thr Tyr
        35                  40                  45

Asp Gly Tyr Pro Lys Asn Tyr Gly Tyr Arg Asn Tyr His Glu Asn Gly
    50                  55                  60

Ile Asn Tyr Asp Glu Phe Cys Gly Gly Tyr His Arg Ala Phe Asp Val
65                  70                  75                  80

Tyr Ser Asn Glu Thr Asn Asp Val Pro Ala Val Thr Ser Gly Thr Val
                85                  90                  95

Ile Glu Ala Asn Asp Tyr Gly Asn Phe Gly Gly Thr Phe Val Ile Arg
        100                 105                 110

Asp Ala Asn Asp Asn Asp Trp Ile Tyr Gly His Leu Gln Arg Gly Ser
    115                 120                 125

Met Arg Phe Val Val Gly Asp Lys Val Asn Gln Gly Asp Ile Ile Gly
    130                 135                 140

Leu Gln Gly Asn Ser Asn Tyr Tyr Asp Asn Pro Met Ser Val His Leu
145                 150                 155                 160

His Leu Gln Leu Arg Pro Lys Asp Ala Lys Lys Asp Glu Lys Ser Gln
                165                 170                 175

Val Cys Ser Gly Leu Ala Met Glu Lys Tyr Asp Ile Thr Asn Leu Asn
            180                 185                 190

Ala Lys Gln Asp Lys Ser Lys Asn Gly Ser Val Lys Glu Leu Lys His
        195                 200                 205

Ile Tyr Ser Asn His Ile Lys Gly Asn Lys Ile Thr Ala Pro Lys Pro
    210                 215                 220

Ser Ile Gln Gly Val Val Ile His Asn Asp Tyr Gly Ser Met Thr Pro
225                 230                 235                 240

Ser Gln Tyr Leu Pro Trp Leu Tyr Ala Arg Glu Asn Asn Gly Thr His
                245                 250                 255

Val Asn Gly Trp Ala Ser Val Tyr Ala Asn Arg Asn Glu Val Leu Trp
            260                 265                 270

Tyr His Pro Thr Asp Tyr Val Glu Trp His Cys Gly Asn Gln Trp Ala
        275                 280                 285

Asn Ala Asn Leu Ile Gly Phe Glu Val Cys Glu Ser Tyr Pro Gly Arg
    290                 295                 300

Ile Ser Asp Lys Leu Phe Leu Glu Asn Glu Glu Ala Thr Leu Lys Val
305                 310                 315                 320

Ala Ala Asp Val Met Lys Ser Tyr Gly Leu Pro Val Asn Arg Asn Thr
                325                 330                 335

Val Arg Leu His Asn Glu Phe Phe Gly Thr Ser Cys Pro His Arg Ser
            340                 345                 350

Trp Asp Leu His Val Gly Lys Gly Glu Pro Tyr Thr Thr Thr Asn Ile
        355                 360                 365

Asn Lys Met Lys Asp Tyr Phe Ile Lys Arg Ile Lys His Tyr Tyr Asp
    370                 375                 380

Gly Gly Lys Leu Glu Val Ser Lys Ala Ala Thr Ile Lys Gln Ser Asp
385                 390                 395                 400

Val Lys Gln Glu Val Lys Lys Gln Glu Ala Lys Gln Ile Val Lys Ala
                405                 410                 415

Thr Asp Trp Lys Gln Asn Lys Asp Gly Ile Trp Tyr Lys Ala Glu His
            420                 425                 430

Ala Ser Phe Thr Val Thr Ala Pro Glu Gly Ile Ile Thr Arg Tyr Lys
        435                 440                 445

Gly Pro Trp Thr Gly His Pro Gln Ala Gly Val Leu Gln Lys Gly Gln
    450                 455                 460

Thr Ile Lys Tyr Asp Glu Val Gln Lys Phe Asp Gly His Val Trp Val
465                 470                 475                 480

Ser Trp Glu Thr Phe Glu Gly Glu Thr Val Tyr Met Pro Val Arg Thr
                485                 490                 495

Trp Asp Ala Lys Thr Gly Lys Val Gly Lys Leu Trp Gly Glu Ile Lys
            500                 505                 510

<210>  81
<211>  1539
<212>  DNA
<213>  artificial

<220>
<223>  Magainin-Ply2638A

<400>  81
atgggtatcg gcaaattcct gcactccgca aaaaaattcg gcaaagcttt cgtgggcgaa      60

attatgaact ctggatccat gctaactgct attgactatc ttacgaaaaa aggttggaaa     120

atatcatctg accctcgcac ttacgatggt taccctaaaa actacggcta cagaaattac     180

catgaaaacg gcattaatta tgatgagttt tgtggtggtt atcatagagc ttttgatgtt     240

tacagtaacg aaactaacga cgtgcctgct gttactagcg gaacagttat tgaagcaaac     300

gattacggta attttggtgg tacattcgtt attagagacg ctaacgataa cgattggata     360

tatgggcatc tacaacgtgg ctcaatgcga tttgttgtag gcgacaaagt caatcaaggt 420

gacattattg gtttacaagg taatagcaac tattacgaca atcctatgag tgtacattta 480

catttacaat tacgccctaa agacgcaaag aaagatgaaa aatcacaagt atgtagtggt 540

ttggctatgg aaaaatatga cattacaaat ttaaatgcta aacaagataa atcaaagaat 600

gggagcgtga aagagttgaa acatatctat tcaaaccata ttaaaggtaa caagattaca 660

gcaccaaaac ctagtattca aggtgtggtc atccacaatg attatggtag tatgacacct 720

agtcaatact taccatggtt atatgcacgt gagaataacg gtacacacgt taacggttgg 780

gctagtgttt atgcaaatag aaacgaagtg ctttggtatc atccgacaga ctacgtagag 840

tggcattgtg gtaatcaatg ggcaaatgct aacttaatcg gatttgaagt gtgtgagtcg 900

tatcctggta gaatctcgga caaattattc ttagaaaatg aagaagcgac attgaaagta 960

gctgcggatg tgatgaagtc gtacggatta ccagttaatc gcaacactgt acgtctgcat 1020

aacgaattct tcggaacttc ttgtccacat cgttcgtggg acttgcatgt tggcaaaggt 1080

gagccttaca caactactaa tattaataaa atgaaagact acttcatcaa acgcatcaaa 1140

cattattatg acggtggaaa gctagaagta agcaaagcag caactatcaa acaatctgac 1200

gttaagcaag aagttaaaaa gcaagaagca aaacaaattg tgaaagcaac agattggaaa 1260

cagaataaag atggcatttg gtataaagct gaacatgctt cgttcacagt gacagcacca 1320

gagggaatta tcacaagata caaaggtcct tggactggtc acccacaagc tggtgtatta 1380

caaaaaggtc aaacgattaa atatgatgag gttcaaaaat ttgacggtca tgtttgggta 1440

tcgtgggaaa cgtttgaggg cgaaactgta tacatgccgg tacgcacatg ggacgctaaa 1500

actggtaaag ttggtaagtt gtggggcgaa attaaatga 1539

<210> 82
<211> 514
<212> PRT
<213> artificial

<220>
<223> Pleurocidin-Ply2638A

<400> 82

Met Gly Trp Gly Ser Phe Phe Lys Lys Ala Ala His Val Gly Lys His
1               5                   10                  15

Val Gly Lys Ala Ala Leu Thr His Tyr Leu Gly Ser Met Leu Thr Ala
            20                  25                  30

Ile Asp Tyr Leu Thr Lys Lys Gly Trp Lys Ile Ser Ser Asp Pro Arg
        35                  40                  45

Thr Tyr Asp Gly Tyr Pro Lys Asn Tyr Gly Tyr Arg Asn Tyr His Glu
    50                  55                  60

Asn Gly Ile Asn Tyr Asp Glu Phe Cys Gly Gly Tyr His Arg Ala Phe
65                  70                  75                  80

Asp Val Tyr Ser Asn Glu Thr Asn Asp Val Pro Ala Val Thr Ser Gly
                85                  90                  95

Thr Val Ile Glu Ala Asn Asp Tyr Gly Asn Phe Gly Gly Thr Phe Val
            100                 105                 110

Ile Arg Asp Ala Asn Asp Asn Asp Trp Ile Tyr Gly His Leu Gln Arg
        115                 120                 125

Gly Ser Met Arg Phe Val Val Gly Asp Lys Val Asn Gln Gly Asp Ile
    130                 135                 140

Ile Gly Leu Gln Gly Asn Ser Asn Tyr Tyr Asp Asn Pro Met Ser Val
145                 150                 155                 160

His Leu His Leu Gln Leu Arg Pro Lys Asp Ala Lys Lys Asp Glu Lys
                165                 170                 175

Ser Gln Val Cys Ser Gly Leu Ala Met Glu Lys Tyr Asp Ile Thr Asn
            180                 185                 190

Leu Asn Ala Lys Gln Asp Lys Ser Lys Asn Gly Ser Val Lys Glu Leu
        195                 200                 205

Lys His Ile Tyr Ser Asn His Ile Lys Gly Asn Lys Ile Thr Ala Pro
    210                 215                 220

Lys Pro Ser Ile Gln Gly Val Val Ile His Asn Asp Tyr Gly Ser Met
225                 230                 235                 240

Thr Pro Ser Gln Tyr Leu Pro Trp Leu Tyr Ala Arg Glu Asn Asn Gly
                245                 250                 255

Thr His Val Asn Gly Trp Ala Ser Val Tyr Ala Asn Arg Asn Glu Val
            260                 265                 270

Leu Trp Tyr His Pro Thr Asp Tyr Val Glu Trp His Cys Gly Asn Gln
        275                 280                 285

Trp Ala Asn Ala Asn Leu Ile Gly Phe Glu Val Cys Glu Ser Tyr Pro
    290                 295                 300

Gly Arg Ile Ser Asp Lys Leu Phe Leu Glu Asn Glu Glu Ala Thr Leu
305                 310                 315                 320

Lys Val Ala Ala Asp Val Met Lys Ser Tyr Gly Leu Pro Val Asn Arg
                325                 330                 335

Asn Thr Val Arg Leu His Asn Glu Phe Phe Gly Thr Ser Cys Pro His
            340                 345                 350

Arg Ser Trp Asp Leu His Val Gly Lys Gly Glu Pro Tyr Thr Thr Thr
        355                 360                 365

Asn Ile Asn Lys Met Lys Asp Tyr Phe Ile Lys Arg Ile Lys His Tyr
    370                 375                 380

Tyr Asp Gly Gly Lys Leu Glu Val Ser Lys Ala Ala Thr Ile Lys Gln
385                 390                 395                 400

Ser Asp Val Lys Gln Glu Val Lys Lys Gln Glu Ala Lys Gln Ile Val
                405                 410                 415

Lys Ala Thr Asp Trp Lys Gln Asn Lys Asp Gly Ile Trp Tyr Lys Ala
            420                 425                 430

Glu His Ala Ser Phe Thr Val Thr Ala Pro Glu Gly Ile Ile Thr Arg
        435                 440                 445

Tyr Lys Gly Pro Trp Thr Gly His Pro Gln Ala Gly Val Leu Gln Lys
    450                 455                 460

Gly Gln Thr Ile Lys Tyr Asp Glu Val Gln Lys Phe Asp Gly His Val
465                 470                 475                 480

Trp Val Ser Trp Glu Thr Phe Glu Gly Glu Thr Val Tyr Met Pro Val
                485                 490                 495

Arg Thr Trp Asp Ala Lys Thr Gly Lys Val Gly Lys Leu Trp Gly Glu
            500                 505                 510

Ile Lys

<210>  83
<211>  1545
<212>  DNA
<213>  artificial

<220>

<223>  Pleurocidin-Ply2638A

<400>  83
atgggctggg gttctttctt taaaaaagcg gctcacgttg gcaaacatgt aggtaaagca      60

gctctgaccc actatctggg atccatgcta actgctattg actatcttac gaaaaaaggt     120

tggaaaatat catctgaccc tcgcacttac gatggttacc ctaaaaacta cggctacaga     180

aattaccatg aaaacggcat taattatgat gagttttgtg gtggttatca tagagctttt     240

gatgtttaca gtaacgaaac taacgacgtg cctgctgtta ctagcggaac agttattgaa     300

gcaaacgatt acggtaattt tggtggtaca ttcgttatta gagacgctaa cgataacgat     360

tggatatatg ggcatctaca acgtggctca atgcgatttg ttgtaggcga caaagtcaat     420

caaggtgaca ttattggttt acaaggtaat agcaactatt acgacaatcc tatgagtgta     480

catttacatt tacaattacg ccctaaagac gcaaagaaag atgaaaaatc acaagtatgt     540

agtggtttgg ctatggaaaa atatgacatt acaaatttaa atgctaaaca agataaatca     600

aagaatggga gcgtgaaaga gttgaaacat atctattcaa accatattaa aggtaacaag     660

attacagcac caaaacctag tattcaaggt gtggtcatcc acaatgatta tggtagtatg     720

acacctagtc aatacttacc atggttatat gcacgtgaga ataacggtac acacgttaac     780

ggttgggcta gtgtttatgc aaatagaaac gaagtgcttt ggtatcatcc gacagactac     840

gtagagtggc attgtggtaa tcaatgggca aatgctaact taatcggatt tgaagtgtgt     900

gagtcgtatc ctggtagaat ctcggacaaa ttattcttag aaaatgaaga agcgacattg     960

aaagtagctg cggatgtgat gaagtcgtac ggattaccag ttaatcgcaa cactgtacgt    1020

ctgcataacg aattcttcgg aacttcttgt ccacatcgtt cgtgggactt gcatgttggc    1080

aaaggtgagc cttacacaac tactaatatt aataaaatga aagactactt catcaaacgc    1140

atcaaacatt attatgacgg tggaaagcta gaagtaagca aagcagcaac tatcaaacaa    1200

tctgacgtta agcaagaagt taaaaagcaa gaagcaaaac aaattgtgaa agcaacagat    1260

tggaaacaga ataaagatgg catttggtat aaagctgaac atgcttcgtt cacagtgaca    1320

gcaccagagg gaattatcac aagatacaaa ggtccttgga ctggtcaccc acaagctggt    1380

gtattacaaa aaggtcaaac gattaaatat gatgaggttc aaaaatttga cggtcatgtt    1440

tgggtatcgt gggaaacgtt tgagggcgaa actgtataca tgccggtacg cacatgggac    1500

gctaaaactg gtaaagttgg taagttgtgg ggcgaaatta aatga                    1545

<210>  84
<211>  510
<212>  PRT
<213>  artificial

<220>
<223>  BuforinII-Ply2638A

<400>  84

Met Thr Arg Ser Ser Arg Ala Gly Leu Gln Phe Pro Val Gly Arg Val
1               5                   10                  15

His Arg Leu Leu Arg Lys Gly Ser Met Leu Thr Ala Ile Asp Tyr Leu
            20                  25                  30

Thr Lys Lys Gly Trp Lys Ile Ser Ser Asp Pro Arg Thr Tyr Asp Gly
        35                  40                  45

Tyr Pro Lys Asn Tyr Gly Tyr Arg Asn Tyr His Glu Asn Gly Ile Asn
    50                  55                  60

Tyr Asp Glu Phe Cys Gly Gly Tyr His Arg Ala Phe Asp Val Tyr Ser
65                  70                  75                  80

Asn Glu Thr Asn Asp Val Pro Ala Val Thr Ser Gly Thr Val Ile Glu
                85                  90                  95

Ala Asn Asp Tyr Gly Asn Phe Gly Gly Thr Phe Val Ile Arg Asp Ala
            100                 105                 110

Asn Asp Asn Asp Trp Ile Tyr Gly His Leu Gln Arg Gly Ser Met Arg
        115                 120                 125

Phe Val Val Gly Asp Lys Val Asn Gln Gly Asp Ile Ile Gly Leu Gln
    130                 135                 140

Gly Asn Ser Asn Tyr Tyr Asp Asn Pro Met Ser Val His Leu His Leu
145                 150                 155                 160

Gln Leu Arg Pro Lys Asp Ala Lys Lys Asp Glu Lys Ser Gln Val Cys
                165                 170                 175

Ser Gly Leu Ala Met Glu Lys Tyr Asp Ile Thr Asn Leu Asn Ala Lys
            180                 185                 190

Gln Asp Lys Ser Lys Asn Gly Ser Val Lys Glu Leu Lys His Ile Tyr
        195                 200                 205

Ser Asn His Ile Lys Gly Asn Lys Ile Thr Ala Pro Lys Pro Ser Ile
    210                 215                 220

Gln Gly Val Val Ile His Asn Asp Tyr Gly Ser Met Thr Pro Ser Gln
225                 230                 235                 240

Tyr Leu Pro Trp Leu Tyr Ala Arg Glu Asn Asn Gly Thr His Val Asn
                245                 250                 255

Gly Trp Ala Ser Val Tyr Ala Asn Arg Asn Glu Val Leu Trp Tyr His
            260                 265                 270

Pro Thr Asp Tyr Val Glu Trp His Cys Gly Asn Gln Trp Ala Asn Ala
        275                 280                 285

Asn Leu Ile Gly Phe Glu Val Cys Glu Ser Tyr Pro Gly Arg Ile Ser
    290                 295                 300

Asp Lys Leu Phe Leu Glu Asn Glu Glu Ala Thr Leu Lys Val Ala Ala
305                 310                 315                 320

Asp Val Met Lys Ser Tyr Gly Leu Pro Val Asn Arg Asn Thr Val Arg
                325                 330                 335

Leu His Asn Glu Phe Phe Gly Thr Ser Cys Pro His Arg Ser Trp Asp
            340                 345                 350

Leu His Val Gly Lys Gly Glu Pro Tyr Thr Thr Thr Asn Ile Asn Lys
        355                 360                 365

Met Lys Asp Tyr Phe Ile Lys Arg Ile Lys His Tyr Tyr Asp Gly Gly
    370                 375                 380

Lys Leu Glu Val Ser Lys Ala Ala Thr Ile Lys Gln Ser Asp Val Lys
385                 390                 395                 400

Gln Glu Val Lys Lys Gln Glu Ala Lys Gln Ile Val Lys Ala Thr Asp
                405                 410                 415

Trp Lys Gln Asn Lys Asp Gly Ile Trp Tyr Lys Ala Glu His Ala Ser
            420                 425                 430

Phe Thr Val Thr Ala Pro Glu Gly Ile Ile Thr Arg Tyr Lys Gly Pro
        435                 440                 445

Trp Thr Gly His Pro Gln Ala Gly Val Leu Gln Lys Gly Gln Thr Ile
    450                 455                 460

Lys Tyr Asp Glu Val Gln Lys Phe Asp Gly His Val Trp Val Ser Trp
465                 470                 475                 480

Glu Thr Phe Glu Gly Glu Thr Val Tyr Met Pro Val Arg Thr Trp Asp
                485                 490                 495

Ala Lys Thr Gly Lys Val Gly Lys Leu Trp Gly Glu Ile Lys
            500                 505                 510

<210>  85
<211>  1533
<212>  DNA
<213>  artificial

<220>
<223>  BuforinII-Ply2638A

<400>  85
atgacccgta gctctcgtgc tggcctgcag tttccggttg gtcgcgtgca ccgtctgctc      60

cgcaaaggat ccatgctaac tgctattgac tatcttacga aaaaaggttg gaaaatatca     120

tctgaccctc gcacttacga tggttaccct aaaaactacg gctacagaaa ttaccatgaa     180

aacggcatta attatgatga gttttgtggt ggttatcata gagcttttga tgtttacagt     240

aacgaaacta acgacgtgcc tgctgttact agcggaacag ttattgaagc aaacgattac     300

ggtaattttg gtggtacatt cgttattaga gacgctaacg ataacgattg gatatatggg     360

catctacaac gtggctcaat gcgatttgtt gtaggcgaca aagtcaatca aggtgacatt     420

attggtttac aaggtaatag caactattac gacaatccta tgagtgtaca tttacattta     480

caattacgcc ctaaagacgc aaagaaagat gaaaaatcac aagtatgtag tggtttggct     540

atggaaaaat atgacattac aaatttaaat gctaaacaag ataaatcaaa gaatgggagc     600

gtgaaagagt tgaaacatat ctattcaaac catattaaag gtaacaagat tacagcacca     660

aaacctagta ttcaaggtgt ggtcatccac aatgattatg gtagtatgac acctagtcaa     720

tacttaccat ggttatatgc acgtgagaat aacggtacac acgttaacgg ttgggctagt     780

gtttatgcaa atagaaacga agtgctttgg tatcatccga cagactacgt agagtggcat     840

tgtggtaatc aatgggcaaa tgctaactta atcggatttg aagtgtgtga gtcgtatcct     900

ggtagaatct cggacaaatt attcttagaa aatgaagaag cgacattgaa agtagctgcg     960

gatgtgatga agtcgtacgg attaccagtt aatcgcaaca ctgtacgtct gcataacgaa    1020

ttcttcggaa cttcttgtcc acatcgttcg tgggacttgc atgttggcaa aggtgagcct    1080

tacacaacta ctaatattaa taaaatgaaa gactacttca tcaaacgcat caaacattat    1140

tatgacggtg gaaagctaga agtaagcaaa gcagcaacta tcaaacaatc tgacgttaag    1200

caagaagtta aaaagcaaga agcaaaacaa attgtgaaag caacagattg gaaacagaat    1260

aaagatggca tttggtataa agctgaacat gcttcgttca cagtgacagc accagaggga    1320

attatcacaa gatacaaagg tccttggact ggtcacccac aagctggtgt attacaaaaa    1380

ggtcaaacga ttaaatatga tgaggttcaa aaatttgacg gtcatgtttg ggtatcgtgg    1440

gaaacgtttg agggcgaaac tgtatacatg ccggtacgca catgggacgc taaaactggt    1500

aaagttggta agttgtgggg cgaaattaaa tga                                    1533

<210>  86
<211>  524
<212>  PRT
<213>  artificial

<220>
<223>  Ply2638A-LL-37

<400>  86

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp

195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Leu Leu Gly Asp Phe Phe Arg Lys Ser
                485                 490                 495

Lys Glu Lys Ile Gly Lys Glu Phe Lys Arg Ile Val Gln Arg Ile Lys
            500                 505                 510

Asp Phe Leu Arg Asn Leu Val Pro Arg Thr Glu Ser
        515                 520

<210>  87
<211>  1575
<212>  DNA
<213>  artificial

<220>
<223>  Ply2638A-LL-37

<400>  87
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct     420

aaagacgcaa agaaagatga aaaatcacaa gtatgtagtg gtttggctat ggaaaaatat     480

gacattacaa atttaaatgc taaacaagat aaatcaaaga atgggagcgt gaaagagttg     540

aaacatatct attcaaacca tattaaaggt aacaagatta cagcaccaaa acctagtatt     600

caaggtgtgg tcatccacaa tgattatggt agtatgacac ctagtcaata cttaccatgg     660

ttatatgcac gtgagaataa cggtacacac gttaacggtt gggctagtgt ttatgcaaat     720

agaaacgaag tgctttggta tcatccgaca gactacgtag agtggcattg tggtaatcaa     780

tgggcaaatg ctaacttaat cggatttgaa gtgtgtgagt cgtatcctgg tagaatctcg     840

gacaaattat tcttagaaaa tgaagaagcg acattgaaag tagctgcgga tgtgatgaag     900

tcgtacggat taccagttaa tcgcaacact gtacgtctgc ataacgaatt cttcggaact     960

tcttgtccac atcgttcgtg ggacttgcat gttggcaaag gtgagcctta cacaactact    1020

aatattaata aaatgaaaga ctacttcatc aaacgcatca aacattatta tgacggtgga 1080

aagctagaag taagcaaagc agcaactatc aaacaatctg acgttaagca agaagttaaa 1140

aagcaagaag caaaacaaat tgtgaaagca acagattgga aacagaataa agatggcatt 1200

tggtataaag ctgaacatgc ttcgttcaca gtgacagcac cagagggaat tatcacaaga 1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc cctcctgggt gacttctttc gcaaatccaa agagaaaatc 1500

ggcaaagagt tcaaacgtat cgtgcagcgc attaaagact ttctgcgtaa cctggttccg 1560

cgtaccgaat cttaa 1575

<210> 88
<211> 516
<212> PRT
<213> artificial

<220>
<223> Ply2638A-SMAP-29

<400> 88

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Arg Gly Leu Arg Arg Leu Gly Arg Lys
                485                 490                 495

Ile Ala His Gly Val Lys Lys Tyr Gly Pro Thr Val Leu Arg Ile Ile
            500                 505                 510

Arg Ile Ala Gly
        515

<210>  89
<211>  1551
<212>  DNA
<213>  artificial

<220>
<223>  Ply2638A-SMAP-29

<400>  89
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct     420

aaagacgcaa agaaagatga aaaatcacaa gtatgtagtg gtttggctat ggaaaaatat     480

gacattacaa atttaaatgc taaacaagat aaatcaaaga atgggagcgt gaaagagttg     540

aaacatatct attcaaacca tattaaaggt aacaagatta cagcaccaaa acctagtatt 600

caaggtgtgg tcatccacaa tgattatggt agtatgacac ctagtcaata cttaccatgg 660

ttatatgcac gtgagaataa cggtacacac gttaacggtt gggctagtgt ttatgcaaat 720

agaaacgaag tgctttggta tcatccgaca gactacgtag agtggcattg tggtaatcaa 780

tgggcaaatg ctaacttaat cggatttgaa gtgtgtgagt cgtatcctgg tagaatctcg 840

gacaaattat tcttagaaaa tgaagaagcg acattgaaag tagctgcgga tgtgatgaag 900

tcgtacggat taccagttaa tcgcaacact gtacgtctgc ataacgaatt cttcggaact 960

tcttgtccac atcgttcgtg ggacttgcat gttggcaaag gtgagcctta cacaactact 1020

aatattaata aaatgaaaga ctacttcatc aaacgcatca aacattatta tgacggtgga 1080

aagctagaag taagcaaagc agcaactatc aaacaatctg acgttaagca agaagttaaa 1140

aagcaagaag caaaacaaat tgtgaaagca acagattgga aacagaataa agatggcatt 1200

tggtataaag ctgaacatgc ttcgttcaca gtgacagcac cagagggaat tatcacaaga 1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc ccgtggtctg cgtcgcctgg gtcgcaaaat tgcgcacggc 1500

gtcaaaaaat acggcccgac cgtgctgcgc attatccgca tcgctggtta a 1551

<210> 90
<211> 523
<212> PRT
<213> artificial

<220>
<223> Ply2638A-Cecropin A (A.aegypti)

<400> 90

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly

65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Gly Gly Leu Lys Lys Leu Gly Lys Lys
                485                 490                 495

Leu Glu Gly Ala Gly Lys Arg Val Phe Asn Ala Ala Glu Lys Ala Leu
            500                 505                 510

Pro Val Val Ala Gly Ala Lys Ala Leu Arg Lys
        515                 520

<210>  91
<211>  1572
<212>  DNA
<213>  artificial

<220>
<223>  Ply2638A-Cecropin A (A.aegypti)

<400>  91
atgctaactg ctattgacta tcttacgaaa aaaggttgga aaatatcatc tgaccctcgc      60

acttacgatg gttaccctaa aaactacggc tacagaaatt accatgaaaa cggcattaat 120

tatgatgagt tttgtggtgg ttatcataga gcttttgatg tttacagtaa cgaaactaac 180

gacgtgcctg ctgttactag cggaacagtt attgaagcaa acgattacgg taattttggt 240

ggtacattcg ttattagaga cgctaacgat aacgattgga tatatgggca tctacaacgt 300

ggctcaatgc gatttgttgt aggcgacaaa gtcaatcaag gtgacattat tggtttacaa 360

ggtaatagca actattacga caatcctatg agtgtacatt tacatttaca attacgccct 420

aaagacgcaa agaaagatga aaaatcacaa gtatgtagtg gtttggctat ggaaaaatat 480

gacattacaa atttaaatgc taaacaagat aaatcaaaga atgggagcgt gaaagagttg 540

aaacatatct attcaaacca tattaaaggt aacaagatta cagcaccaaa acctagtatt 600

caaggtgtgg tcatccacaa tgattatggt agtatgacac ctagtcaata cttaccatgg 660

ttatatgcac gtgagaataa cggtacacac gttaacggtt gggctagtgt ttatgcaaat 720

agaaacgaag tgctttggta tcatccgaca gactacgtag agtggcattg tggtaatcaa 780

tgggcaaatg ctaacttaat cggatttgaa gtgtgtgagt cgtatcctgg tagaatctcg 840

gacaaattat tcttagaaaa tgaagaagcg acattgaaag tagctgcgga tgtgatgaag 900

tcgtacggat taccagttaa tcgcaacact gtacgtctgc ataacgaatt cttcggaact 960

tcttgtccac atcgttcgtg ggacttgcat gttggcaaag gtgagcctta cacaactact 1020

aatattaata aaatgaaaga ctacttcatc aaacgcatca aacattatta tgacggtgga 1080

aagctagaag taagcaaagc agcaactatc aaacaatctg acgttaagca agaagttaaa 1140

aagcaagaag caaaacaaat tgtgaaagca acagattgga aacagaataa agatggcatt 1200

tggtataaag ctgaacatgc ttcgttcaca gtgacagcac cagagggaat tatcacaaga 1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc cggcggcctg aaaaaactgg gcaaaaaact ggaaggtgcc 1500

ggcaaacgtg tgttcaacgc tgcagaaaaa gcactgccgg ttgtagctgg tgctaaagct 1560

ctccgtaaat aa 1572

<210> 92
<211> 526
<212> PRT
<213> artificial

<220>
<223> Ply2638A-Sarcotoxin IA

<400> 92

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Gly Trp Leu Lys Lys Ile Gly Lys Lys
                485                 490                 495

Ile Glu Arg Val Gly Gln His Thr Arg Asp Ala Thr Ile Gln Gly Leu
            500                 505                 510

Gly Ile Ala Gln Gln Ala Ala Asn Val Ala Ala Thr Ala Arg
        515                 520                 525

<210>  93
<211>  1581
<212>  DNA
<213>  artificial

<220>
<223>  Ply2638A-Sarcotoxin IA

<400>  93
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct     420

aaagacgcaa agaaagatga aaaatcacaa gtatgtagtg gtttggctat ggaaaaatat     480

gacattacaa atttaaatgc taaacaagat aaatcaaaga atgggagcgt gaaagagttg     540

aaacatatct attcaaacca tattaaaggt aacaagatta cagcaccaaa acctagtatt     600

caaggtgtgg tcatccacaa tgattatggt agtatgacac ctagtcaata cttaccatgg     660

ttatatgcac gtgagaataa cggtacacac gttaacggtt gggctagtgt ttatgcaaat     720

agaaacgaag tgctttggta tcatccgaca gactacgtag agtggcattg tggtaatcaa     780

tgggcaaatg ctaacttaat cggatttgaa gtgtgtgagt cgtatcctgg tagaatctcg     840

gacaaattat tcttagaaaa tgaagaagcg acattgaaag tagctgcgga tgtgatgaag     900

tcgtacggat taccagttaa tcgcaacact gtacgtctgc ataacgaatt cttcggaact     960

tcttgtccac atcgttcgtg ggacttgcat gttggcaaag gtgagcctta cacaactact    1020

aatattaata aaatgaaaga ctacttcatc aaacgcatca aacattatta tgacggtgga    1080

aagctagaag taagcaaagc agcaactatc aaacaatctg acgttaagca agaagttaaa    1140

aagcaagaag caaaacaaat tgtgaaagca acagattgga aacagaataa agatggcatt    1200

tggtataaag ctgaacatgc ttcgttcaca gtgacagcac cagagggaat tatcacaaga    1260

tacaaaggtc cttggactgg tcacccacaa gctggtgtat tacaaaaagg tcaaacgatt    1320

aaatatgatg aggttcaaaa atttgacggt catgtttggg tatcgtggga aacgtttgag    1380

ggcgaaactg tatacatgcc ggtacgcaca tgggacgcta aaactggtaa agttggtaag    1440

ttgtggggcg aaattaaatc cggatggctc aaaaagattg gcaagaaaat cgagcgagtc 1500

ggtcagcata cgcgtgatgc aactatccag ggtttaggta tcgcacagca agcagctaat 1560

gtagcagcta ctgctcggta a 1581

<210> 94
<211> 541
<212> PRT
<213> artificial

<220>
<223> Ply2638A-OR-7

<400> 94

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe

435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Lys Thr Tyr Tyr Gly Thr Asn Gly Val
                485                 490                 495

His Cys Thr Lys Asn Ser Leu Trp Gly Lys Val Arg Leu Lys Asn Met
            500                 505                 510

Lys Tyr Asp Gln Asn Thr Thr Tyr Met Gly Arg Leu Gln Asp Ile Leu
        515                 520                 525

Leu Gly Trp Ala Thr Gly Ala Phe Gly Lys Thr Phe His
    530                 535                 540

<210>  95
<211>  1626
<212>  DNA
<213>  artificial

<220>
<223>  Ply2638A-OR-7

<400>  95
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct     420

aaagacgcaa agaaagatga aaaatcacaa gtatgtagtg gtttggctat ggaaaaatat     480

gacattacaa atttaaatgc taaacaagat aaatcaaaga atgggagcgt gaaagagttg     540

aaacatatct attcaaacca tattaaaggt aacaagatta cagcaccaaa acctagtatt     600

caaggtgtgg tcatccacaa tgattatggt agtatgacac ctagtcaata cttaccatgg     660

ttatatgcac gtgagaataa cggtacacac gttaacggtt gggctagtgt ttatgcaaat     720

agaaacgaag tgctttggta tcatccgaca gactacgtag agtggcattg tggtaatcaa     780

tgggcaaatg ctaacttaat cggatttgaa gtgtgtgagt cgtatcctgg tagaatctcg     840

gacaaattat tcttagaaaa tgaagaagcg acattgaaag tagctgcgga tgtgatgaag 900

tcgtacggat taccagttaa tcgcaacact gtacgtctgc ataacgaatt cttcggaact 960

tcttgtccac atcgttcgtg ggacttgcat gttggcaaag gtgagcctta cacaactact 1020

aatattaata aaatgaaaga ctacttcatc aaacgcatca aacattatta tgacggtgga 1080

aagctagaag taagcaaagc agcaactatc aaacaatctg acgttaagca agaagttaaa 1140

aagcaagaag caaaacaaat tgtgaaagca acagattgga aacagaataa agatggcatt 1200

tggtataaag ctgaacatgc ttcgttcaca gtgacagcac cagagggaat tatcacaaga 1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc caaaacctac tatggcacca acggcgtaca ctgcaccaaa 1500

aacagcctgt ggggtaaagt gcgtctgaaa aacatgaaat acgaccagaa caccacgtat 1560

atgggccgtc tccaggatat cctgctgggt tgggcaactg gtgccttcgg caaaaccttt 1620

cactaa 1626

<210> 96
<211> 500
<212> PRT
<213> artificial

<220>
<223> Ply2638A-Indolicidin

<400> 96

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
        100                 105                 110

Gln Gly Asp Ile Ile Gly Leu Gln Gly Asn Ser Asn Tyr Tyr Asp Asn
    115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
        180                 185                 190

Ile Thr Ala Pro Lys Pro Ser Ile Gln Gly Val Val Ile His Asn Asp
    195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
        260                 265                 270

Glu Ser Tyr Pro Gly Arg Ile Ser Asp Lys Leu Phe Leu Glu Asn Glu
    275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
        340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Ile Leu Pro Trp Lys Trp Pro Trp Trp
                485                 490                 495

Pro Trp Arg Arg
            500

<210>  97
<211>  1503
<212>  DNA
<213>  artificial

<220>
<223>  Ply2638A-Indolicidin

<400>  97
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct 420

aaagacgcaa agaaagatga aaaatcacaa gtatgtagtg gtttggctat ggaaaaatat 480

gacattacaa atttaaatgc taaacaagat aaatcaaaga atgggagcgt gaaagagttg 540

aaacatatct attcaaacca tattaaaggt aacaagatta cagcaccaaa acctagtatt 600

caaggtgtgg tcatccacaa tgattatggt agtatgacac ctagtcaata cttaccatgg 660

ttatatgcac gtgagaataa cggtacacac gttaacggtt gggctagtgt ttatgcaaat 720

agaaacgaag tgctttggta tcatccgaca gactacgtag agtggcattg tggtaatcaa 780

tgggcaaatg ctaacttaat cggatttgaa gtgtgtgagt cgtatcctgg tagaatctcg 840

gacaaattat tcttagaaaa tgaagaagcg acattgaaag tagctgcgga tgtgatgaag 900

tcgtacggat taccagttaa tcgcaacact gtacgtctgc ataacgaatt cttcggaact 960

tcttgtccac atcgttcgtg ggacttgcat gttggcaaag gtgagcctta cacaactact 1020

aatattaata aaatgaaaga ctacttcatc aaacgcatca aacattatta tgacggtgga 1080

aagctagaag taagcaaagc agcaactatc aaacaatctg acgttaagca agaagttaaa 1140

aagcaagaag caaaacaaat tgtgaaagca acagattgga aacagaataa agatggcatt 1200

tggtataaag ctgaacatgc ttcgttcaca gtgacagcac cagagggaat tatcacaaga 1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc catcctgccg tggaaatggc cgtggtggcc atggcgtcgc 1500

taa 1503

<210> 98
<211> 511
<212> PRT
<213> artificial

<220>
<223> Ply2638A-Pseudin1

<400> 98

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Gly Leu Asn Thr Leu Lys Lys Val Phe
                485                 490                 495

Gln Gly Leu His Glu Ala Ile Lys Leu Ile Asn Asn His Val Gln
            500                 505                 510

<210>  99
<211>  1536
<212>  DNA
<213>  artificial

<220>
<223>  Ply2638A-Pseudin1

<400>  99
atgctaactg ctattgacta tcttacgaaa aaaggttgga aaatatcatc tgaccctcgc      60

acttacgatg gttaccctaa aaactacggc tacagaaatt accatgaaaa cggcattaat 120

tatgatgagt tttgtggtgg ttatcataga gcttttgatg tttacagtaa cgaaactaac 180

gacgtgcctg ctgttactag cggaacagtt attgaagcaa acgattacgg taattttggt 240

ggtacattcg ttattagaga cgctaacgat aacgattgga tatatgggca tctacaacgt 300

ggctcaatgc gatttgttgt aggcgacaaa gtcaatcaag gtgacattat tggtttacaa 360

ggtaatagca actattacga caatcctatg agtgtacatt tacatttaca attacgccct 420

aaagacgcaa agaaagatga aaaatcacaa gtatgtagtg gtttggctat ggaaaaatat 480

gacattacaa atttaaatgc taaacaagat aaatcaaaga atgggagcgt gaaagagttg 540

aaacatatct attcaaacca tattaaaggt aacaagatta cagcaccaaa acctagtatt 600

caaggtgtgg tcatccacaa tgattatggt agtatgacac ctagtcaata cttaccatgg 660

ttatatgcac gtgagaataa cggtacacac gttaacggtt gggctagtgt ttatgcaaat 720

agaaacgaag tgctttggta tcatccgaca gactacgtag agtggcattg tggtaatcaa 780

tgggcaaatg ctaacttaat cggatttgaa gtgtgtgagt cgtatcctgg tagaatctcg 840

gacaaattat tcttagaaaa tgaagaagcg acattgaaag tagctgcgga tgtgatgaag 900

tcgtacggat taccagttaa tcgcaacact gtacgtctgc ataacgaatt cttcggaact 960

tcttgtccac atcgttcgtg ggacttgcat gttggcaaag gtgagcctta cacaactact 1020

aatattaata aaatgaaaga ctacttcatc aaacgcatca aacattatta tgacggtgga 1080

aagctagaag taagcaaagc agcaactatc aaacaatctg acgttaagca agaagttaaa 1140

aagcaagaag caaaacaaat tgtgaaagca acagattgga aacagaataa agatggcatt 1200

tggtataaag ctgaacatgc ttcgttcaca gtgacagcac cagagggaat tatcacaaga 1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc cggtctgaac accctgaaaa aagtattcca gggtctgcat 1500

gaagcgatta aactgatcaa caaccacgtt cagtaa 1536

<210> 100
<211> 510
<212> PRT
<213> artificial

<220>
<223> Ply2638A-Magainin

<400> 100

Met Leu Thr Ala Ile Asp Tyr Leu Thr Lys Lys Gly Trp Lys Ile Ser

1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Gly Ile Gly Lys Phe Leu His Ser Ala
                485                 490                 495

Lys Lys Phe Gly Lys Ala Phe Val Gly Glu Ile Met Asn Ser
            500                 505                 510

<210>  101
<211>  1533
<212>  DNA
<213>  artificial

<220>
<223>  Ply2638A-Magainin

<400>  101
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct     420

aaagacgcaa agaaagatga aaaatcacaa gtatgtagtg gtttggctat ggaaaaatat     480

gacattacaa atttaaatgc taaacaagat aaatcaaaga atgggagcgt gaaagagttg     540

aaacatatct attcaaacca tattaaaggt aacaagatta cagcaccaaa acctagtatt     600

caaggtgtgg tcatccacaa tgattatggt agtatgacac ctagtcaata cttaccatgg     660

ttatatgcac gtgagaataa cggtacacac gttaacggtt gggctagtgt ttatgcaaat     720

agaaacgaag tgctttggta tcatccgaca gactacgtag agtggcattg tggtaatcaa     780

tgggcaaatg ctaacttaat cggatttgaa gtgtgtgagt cgtatcctgg tagaatctcg     840

gacaaattat tcttagaaaa tgaagaagcg acattgaaag tagctgcgga tgtgatgaag     900

tcgtacggat taccagttaa tcgcaacact gtacgtctgc ataacgaatt cttcggaact     960

tcttgtccac atcgttcgtg ggacttgcat gttggcaaag gtgagcctta cacaactact    1020

aatattaata aaatgaaaga ctacttcatc aaacgcatca aacattatta tgacggtgga    1080

aagctagaag taagcaaagc agcaactatc aaacaatctg acgttaagca agaagttaaa    1140

aagcaagaag caaaacaaat tgtgaaagca acagattgga aacagaataa agatggcatt    1200

tggtataaag ctgaacatgc ttcgttcaca gtgacagcac cagagggaat tatcacaaga    1260

tacaaaggtc cttggactgg tcacccacaa gctggtgtat tacaaaaagg tcaaacgatt    1320

aaatatgatg aggttcaaaa atttgacggt catgtttggg tatcgtggga aacgtttgag    1380

ggcgaaactg tatacatgcc ggtacgcaca tgggacgcta aaactggtaa agttggtaag    1440

ttgtggggcg aaattaaatc cggtatcggc aaattcctgc actccgcaaa aaaattcggc    1500

aaagctttcg tgggcgaaat tatgaactct taa                                 1533

<210>  102
<211>  512
<212>  PRT
<213>  artificial

<220>
<223>  Ply2638A-Pleurocidin

<400>  102

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg

210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly
            420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Gly Trp Gly Ser Phe Phe Lys Lys Ala
                485                 490                 495

Ala His Val Gly Lys His Val Gly Lys Ala Ala Leu Thr His Tyr Leu
            500                 505                 510

<210>  103
<211>  1539
<212>  DNA
<213>  artificial

<220>
<223>  Ply2638A-Pleurocidin

<400>  103
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct     420

aaagacgcaa agaaagatga aaaatcacaa gtatgtagtg gtttggctat ggaaaaatat     480

gacattacaa atttaaatgc taaacaagat aaatcaaaga atgggagcgt gaaagagttg     540

aaacatatct attcaaacca tattaaaggt aacaagatta cagcaccaaa acctagtatt     600

caaggtgtgg tcatccacaa tgattatggt agtatgacac ctagtcaata cttaccatgg     660

ttatatgcac gtgagaataa cggtacacac gttaacggtt gggctagtgt ttatgcaaat     720

agaaacgaag tgctttggta tcatccgaca gactacgtag agtggcattg tggtaatcaa     780

tgggcaaatg ctaacttaat cggatttgaa gtgtgtgagt cgtatcctgg tagaatctcg     840

gacaaattat tcttagaaaa tgaagaagcg acattgaaag tagctgcgga tgtgatgaag     900

tcgtacggat taccagttaa tcgcaacact gtacgtctgc ataacgaatt cttcggaact     960

tcttgtccac atcgttcgtg ggacttgcat gttggcaaag gtgagcctta cacaactact    1020

aatattaata aaatgaaaga ctacttcatc aaacgcatca aacattatta tgacggtgga    1080

aagctagaag taagcaaagc agcaactatc aaacaatctg acgttaagca agaagttaaa    1140

aagcaagaag caaaacaaat tgtgaaagca acagattgga aacagaataa agatggcatt    1200

tggtataaag ctgaacatgc ttcgttcaca gtgacagcac cagagggaat tatcacaaga    1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc cggctggggt tctttcttta aaaaagcggc tcacgttggc 1500

aaacatgtag gtaaagcagc tctgacccac tatctgtaa 1539

<210> 104
<211> 508
<212> PRT
<213> artificial

<220>
<223> Ply2638A-BuforinII

<400> 104

Met Leu Thr Ala Ile Asp Tyr Leu Thr Lys Lys Gly Trp Lys Ile Ser
1               5                   10                  15

Ser Asp Pro Arg Thr Tyr Asp Gly Tyr Pro Lys Asn Tyr Gly Tyr Arg
            20                  25                  30

Asn Tyr His Glu Asn Gly Ile Asn Tyr Asp Glu Phe Cys Gly Gly Tyr
        35                  40                  45

His Arg Ala Phe Asp Val Tyr Ser Asn Glu Thr Asn Asp Val Pro Ala
    50                  55                  60

Val Thr Ser Gly Thr Val Ile Glu Ala Asn Asp Tyr Gly Asn Phe Gly
65                  70                  75                  80

Gly Thr Phe Val Ile Arg Asp Ala Asn Asp Asn Asp Trp Ile Tyr Gly
                85                  90                  95

His Leu Gln Arg Gly Ser Met Arg Phe Val Val Gly Asp Lys Val Asn
            100                 105                 110

Gln Gly Asp Ile Ile Gly Leu Gln Gly Asn Ser Asn Tyr Tyr Asp Asn
        115                 120                 125

Pro Met Ser Val His Leu His Leu Gln Leu Arg Pro Lys Asp Ala Lys
    130                 135                 140

Lys Asp Glu Lys Ser Gln Val Cys Ser Gly Leu Ala Met Glu Lys Tyr
145                 150                 155                 160

Asp Ile Thr Asn Leu Asn Ala Lys Gln Asp Lys Ser Lys Asn Gly Ser
                165                 170                 175

Val Lys Glu Leu Lys His Ile Tyr Ser Asn His Ile Lys Gly Asn Lys
            180                 185                 190

Ile Thr Ala Pro Lys Pro Ser Ile Gln Gly Val Val Ile His Asn Asp
        195                 200                 205

Tyr Gly Ser Met Thr Pro Ser Gln Tyr Leu Pro Trp Leu Tyr Ala Arg
    210                 215                 220

Glu Asn Asn Gly Thr His Val Asn Gly Trp Ala Ser Val Tyr Ala Asn
225                 230                 235                 240

Arg Asn Glu Val Leu Trp Tyr His Pro Thr Asp Tyr Val Glu Trp His
                245                 250                 255

Cys Gly Asn Gln Trp Ala Asn Ala Asn Leu Ile Gly Phe Glu Val Cys
            260                 265                 270

Glu Ser Tyr Pro Gly Arg Ile Ser Asp Lys Leu Phe Leu Glu Asn Glu
        275                 280                 285

Glu Ala Thr Leu Lys Val Ala Ala Asp Val Met Lys Ser Tyr Gly Leu
    290                 295                 300

Pro Val Asn Arg Asn Thr Val Arg Leu His Asn Glu Phe Phe Gly Thr
305                 310                 315                 320

Ser Cys Pro His Arg Ser Trp Asp Leu His Val Gly Lys Gly Glu Pro
                325                 330                 335

Tyr Thr Thr Thr Asn Ile Asn Lys Met Lys Asp Tyr Phe Ile Lys Arg
            340                 345                 350

Ile Lys His Tyr Tyr Asp Gly Gly Lys Leu Glu Val Ser Lys Ala Ala
        355                 360                 365

Thr Ile Lys Gln Ser Asp Val Lys Gln Glu Val Lys Lys Gln Glu Ala
    370                 375                 380

Lys Gln Ile Val Lys Ala Thr Asp Trp Lys Gln Asn Lys Asp Gly Ile
385                 390                 395                 400

Trp Tyr Lys Ala Glu His Ala Ser Phe Thr Val Thr Ala Pro Glu Gly
                405                 410                 415

Ile Ile Thr Arg Tyr Lys Gly Pro Trp Thr Gly His Pro Gln Ala Gly

420                 425                 430

Val Leu Gln Lys Gly Gln Thr Ile Lys Tyr Asp Glu Val Gln Lys Phe
        435                 440                 445

Asp Gly His Val Trp Val Ser Trp Glu Thr Phe Glu Gly Glu Thr Val
    450                 455                 460

Tyr Met Pro Val Arg Thr Trp Asp Ala Lys Thr Gly Lys Val Gly Lys
465                 470                 475                 480

Leu Trp Gly Glu Ile Lys Ser Thr Arg Ser Ser Arg Ala Gly Leu Gln
                485                 490                 495

Phe Pro Val Gly Arg Val His Arg Leu Leu Arg Lys
            500                 505

<210>  105
<211>  1527
<212>  DNA
<213>  artificial

<220>
<223>  Ply2638A-BuforinII

<400>  105
atgctaactg ctattgacta tcttacgaaa aaaggttgga aaatatcatc tgaccctcgc      60

acttacgatg gttaccctaa aaactacggc tacagaaatt accatgaaaa cggcattaat     120

tatgatgagt tttgtggtgg ttatcataga gcttttgatg tttacagtaa cgaaactaac     180

gacgtgcctg ctgttactag cggaacagtt attgaagcaa acgattacgg taattttggt     240

ggtacattcg ttattagaga cgctaacgat aacgattgga tatatgggca tctacaacgt     300

ggctcaatgc gatttgttgt aggcgacaaa gtcaatcaag gtgacattat tggtttacaa     360

ggtaatagca actattacga caatcctatg agtgtacatt tacatttaca attacgccct     420

aaagacgcaa agaaagatga aaaatcacaa gtatgtagtg gtttggctat ggaaaaatat     480

gacattacaa atttaaatgc taaacaagat aaatcaaaga atgggagcgt gaaagagttg     540

aaacatatct attcaaacca tattaaaggt aacaagatta cagcaccaaa acctagtatt     600

caaggtgtgg tcatccacaa tgattatggt agtatgacac ctagtcaata cttaccatgg     660

ttatatgcac gtgagaataa cggtacacac gttaacggtt gggctagtgt ttatgcaaat     720

agaaacgaag tgctttggta tcatccgaca gactacgtag agtggcattg tggtaatcaa     780

tgggcaaatg ctaacttaat cggatttgaa gtgtgtgagt cgtatcctgg tagaatctcg     840

gacaaattat tcttagaaaa tgaagaagcg acattgaaag tagctgcgga tgtgatgaag     900

tcgtacggat taccagttaa tcgcaacact gtacgtctgc ataacgaatt cttcggaact     960

tcttgtccac atcgttcgtg ggacttgcat gttggcaaag gtgagcctta cacaactact 1020

aatattaata aaatgaaaga ctacttcatc aaacgcatca aacattatta tgacggtgga 1080

aagctagaag taagcaaagc agcaactatc aaacaatctg acgttaagca agaagttaaa 1140

aagcaagaag caaaacaaat tgtgaaagca acagattgga aacagaataa agatggcatt 1200

tggtataaag ctgaacatgc ttcgttcaca gtgacagcac cagagggaat tatcacaaga 1260

tacaaaggtc cttggactgg tcacccacaa gctggtgtat tacaaaaagg tcaaacgatt 1320

aaatatgatg aggttcaaaa atttgacggt catgtttggg tatcgtggga aacgtttgag 1380

ggcgaaactg tatacatgcc ggtacgcaca tgggacgcta aaactggtaa agttggtaag 1440

ttgtggggcg aaattaaatc cacccgtagc tctcgtgctg gcctgcagtt tccggttggt 1500

cgcgtgcacc gtctgctccg caaataa 1527